# FASCICULE DE BREVET EUROPEEN

(11) **EP 2 262 805 B1**
(45) Date de publication et mention de la délivrance du brevet: **22.10.2014**
(21) Numéro de dépôt: 09754045.4
(22) Date de dépôt: 20.03.2009
(51) Int. Cl.: C07D 471/04, C07D 213/28, C07D 233/56, A61K 31/437, A61P 25/00, A61P 19/00, A61P 35/00

(54) **DÉRIVÉS POLYSUBSTITUES DE 2-HETEROARYL-6-PHENYL-IMIDAZO[1,2-A]PYRIDINES, LEUR PRÉPARATION ET LEUR APPLICATION EN THÉRAPEUTIQUE**
POLYSUBSTITUIERTE 2-HETEROARYL-6-PHENYL-IMIDAZO-[L,2-A-]PYRIDIN-DERIVATE SOWIE DEREN HERSTELLUNG UND THERAPEUTISCHE ANWENDUNG
POLYSUBSTITUTED DERIVATIVES OF 2-HETEROARYL-6-PHENYL-IMIDAZO[L,2-A] PYRIDINES, AND PREPARATION AND THERAPEUTIC USE THEREOF

(30) Priorité: 21.03.2008 FR 0801584
(43) Date de publication de la demande: 22.12.2010
(73) Titulaire: SANOFI, 75008 Paris (FR)
(72) Inventeur: ALMARIO GARCIA, Antonio, F-75013 Paris (FR); DE PERETTI, Danielle, F-75013 Paris (FR); EVANNO, Yannick, F-75013 Paris (FR); LARDENOIS, Patrick, F-75013 Paris (FR); MACHNIK, David, F-75013 Paris (FR); RAKOTOARISOA, Nathalie, F-75013 Paris (FR)
(74) Mandataire: Le Coupanec, Pascale A.M.P.
(86) Numéro de dépôt international: PCT/FR2009/000303
(87) Numéro de publication internationale: WO 2009/144395

(56) Documents cités:
- WO-A-2008/034974
- FR-A- 2 903 105
- FR-A- 2 903 107

## Description

La présente invention se rapporte à des dérivés polysubstitués de 2-hétéroaryl-6-phényl-imidazo[1,2-a]pyridine, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

Les demandes WO2008/034974, FR2903105 et FR2903107 de la société déposante décrivent respectivement des dérivés de 2-aryl-6-phényl-imidazo[1,2-α]pyridines, des dérivés de 2-benzoyl-imidazo[1,2-a]pyridines et des dérivés d'imidazo[1,2-α]pyridine-2-carboxamides, utiles pour la prévention ou le traitement de maladies impliquant les récepteurs nucléaires Nurr-1.

La présente invention décrit les composés de formule (I) : 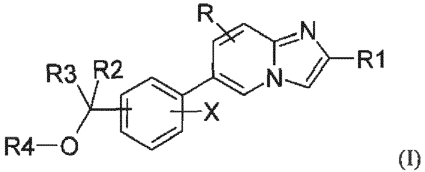 dans laquelle :
R₁ représente :
   un groupe hétéroaryle ou hétérocyclique, ce groupe pouvant éventuellement être substitué par un ou plusieurs atomes ou groupes choisis indépendamment les uns des autres parmi les atomes ou groupes suivants : halogène, (C₁-C₁₀)alkyle, halo(C₁-C₁₀)alkyle, (C₁-C₁₀)alcoxy, halo(C₁-C₁₀)alcoxy, oxo, (C₁-C₁₀)thioalkyle, -S(O)(C₁-C₁₀)alkyle, -S(O)₂(C₁-C₁₀-alkyle), hydroxyle, cyano, nitro, hydroxy(C₁-C₁₀)alkylène, NRaRb(C₁-C₁₀)alkylène, (C₁-C₁₀)alcoxy(C₁-C₁₀)alkylène-oxy, NRaRb, CONRaRb, SO₂NRaRb, NRcCORd, OC(O)NRaRb, OCO(C₁-C₁₀)alkyle, NRcC(O)ORe, NRcSO₂Re aryl(C₁-C₁₀)alkylène, aryle ou hétéroaryle monocyclique, l'aryle ou l'hétéroaryle monocyclique étant éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, oxo, nitro, cyano ou OCO(C₁-C₁₀)alkyle et R1 est lié à l'imidazo[1,2-a]pyridine par un carbone aromatique;
X représente de 1 à 4 substituants identiques ou différents l'un de l'autre choisi parmi hydrogène, un halogène, (C₁-C₁₀)alkyle, (C₁-C₁₀)alcoxy, NRaRb, nitro, cyano, le groupe (C₁-C₁₀)alkyle pouvant être éventuellement substitué par un ou plusieurs groupes choisis parmi un halogène, (C₁-C₁₀)alcoxy, (C₁-C₁₀)haloalcoxy, NRaRb ou hydroxyle ;
R représente en position 3, 5, 7 ou 8 de l'imidazo[1,2-a]pyridine de 1 à 4 substituants identiques ou différents l'un de l'autre choisis parmi un hydrogène, un halogène, (C₁-C₁₀)alkyle, halo(C₁-C₁₀)alkyle, (C₁-C₁₀)alcoxy ;
R₂ et R₃ représentent, indépendamment l'un de l'autre,
   un atome d'hydrogène,
   un groupe (C₁-C₁₀)alkyle, éventuellement substitué par un groupe Rf ;
   un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro ou cyano ;
R₂ et X peuvent former ensemble avec les atomes de carbone qui les porte un cycle carboné de 5 à 7 atomes de carbone;
R₄ représente :
   un atome d'hydrogène,
   un groupe (C₁-C₁₀)alkyle, éventuellement substitué par un groupe Rf ;
   un groupe aryle, éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano, (C₁-C₁₀)alkyl(CO)-, CONRaRb, NRcCORd, OC(O)NRaRb, OCO(C₁-C₁₀)alkyle, NRcC(O)ORe ou aryle, l'aryle étant éventuellement substitué par un ou plusieurs substituants choisis parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro ou cyano ;
Ra et Rb représentent, indépendamment l'un de l'autre, un atome d'hydrogène ou un groupe (C₁-C₁₀)alkyle, aryl(C₁-C₁₀)alkylène ou aryle ;
ou Ra et Rb forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe (C₁-C₁₀)alkyle, aryle ou aryl(C₁-C₁₀)alkylène ;
Rc et Rd représentent, indépendamment l'un de l'autre, un atome d'hydrogène ou un groupe (C₁-C₁₀)alkyle, aryl(C₁-C₁₀)alkylène, aryle,
ou Rc et Rd forment ensemble un groupe (C₂-C₅)alkylène ;
Re représente un groupe (C₁-C₁₀)alkyle, aryl(C₁-C₁₀)alkylène, aryle,
ou Rc et Re forment ensemble un groupe (C₂-C₅)alkylène ;
Rf représente un atome d'halogène, un groupe (C₁-C₁₀)alcoxy, halo(C₁-C₁₀)alcoxy, hydroxyle, cyano, NRaRb, C(O)NRaRb, NRcCORd, OC(O)NRaRb, OCO(C₁-C₁₀)alkyle, NRcCOORe, SO₂NRaRb, NRcSO₂Re, aryl(C₁-C₁₀)alkylène, aryle, l'aryle étant éventuellement substitué par un ou plusieurs substituants choisi parmi un halogène, un groupe (C₁-C₁₀)alkyle, halo(C₁-C₁₀)alkyle, (C₁-C₁₀)alcoxy, halo(C₁-C₁₀)alcoxy, NRaRb, hydroxyle, nitro, cyano ou OCO(C₁-C₁₀)alkyle;
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un groupe (Cₓ-Cₜ) : un groupe comprenant entre x et t atomes de carbone ;
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl, cyclopropylméthyl etc ;
- un groupe alkylène : un groupe alkyle divalent
- un groupe alcoxy.: un radical -0-alkyle où le groupe alkyle est tel que précédemment défini ;
- un groupe haloalkyle : un groupe alkyle substitué par un ou plusieurs atomes d'halogène identiques ou différents. A titre d'exemples, on peut citer les groupes CF₃, CH₂CF₃, CHF₂, CCl₃.
- un groupe haloalcoxy : un radical -O-alkyle où le groupe alkyle est tel que précédemment défini et substitué par un ou plusieurs atomes d'halogène identiques ou différents. A titre d'exemples, on peut citer les groupes OCF₃, OCHF₂, OCCl₃.
- un groupe thioalkyle : un radical S-alkyle où le groupe alkyle est tel que précédemment défini ;
- un groupe aryle : un groupe aromatique mono ou bicyclique comportant de 6 à 10 atomes. A titre d'exemples de groupes aryle, on peut citer les groupes phényle et naphthyle ;
- un groupe hétéroaryle : un groupe aromatique mono ou bicyclique comportant de 5 à 10 atomes dont de 1 à 4 hétéroatomes choisis parmi N, O et S. A titre d'exemples de groupes hétéroaryles, on peut citer : pyrrole, furane, thiophène, pyrazole, imidazole, triazole, tetrazole, oxazole, isoxazole, oxadiazole, thiazole, isothiazole, thiadiazole, pyridine, pyrimidine, pyrazine, pyridazine, triazine, thiéno-thiophène, furo-furane, thiéno-furane, furo-pyrrole, thiéno-pyrrole, pyrrolo-pyrrole, pyrrolo-isoxazole, furo-isoxazole, thiéno-isoxazole, isoxazolo-isoxazole, pyrrolo-oxazole, furo-oxazole, thiéno-oxazole, oxazolo-isoxazole, oxazolo-oxazole, pyrrolo-isothiazole, furo-isothiazole, thiéno-isothiazole, isothiazolo-isoxazole, isothiazolo-oxazole, isothiazolo-isothiazole, pyrrolo-thiazole, furo-thiazole, thiéno-thiazole, thiazolo-oxazole, thiazolo-isoxazole, thiazolo-isothiazole, thiazolo-thiazole, pyrrolo-pyrazole, furo-pyrazole, thiéno-pyrazole, pyrazolo-isoxazole, pyrazolo-oxazole, pyrazolo-isothiazole, pyrazolo-thiazole, pyrazolo-pyrazole, pyrrolo-imidazole, furo-imidazole, thiéno-imidazole, imidazo-isoxazole, imidazo-oxazole, imidazo-isothiazole, imidazo-thiazole, imidazo-pyrazole, imidazo-imidazole, pyrrolo-oxadiazole, furo-oxadiazole, thiéno-oxadiazole, pyrazolo-oxadiazole, imidazo-oxadiazole, furo-thiadiazole, thiéno-thiadiazole, pyrrolo-thiadiazole, imidazo-thiadiazole, pyrazolo-thiadiazole, thiéno-triazole, pyrrolo-triazole, furo-triazole, oxazolo-triazole, isoxazolo-triazole, thiazolo-triazole, isothiazolo-triazole, pyrazolo-triazole, imidazo-triazole, indole, isoindole, benzimidazole, indazole, indolizine, benzofurane, isobenzofurane, benzothiophène, benzo[c]thiophène, pyrrolopyridine, imidazopyridine, pyrazolopyridine, triazolopyridine, tétrazolopyridine, pyrrolopyrimidine, imidazopyrimidine, pyrazolopyrimidine, triazolopyrimidine, pyrrolopyrazine, imidazopyrazine, pyrazolopyrazine, triazolopyrazine, pyrrolopyridazine, imidazopyridazine, pyrazolopyridazine, triazolopyridazine, pyrrolotriazine, imidazotriazine, pyrazolotriazine, furopyridine, furopyrimidine, furopyrazine, furopyridazine, furotriazine, oxazolopyridine, oxazolopyrimidine, oxazolopyrazine, oxazolopyridazine, isoxazolopyridine, isoxazolopyrimidine, isoxazolopyrazine, isoxazolopyridazine, oxadiazolopyridine, benzoxazole, benzisoxazole, benzoxadiazole, thiénopyridine, thiénopyrimidine, thiénopyrazine, thiénopyridazine, thiénotriazine, thiazolopyridine, thiazolopyrimidine, thiazolopyrazine, thiazolopyridazine, isothiazolopyridine, isothiazolopyrimidine, isothiazolopyrazine, isothiazolopyridazine, thiadiazolopyridine, benzothiazole, benzoisothiazole, benzothiadiazole, benzotriazole, quinoléine, isoquinoléine, cinnoline, phthalazine, quinoxaline, quinazoline, naphthyridine, benzotriazine, pyridopyrimidine, pyridopyrazine, pyridopyridazine, pyridotriazine, pyrimidopyrimidine, pyrimidopyrazine, pyrimidopyridazine, pyrazinopyrazine, pyrazinopyridazine, pyridazinopyridazine.
- un groupe hétérocyclique : un groupe bicyclique de 9 à 10 atomes comportant de 1 à 4 hétéroatomes choisis parmi N, O et S, dont un cycle est aromatique et l'autre cycle est saturé ou partiellement saturé, chacun des cycles ne comportant au maximum que 2 hétéroatomes. A titre d'exemples de groupes bicycliques, on peut citer : benzodioxole, benzoxathiole, benzopyrane, benzothiopyrane, benzoxazine, benzothiazine, benzodioxine, benzothioxine, dioxolo-pyridine, oxathiolo-pyridine, pyrano-pyridine, thiopyrano-pyridine, oxazino-pyridine, thiazino-pyridine, dioxino-pyridine, thioxino-pyridine, dioxolo-pyrimidine, oxathiolo-pyrimidine, pyrano-pyrimidine, thiopyrano-pyrimidine, oxazino-pyrimidine, thiazino-pyrimidine, dioxino-pyrimidine, thioxino-pyrimidine, dioxolo-pyrazine, oxathiolo-pyrazine, pyrano-pyrazine, thiopyrano-pyrazine, oxazino-pyrazine, thiazino-pyrazine, dioxino-pyrazine, thioxino-pyrazine, dioxolo-pyridazine, oxathiolo-pyridazine, pyrano-pyridazine, thiopyrano-pyridazine, oxazino-pyridazine, thiazino-pyridazine, dioxino-pyridazine, thioxino-pyridazine, indole, isoindole, benzimidazole, indazole, indolizine, benzofuran, isobenzofuran, benzothiophene, benzo[c]thiophene pyrrolopyridine, imidazopyridine, pyrazolopyridine, pyrrolopyrimidine, imidazopyrimidine, pyrazolopyrimidine, pyrrolopyrazine, imidazopyrazine, pyrazolopyrazine, pyrrolopyridazine, imidazopyridazine, pyrazolopyridazine, furopyridine, furopyrimidine, furopyrazine,furopyridazine, oxazolopyridine, oxazolopyrimidine, oxazolopyrazine, oxazolopyridazine, isoxazolopyridine, isoxazolopyrimidine, isoxazolopyrazine, isoxazolopyridazine, benzoxazole, benzisoxazole, benzoxadiazole, thienopyridine, thienopyrimidine, thienopyrazine, thienopyridazine, thiazolopyridine, thiazolopyrimidine, thiazolopyrazine, thiazolopyridazine, isothiazolopyridine, isothiazolopyrimidine, isothiazolopyrazine, isothiazolopyridazine, benzothiazole, benzoisothiazoie, quinoléine, isoquinoléine, cinnoline, phthalazine, quinoxaline, quinazoline, naphthyridine, pyridopyrimidine, pyridopyrazine, pyridopyridazine, pyrimidopyrimidine, pyrimidopyrazine, pyrimidopyridazine, pyrazinopyrazine, pyrazinopyridazine, pyridazinopyridazine, l'un des cycles de ces groupes bicycliques étant sous forme saturée ou partiellement saturée, par exemple dihydrobenzofurane, tétrahydroquinoléine, dihydrobenzoxazole, benzodioxole.
- un carbone aromatique : un carbone qui est inclus dans un cycle aromatique
- les atomes de soufre et d'azote peuvent être à l'état oxydé (N-oxide, sulfoxide, sulfone)

Parmi les composés de formule (I) figurent les composés (I) objets de l'invention pour lesquels :
R₁ représente un groupe isoxazolyle, pyridinyle, thiazolyle, quinoléinyle, benzo[1,3]dioxolyle, indolyle, 1,2,3,4-tétrahydroquinoléinyle, benzofuranyle, dihydrobenzofuranyle, dihydrobenzoxazolyle, furyle, thiényle, pyrrolo[2,3-b]pyridinyle, pyrimidinyle, benzothiazolyle, benzothiophényle, benzimidazolyle, indazolyle, benzisoxazolyle, isoquinoléinyle, pyrazolyle ;
ces groupes pouvant éventuellement être substitués par un ou plusieurs atomes ou groupes choisis indépendamment les uns des autres parmi halogène, (C₁-C₁₀)alkyle, oxo, NRaRb ; (C₁-C₁₀)alcoxy, aryle, CONRaRb ;
Ra et Rb représentent, indépendamment l'un de l'autre, un atome d'hydrogène ou un groupe (C₁-C₁₀)alkyle ;
X représente 1 ou 2 atomes d'hydrogène ou d'halogène ;
R représente en position 3 de l'imidazo[1,2-*a*]pyridine un atome d'hydrogène, ou un groupe (C₁-C₁₀)alkyle ;
R₂ et R₃ représentent, indépendamment l'un de l'autre un atome d'hydrogène ou un groupe (C₁-C₁₀)alkyle ;
R₄ représente un atome d'hydrogène, ou un groupe (C₁-C₁₀)alkyle, éventuellement substitué par un groupe Rf ;
Rf représente un groupe (C₁-C₁₀)alcoxy ;
à l'état de base ou de sel d'addition à un acide.

Parmi les composés de formule (I), un premier groupe de composés est constitué des composés pour lesquels :
R₁ représente un groupe isoxazolyle, pyridinyle, thiazolyle, quinoléinyle, benzo[1,3]dioxolyle, indolyle, 1,2,3,4-tétrahydroquinoléinyle, benzofuranyle, dihydrobenzofuranyle, dihydrobenzoxazolyle, furyle, thiényle, pyrrolo[2,3-*b*]pyridinyle, pyrimidinyle, benzothiazolyle, benzothiophényle, benzimidazolyle, indazolyle, benzisoxazolyle, isoquinoléinyle, pyrazolyle ;
ces groupes pouvant éventuellement être substitués par un ou plusieurs atomes ou groupes choisis indépendamment les uns des autres parmi halogène, (C₁-C₁₀)alkyle, oxo, NRaRb ; (C₁-C₁₀)alcoxy, aryle, CONRaRb ;
Ra et Rb représentent, indépendamment l'un de l'autre, un atome d'hydrogène ou un groupe (C₁-C₁₀)alkyle ;
les autres substituants étant définis tels que précédemment.

Parmi les composés de formule (I), un deuxième groupe de composés est constitué des composés pour lesquels :
R₁ représente un groupe isoxazolyle, pyridinyle, thiawlyle, quinoléinyle, benzo[1,3]dioxolyle, indolyle, 1,2,3,4-tétrahydroquinoléinyle, benzofuryle, dihydrobenzofuryle, dihydrobenzoxazolyle, furyle, thiényle, pyrrolo[2,3-b]pyridinyle, pyrimidinyle, benzothiazolyle, benzothiophényle, benzimidazolyle, indazolyle, benzisoxazolyle , isoquinoléinyle, pyrazolyle ;
ces groupes pouvant éventuellement être substitués par un ou plusieurs atomes ou groupes choisis indépendamment les uns des autres parmi halogène, méthyle, oxo, NRaRb; méthoxy, éthoxy, phényle, isopentyle, CONHC(CH₃)₃ ;
Ra et Rb représentent, indépendamment l'un de l'autre, un atome d'hydrogène ou un groupe méthyle ;
les autres substituants étant définis tels que précédemment.

Parmi les composés de formule (I), un troisième groupe de composés est constitué des composés pour lesquels :
X représente 1 ou 2 atomes d'hydrogène ou d'halogéne ;
les autres substituants étant définis tels que précédemment.

Parmi les composés de formule (I), un quatrième groupe de composés est constitué des composés pour lesquels :
X représente 1 ou 2 atomes d'hydrogène ou de fluor ;
les autres substituants étant définis tels que précédemment.

Parmi les composés de formule (I), un cinquième groupe de composés est constitué des composés pour lesquels :
R représente en position 3, 5, 7 ou 8 de l'imidazo[1,2-a]pyridine un atome d'hydrogène, ou un groupe (C₁-C₁₀)alkyle ;
les autres substituants étant définis tels que précédemment.

Parmi les composés de formule (I), un sixième groupe de composés est constitué des composés pour lesquels :
R représente en position 3, 5, 7 ou 8 de l'imidazo[1,2-*a*]pyridine un atome d'hydrogène, ou un groupe méthyle ;
les autres substituants étant définis tels que précédemment.

Parmi les composés de formule (I), un septième groupe de composés est constitué des composés pour lesquels :
R₂ et R₃ représentent, indépendamment l'un de l'autre un atome d'hydrogène ou un groupe (C₁-C₁₀)alkyle ;
les autres substituants étant définis tels que précédemment.

Parmi les composés de formule (I), un huitième groupe de composés est constitué des composés pour lesquels :
R₂ et R₃ représentent, indépendamment l'un de l'autre un atome d'hydrogène ou un groupe méthyle ;
les autres substituants étant définis tels que précédemment.

Parmi les composés de formule (I), un neuvième groupe de composés est constitué des composés pour lesquels :
R₄ représente un atome d'hydrogène, ou un groupe (C₁-C₁₀)alkyle, éventuellement substitué par un groupe Rf ;
Rf représente un groupe (C₁-C₁₀)alcoxy ;
les autres substituants étant définis tels que précédemment.

Parmi les composés de formule (I), un dixième groupe de composés est constitué des composés pour lesquels :
R₄ représente un atome d'hydrogène, ou un groupe choisi parmi les groupes méthyle, butyle, méthoxy-éthyle ;
les autres substituants étant définis tels que précédemment.

Parmi les composés de formule (I) objets de l'invention, un onzième groupe de composés est constitué des composés pour lesquels :
le groupe 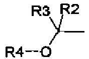 est en position 2, 3 ou 4 du phényle qui le porte ;
les substituants étants tels que définis précédemment.

Parmi les composés de formule (I), un douzième groupe de composés est constitué des composés pour lesquels :
R₁ représente un groupe isoxazolyle, pyridinyle, thiazolyle, quinoléinyle, benzo[1,3]dioxolyle, indolyle, 1,2,3,4-tétrahydroquinoléinyle, benzofuranyle, dihydrobenzofuranyle, dihydrobenzoxazolyle, furyle, thiényle, pyrrolo[2,3-b]pyridinyle, pyrimidinyle, benzothiazolyle, benzothiophényle, benzimidazolyle, indazolyle, benzisoxazolyle, isoquinoléinyle, pyrazolyle ;
ces groupes pouvant éventuellement être substitués par un ou plusieurs atomes ou groupes choisis indépendamment les uns des autres parmi halogène, (C₁-C₁₀)alkyle, oxo, NRaRb ; (C₁-C₁₀)alcoxy, aryle, CONRaRb ;
Ra et Rb représentent, indépendamment l'un de l'autre, un atome d'hydrogène ou un groupe (C₁-C₁₀)alkyle ;
X représente 1 ou 2 atomes d'hydrogène ou d'halogène ;
R représente en position 3, 5, 7 ou 8 de l'imidazo[1,2-a]pyridine un atome d'hydrogène, ou un groupe (C₁-C₁₀)alkyle ;
R₂ et R₃ représentent, indépendamment l'un de l'autre un atome d'hydrogène ou un groupe (C₁-C₁₀)alkyle ;
R₄ représente un atome d'hydrogène, ou un groupe (C₁-C₁₀)alkyle, éventuellement substitué par un groupe Rf ;
Rf représente un groupe (C₁-C₁₀)alcoxy;
à l'état de base ou de sel d'addition à un acide.

Parmi les composés de formule (I), un treizième groupe de composés est constitué des composés pour lesquels :
R₁ représente un groupe isoxazolyle, pyridinyle, thiazolyle, quinoléinyle, benzo[1,3]dioxolyle, indolyle, 1,2,3,4-tétrahydroquinoléinyle, benzofuryle, dihydrobenzofuryle, dihydrobenzoxazolyle, furyle, thiényle, pyrrolo[2,3-*b*]pyridinyle, pyrimidinyle, benzothiazolyle, benzothiophényle, benzimidazolyle, indazolyle, benzisoxazolyle, isoquinoléinyle, pyrazolyle ;
ces groupes pouvant éventuellement être substitués par un ou plusieurs atomes ou groupes choisis indépendamment les uns des autres parmi halogène, méthyle, oxo, NRaRb; éthoxy, phényle, isopentyle, CONHC(CH₃)₃, méthoxy ;
Ra et Rb représentent, indépendamment l'un de l'autre, un atome d'hydrogène ou un groupe méthyle ;
X représente 1 ou 2 atomes d'hydrogène ou de fluor ;
R représente en position 3, 5, 7 ou 8 de l'imidazo[1,2-*a*]pyridine un atome d'hydrogène, ou un groupe méthyle ;
R₂ et R₃ représentent, indépendamment l'un de l'autre un atome d'hydrogène ou un groupe méthyle ;
R₄ représente un atome d'hydrogène, ou un groupe choisi parmi les groupes méthyle, butyle, méthoxy-éthyle ;
à l'état de base ou de sel d'addition à un acide.

Parmi les composés de formule (I), un quatorzième groupe de composés est constitué des composés pour lesquels :
R₁ représente un groupe isoxazolyle, pyridinyle, thiazolyle, quinoléinyle, benzo[1,3]dioxolyle, indolyle, 1,2,3,4-tétrahydroquinoléinyle, benzofuranyle, dihydrobenzofuranyle, dihydrobenzoxazolyle, furyle, thiényle, pyrrolo[2,3-b]pyridinyle, pyrimidinyle, benzothiazolyle, benzothiophényle, benzimidazolyle, indazolyle, benzisoxazolyle, isoquinoléinyle, pyrazolyle ;
ces groupes pouvant éventuellement être substitués par un ou plusieurs atomes ou groupes choisis indépendamment les uns des autres parmi halogène, (C₁-C₁₀)alkyle, oxo, NRaRb ; (C₁-C₁₀)alcoxy, aryle, CONRaRb ;
Ra et Rb représentent, indépendamment l'un de l'autre, un atome d'hydrogène ou un groupe (C₁-C₁₀)alkyle ;
X représente 1 ou 2 atomes d'hydrogène ou d'halogène ;
R représente en position 3, 5, 7 ou 8 de l'imidazo[1,2-a]pyridine un atome d'hydrogène, ou un groupe (C₁-C₁₀)alkyle ;
R₂ et R₃ représentent, indépendamment l'un de l'autre un atome d'hydrogène ou un groupe (C₁-C₁₀)alkyle ;
R₄ représente un atome d'hydrogène, ou un groupe (C₁-C₁₀)alkyle, éventuellement substitué par un groupe Rf ;
Rf représente un groupe (C₁-C₁₀)alcoxy ;
le groupe 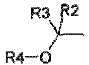 étant en position 2, 3 ou 4 du phényle qui le porte ;
à l'état de base ou de sel d'addition à un acide.

Parmi les composés de formule (I), un quinzième groupe de composés est constitué des composés pour lesquels :
R₁ représente un groupe isoxazolyle, pyridinyle, thiazolyle, quinoléinyle, benzo[1,3]dioxolyle, indolyle, 1,2,3,4-tétrahydroquinoléinyle, benzofuryle, dihydrobenzofuryle, dihydrobenzoxazolyle, furyle, thiényle, pyrrolo[2,3-b]pyridinyle, pyrimidinyle, benzothiazolyle, benzothiophényle, benzimidazolyle, indazolyle, benzisoxazolyle, isoquinoléinyle, pyrazolyle ;
ces groupes pouvant éventuellement être substitués par un ou plusieurs atomes ou groupes choisis indépendamment les uns des autres parmi halogène, méthyle, oxo, NRaRb; éthoxy, phényle, isopentyle, CONHC(CH₃)₃, méthoxy ;
Ra et Rb représentent, indépendamment l'un de l'autre, un atome d'hydrogène ou un groupe méthyle ;
X représente 1 ou 2 atomes d'hydrogène ou de fluor;
R représente en position 3, 5, 7 ou 8 de l'imidazo[1,2-a]pyridine un atome d'hydrogène, ou
un groupe méthyle ;
R₂ et R₃ représentent, indépendamment l'un de l'autre un atome d'hydrogène ou un groupe méthyle ;
R₄ représente un atome d'hydrogène, ou un groupe choisi parmi les groupes méthyle, butyle, méthoxy-éthyle ;
le groupe 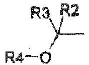 étant en position 2, 3 ou 4 du phényle qui le porte ;
à l'état de base ou de sel d'addition à un acide.

Parmi les composés de formule (I) objets de l'invention, un seizième groupe de composés est constitué des composés pour lesquels :
R₁ représente un groupe isoxazolyle, pyridinyle, thiazolyle, quinoléinyle, benzo[1,3]dioxolyle, indolyle, 1,2,3,4-tétrahydroquinoléinyle, benzofuranyle, dihydrobenzofuranyle, dihydrobenzoxazolyle, furyle, thiényle, pyrrolo[2,3-*b*]pyridinyle, ces groupes pouvant éventuellement être substitués par un ou plusieurs atomes ou groupes choisis indépendamment les uns des autres parmi halogène, (C₁-C₁₀)alkyle, oxo, NRaRb, aryle ;
X représente un hydrogène,
R représente un hydrogène, ou un groupe (C₁-C₁₀)alkyle ;
R₂ et R₃ représentent, indépendamment l'un de l'autre, un atome d'hydrogène ;
R₄ représente un atome d'hydrogène, un groupe (C₁-C₁₀)alkyle, ce groupe étant éventuellement substitué par un groupe Rf ;
Ra et Rb représentent, indépendamment l'un de l'autre, un atome d'hydrogène ou un groupe (C₁-C₁₀)alkyle ;
Rf représente un groupe (C₁-C₁₀)alcoxy;
à l'état de base ou de sel d'addition à un acide.

Parmi les composés de formule (I) objets de l'invention, on peut notamment citer les composés suivants :
- {3-[2-(5-Méthylisoxazol-4-yl)imidazo[1,2-*a*]pyridin-6-yl]phényl}méthanol ;
- [3-[2-(pyridin-4-yl)imidazo[1,2-*a*]pyridin-6-yl]phényl]méthanol et son chlorhydrate ;
- Chlorhydrate (2:1) de 6-(3-*tert*butoxyméthylphényl)-2-(pyridin-3-yl)imidazo[1,2-*a*]pyridine;
- [3-[2-(Thiazol-4-yl)imidazo[1,2-*a*]pyridin-6-yl]phényl]méthanol
- [3-[2-(quinoléin-3-yl)imidazo[1,2-*a*]pyridin-6-yl]phényl]méthanol et son chlorhydrate ;
- {3-[2-(1,3-benzodioxol-5-yl)imidazo[1,2-*a*]pyridin-6-yl]phényl}méthanol et son chlorhydrate ;
- [3-]2-(pyridin-3-yl)imidazo[1,2-*a*]pyridin-6-yl]phényl]méthanol et son chlorhydrate ;
- {3-[2-[(1*H*-indol-5-yl)imidazo[1,2-*a*]pyridin-6-yl]phényl} méthanol et son chlorhydrate ;
- 6-[6-(3-hydroxyméthylphényl)imidazo[1,2-*a*]pyridin-2-yl]-3,4-dihydro-1H-quinoléin-2-one et son chlorhydrate ;
- 2-(5-Bromo-2,3-dihydrobenzofuran-7-yl)-6-(3-*tert-*butoxyméthylphényl)imidazo[1,2-*a*]pyridine ;
- 6-[6-(3-Hydroxyméthylphényl)imidazo[1,2-*a*]pyridin-2-yl]-3*H*-benzoxazol-2-one et son chlorhydrate ;
- Chlorhydrate (1:1) de [2-(2-furan-3-ylimidazo[1,2-*a*]pyridin-6-yl)phényl]méthanol ;
- {3-[2-(5-Bromo-2,3-dihydrobenzofuran-7-yl)imidazo[1,2-*a*]pyridin-6-yl]phényl}méthanol ;
- {3-[2-(5-chlorothién-2-yl)imidazo[1,2-*a*]pyridin-6-yl]phényl}méthanol et son chlorhydrate ;
- {3-[2-(6-diméthylaminopyridin-3-yl)imidazo[1,2-*a*]pyridin-6-yl]phényl}méthanol et son chlorhydrate ;
- {3-[2-(1*H*-indol-4-yl)imidazo[1,2-*a*]pyndin-6-yl]phényl} méthanol et son chlorhydrate ;
- {3-[2-(6-amimopyridin-3-yl)imidazo[1,2-*a*]pyridin-6-yl]phényl} méthanol et son chlorhydrate ;
- {3-[2-(1*H*-indol-3-yl)imidazo[1,2-*a*]pyridin-6-yl]phényl} méthanol et son chlorhydrate ;
- {3-[2-(2-aminopyridin-3-yl)imidazo[1,2-*a*]pyridin-6-yl]phenyl} méthanol et son chlorhydrate ;
- {3-[2-(1*H*-pyrrolo[2,3-*b*]pyridin-5-yl)imidazo[1,2-*a*]pyridin-6-yl]phényl}méthanol et son chlorhydrate ;
- {3-[2-(3Phénylisoxazol-5-yl)imidazo[1,2-*a*]pyridin-6-yl]phényl}méthanol ;
- [3-[2-(Benzofuran-2-yl)imidazo[1,2-*a*]pyridin-6-yl]phényl]méthanol,
- [3-[2-(Benzofuran-3-y)limidazo[1,2-*a*]pyridin-6-yl)phényl]méthanol;
- [4-[2-(Benzofuran-3-yl)imidazo[1,2-*a*]pyridin-6-yl]phényl]méthanol;
- [3-[2-(Pyridin-2-yl)imidazo[1,2-*a*]pyridin-6-yl]phényl]méthanol ;
- [4-[2-(Pyridin-2-yl]imidazo[1,2-*a*]pyridin-6-yl]phényl]méthanol ;
- [3-[2-(Thién-2-yl)imidazo[1,2-*a*]pyridin-6-yl]phényl]méthanol ;
- 2-(Benzofuran-2-yl)-6-[3-(2-méthoxyéthoxyméthyl)phényl]imidazo[1,2-*a*]pyridine ;
- 6-[3-(2-Méthoxyéthoxyméthyl)phényl]-2-(thién-2-yl)imidazo[1,2-*a*]pyridine et son oxalate ;
- [3-(2-Thién-3-ylimidazo[1,2-*a*]pyridin-6-yl)phényl]méthanol ;
- Chlorhydrate (1:1) de [3-(3-méthyl-2-thién-2-yl-imidazo[1,2-a]pyridin-6-yl)phényl]méthanol ;
- {3-[2-(1*H*-indol-3-yl)-3-méthylimidazo[1,2-*a*]pyridin-6-yl]phényl}méthanol et son chlorhydrate ;
- {3-[2-(1*H*-indol-6-yl)imidazo[1,2-*a*]pyridin-6-yl]phényl}méthanol et son chlorhydrate ;
- {3-[2-(2-Ethoxypyrimidin-5-yl)imidazo[1,2-*a*]pyridin-6-yl]phényl}méthanol et son chlorhydrate ;
- [2-(2-Quinoléin-3-ylimidazo[1,2-*a*]pyridin-6-yl)phényl]méthanol et son chlorhydrate ;
- {3-[2-(2-Chloro-pyridin-4-yl)imidazo[1,2-*a*]pyridin-5-yl]phényl}méthanol et son chlorhydrate ;
- [3-(2-Benzothiazol-2-ylimidazo[1,2-*a*]pyridin-6-yl)phényl]methanol et son chlorhydrate ;
- [3-(2-Benzo[b]thiophèn-2-ylimidazo[1,2-*a*]pyridin-6-yl)phényl]méthanol et son chlorhydrate ;
- [3-(2-Benzo[b]thiophén-5-ylimidazo[1,2-*a*]pyridin-6-yl)phényl]méthanol et son chlorhydrate ;
- 3-(2-Benzo[b]thiophèn-3-ylimidazo[1,2-*a*]pyridin-6-yl)phényl]méthanol et son chlorhydrate ;
- {2-[2-(1*H*-Indol-6-yl)imidazo[1,2-*a*]pyridin-6-yl]phényl}méthanol et son chlorhydrate ;
- {3-[2-(2,3-Dihydrobenzofuran-5-yl)imidazo[1,2-*a*]pyridin-6-yl]phényl}méthanol et son chlorhydrate ;
- {2-[2-(1*H*-Indol-5-yl)imidazo[1,2-*a*]pyridin-6-yl]phényl}méthanol et son chlorhydrate ;
- [3-(2-Benzofuran-5-ylimidazo[1,2-*a*]pyridin-6-yl)phényl]méthanol ;
- {3-[2-(3-Chlorothién-2-yl)imidazo[1,2-*a*]pyridin-6-yl]phényl}méthanol ;
- {2-Fluoro-6-[2-(5-methylisoxazol-3-yl)imidazo[1,2-*a*]pyridin-6-yl]phényl}méthanol;
- 2-{3-[2-(1H-Indol-6-yl)imidazo[1,2-*a*]pyridin-6-yl]phényl}propan-2-ol ;
- 2-[3-(3-Méthyl-2-thién-2-ylimidazo[1,2-*a*]pyridin-6-yl)phényl]propan-2-ol ;
- 2-[3-(2-Thién-3-ylimidazo[1,2-*a*]pyridin-6-yl)phényl]propan-2-ol ;
- 2-[3-(2-Pyridin-2-ylimidazo[1,2-*a*]pyridin-6-yl)phényl]propan-2-ol ;
- 2-{3-[2-(5-Chlorothién-2-yl)imidazo[1,2-*a*]pyridin-6-yl]phényl}propan-2-ol ;
- 2-[3-(2-Benzofuran-2-ylimidazo[1,2-*a*]pyridin-6-yl)phényl]propan-2-ol ;
- 2-[3-(2-Thién-2-ylimidazo[1,2-*a*]pyridin-6-yl)phényl]propan-2-ol ;
- 2-[3-(2-Benzofuran-3-ylimidazo[1,2-*a*]pyridin-6-yl)phényl]propan-2-ol ;
- 2-[3-(2-Benzothiazol-2-ylimidazo[1,2-*a*]pyridin-6-yl)phényl]propan-2-ol ;
- 2-[3-(2-Benzo[*b*]thiényl-2-ylimidazo[1,2-*a*]pyridin-6-yl)phényl]propan-2-ol ;
- 2-{3-[2-(1-Méthyl-1H-benzimidazol-2-yl)imidazo[1,2-*a*]pyridin-6-yl]phényl}propan-2-ol ;
- 2-{3-[2-(2,3-Dihydrobenzofuran-5-yl)imidazo[1,2-*a*]pyridin-6-yl]phényl}propan-2-ol;
- 2-[3-(2-Furan-2-ylimidazo[1,2-*a*]pyridin-6-yl)phényl]propan-2-ol ;
- 2-[3-(2-Benzofuran-5-ylimidazo[1,2-*a*]pyridin-6-yl)phényl]propan-2-ol ;
- 2-[3-(2-Benzo[*b*]thiényl-5-ylimidazo[1,2-*a*]pyridin-6-yl)phényl]propan-2-ol ;
- 2-[3-(2-Thiazol-2-ylimidazo[1,2-*a*]pyridin-6-yl)phényl]propan-2-ol ;
- {2-Fluoro-6-[2-(1*H*-indazol-3-yl)imidazo[1,2-*a*]pyridin-6-yl]phényl}méthanol et son chlorhydrate ;
- 2-{3-[2-(1*H*-Indazol-3-yl)imidazo[1,2-*a*]pyridin-6-yl]phényl}propan-2-ol et son chlorhydrate ;
- {2,6-Difluoro-3-[2-(1*H*-indazol-3-yl)imidazo[1,2-*a*]pyridin-6-yl]phényl}méthanol et son chlorhydrate ;
- [2-Fluoro-6-(2-thién-3-ylimidazol[1,2-*a*]pyridin-6-yl)phényl]méthanol ;
- [2-Fluoro-6-(2-pyridin-2-ylimidazo[1,2-*a*]pyridin-6-yl)phényl]méthanol ;
- [2-Fluoro-6-(2-thién-2-ylimidazo[1,2-*a*]pyridin-6-yl)phényl]méthanol ;
- [2-(2-Benzothiazol-2-yl-imidazo[1,2-*a*]pyridin-6-yl)-6-fluorophényl]methanol ;
- [2-(2-Benzo[*b*]thiophén-2-ylimidazo[1,2-*a*]pyridin-6-yl)-6-fluorophényl]méthanol ;
- {2-[2-(2,3-Dihydrobenzofuran-5-yl)imidazo[1,2-*a*]pyridin-6-yl]-6-fluorophényl}méthanol ;
- [2-(2-Benzofuran-5-yl-imidazo[1,2-*a*]pyridin-6-yl)-6-fluorophényl]méthanol ;
- [2-(2-Benzo[b]thiényl-5-ylimidazo[1,2-*a*]pyridin-6-yl)-6-fluorophényl]méthanol ;
- [2-Fluoro-6-(2-thiazol-2-ylimidazo[1,2-*a*]pyridin-6-yl)phényl]méthanol ;
- [2,6-Difluoro-3-(3-méthyl-2-thién-2-ylimidazo[1,2-*a*]pyridin-6-yl)phényl]méthanol ;
- [2,6-Difluoro-3-(2-thién-3-ylimidazo[1,2-*a*]pyridin-6-yl)phényl]méthanol ;
- [2,6-Difluoro-3-(2-pyridin-2-ylimidazo[1,2-*a*]pyridin-6-yl)phényl]méthanol ;
- {3-[2-(5-Chlorothién-2-yl)imidazo[1,2-*a*]pyridin-6-yl]-2,6-difluorophényl}méthanol ;
- [3-(2-Benzofuran-2-ylimidazo[1,2-*a*]pyridin-6-yl)-2,6-difluorophényl]méthanol ;
- [2,6-Difluoro-3-(2-thién-2-ylimidazo[1,2-*a*]pyridin-6-yl)phényl]méthanol;
- [3-(2-Benzofuran-3-ylimidazo[1,2-*a*]pyridin-6-yl)-2,6-difluorophényl]méthanol ;
- [3-(2-Benzothiazol-2-ylimidazo[1,2-*a*]pyridin-6-yl)-2,6-difluorophényl]méthanol ;
- [2,6-Difluoro-3-(2-thiazol-2-ylimidazo[1,2-*a*]pyridin-6-yl)phényl]méthanol ;
- 6-(3-Méthoxyméthylphényl)-2-(1-méthyl-1*H*-indol-6-yl)imidazo[1,2-a]pyridine et son chlorhydrate ;
- 2-(1*H*-Indol-6-yl)-6-(3-méthoxyméthylphényl)imidazo[1,2-*a*]pyridine et son chlorhydrate ;
- 2-{3-[2-(5-Méthylisoxazol-3-yl)imidazo[1,2-*a*]pyridin-6-yl]phényl}propan-2-ol ;
- [2-Fluoro-6-(3-méthyl-2-thiényl-2-ylimidazo[1,2-*a*]pyridin-6-yl)phényl]méthanol ;
- {2-[2-(5-Chlorothiophén-2-yl)imidazo[1,2-*a*]pyridin-6-yl]-6-fluorophényl}méthanol ;
- [2-(2-Benzofuran-2-ylimidazo[1,2-*a*]pyridin-6-yl)-6-fluorophényl]méthanol ;
- [2-(2-Benzofuran-3-ylimidazo[1,2-*a*]pyridin-6-yl)-6-fluorophényl]méthanol ;
- {2-Fluoro-6-[2-(1-méthyl-1*H*-benzimidazol-2-yl)imidazo[1,2-*a*]pyridin-6-yl]phényl}méthanol ;
- [2-Fluoro-6-(2-furan-2-ylimidazo[1,2-*a*]pyridin-6-yl)phényl]méthanol ;
- [3-(2-Benzo[*b*]thiényl-2-ylimidazo[1,2-*a*]pyridin-6-yl)-2,6-difluorophényl]méthanol ;
- {2,6-Difluoro-3-[2-(1-méthyl-1*H*-benzimidazol-2-yl)imidazo[1,2-*a*]pyridin-6-yl]phényl}méthanol ;
- {3-[2-(2,3-Dihydrobenzofuran-5-yl)-imidazo[1,2-*a*]pyridin-6-yl]-2,6-difluorophényl}méthanol ;
- [2,6-Difluoro-3-(2-furan-2-ylimidazo[1,2-*a*]pyridin-6-yl)phényl]méthanol ;
- [3-(2-Benzofuran-5-ylimidazo[1,2-*a*]pyridin-6-yl)-2,6-difluorophényl]méthanol ;
- [3-(2-Benzo[*b*]thiényl-5-ylimidazo[1,2*-a*]pyridin-6-yl)-2,6-difluorophényl]méthanol ;
- 2-(1*H*-Indol-6-yl)-6-[3-[2-(méthoxyéthyl)oxyméthyl]phényl]imidazo[1,2-*a*]pyridine ;
- 2-[3-(2-Benzo[*d*]isoxazol-3-ylimidazo[1,2-*a*]pyridin-6-yl)phényl]propan-2-ol;
- [2-(2-Benzo[*d*]isoxazol-3-ylimidazo[1,2-*a*]pyridin-6-yl)-6-fluorophényl]méthanol ;
- 2-{3-[2-(1*H*-Indol-5-yl)imidazo[1,2-*a*]pyridin-6-yl]phényl}propan-2-ol ;
- 2-{3-[2-(1*H*-Indol-4-yl)imidazo[1,2-*a*]pyridin-6-yl]phényl}propan-2-ol ;
- 2-{3-[2-(2-Méthoxypyridin-3-yl)imidazo[1,2-*a*]pyridin-6-yl]phényl}propan-2-ol ;
- 2-{3-[2-(4-Méthylthién-3-yl)imidazo[1,2-*a*]pyridin-6-yl]phényl}propan-2-ol ;
- 2-{3-[2-(1-Méthyl-1*H*-indol-5-yl)imidazo[1,2-*a*]pyridin-6-yl]phényl}propan-2-ol;
- 2-[3-(2-Quinolin-5-ylimidazo[1,2-*a*]pyridin-6-yl)phényl]propan-2-ol ;
- 2-[3-(2-Isoquinolin-5-ylimidazo[1,2-*a*]pyridin-6-yl)phényl]propan-2-ol ;
- 2-{3-[2-(2,6-Difluoropyridin-4-yl)imidazo[1,2-*a*]pyridin-6-yl]phényl}-propan-2-ol ;
- 2-(3-{2-[1-(3-Méthylbutyl)-1*H*-pyrazol-4-yl]imidazo[1,2-*a*]pyridin-6-yl}phényl)propan-2-ol ;
- 2-[3-(2-Quinolin-3-yl-imidazo[1,2-*a*]pyridin-6-yl)phényl]propan-2-ol ;
- {2-Fluoro-6-[2-(1*H*-indol-5-yl)imidazo[1,2-*a*]pyridin-6-yl]phényl}méthanol;
- {2-Fluoro-6-[2-(6-méthoxypyridin-3-yl)imidazo[1,2-*a*]pyridin-6-yl]phényl} méthanol
- {2-Fluoro-6-[2-(3-fluoropyridin-4-yl)imidazo[1,2-*a*]pyridin-6-yl]phényl}méthanol ;
- {2-Fluoro-6-[2-(4-méthylthién-2-yl)imidazo[1,2-*a*]pyridin-6-yl]phényl}méthanol
- [2-Fluoro-6-(2-pyrimidin-5-ylimidazo[1,2-*a*]pyridin-6-yl)phényl]méthanol ;
- {2-Fluoro-6-[2-(1*H*-indol-4-yl)imidazo[1,2-*a*]pyridin-6-yl]phényl}méthanol ;
- {2-Fluoro-6-[2-(1*H*-indol-6-yl)imidazo[1,2-*a*]pyridin-6-yl]phényl}méthanol;
- {2-Fluoro-6-[2-(2-méthoxypyridin-3-yl)imidazo[1,2-*a*]pyridin-6-yl]phényl}méthanol ;
- {2-Fluoro-6-[2-(4-méthylthién-3-yl)imidazo[1,2-*a*]pyridin-6-yl]phényl}méthanol ;
- {2-Fluoro-6-[2-(1-méthyl-1*H*-indol-5-yl)imidazo[1,2-*a*]pyridin-6-yl]phényl}méthanol ;
- [2-Fluoro-6-(2-quinolin-5-ylimidazo[1,2-*a*]pyridin-6-yl)phényl]méthanol
- [2-Fluoro-6(2-isoquinolin-5-ylimidazo[1,2-*a*]pyridin-6-yl)phényl]méthanol ;
- {2-[2-(2,6-Difluoropyridin-4-yl)imidazo[1,2-*a*]pyridin-6-yl]-6-fluorophényl}méthanol ;
- (2-Fluoro-6-{2-[1-(3-méthylbutyl)-1*H*-pyrazol-4-yl]imidazo[1,2-*a*]pyridin-6-yl}phenyl)méthanol ;
- {2,6-Difluoro-3-[2-(1*H*-indol-5-yl)imidazo[1,2-*a*]pyridin-6-yl]phényl}méthanol ;
- {2,6-Difluoro-3-[2-(6-méthoxypyridin-3-yl)imidazo[1,2-*a*]pyridin-6-yl]phényl}méthanol ;
- {2,6-Difluoro-3-[2-(4-méthylthién-2-yl)imidazo[1,2-*a*]pyridin-6-yl]phényl}méthanol ;
- {2,6-Difluoro-3-[2-(1*H*-indol-6-yl)imidazo[1,2-*a*]pyridin-6-yl]phényl}méthanol ;
- {2,6-Difluoro-3-[2-(2-méthoxypyridin-3-yl)imidazo[1,2-*a*]pyridin-6-yl]phényl}méthanol ;
- {2,6-Difluoro-3-[2-(4-méthylthién-3-yl)imidazo[1,2-*a*]pyridin-6-yl]phényl}méthanol ;
- {2,6-Difluoro-3-[2-(1-méthyl-1*H*-indol-5-yl)imidazo[1,2-*a*]pyridin-6-yl]phényl}méthanol ;
- [2,6-Difluoro-3-(2-quinolin-5-ylimidazo[1,2-*a*]pyridin-6-yl)phényl]méthanol ;
- [2,6-Difluoro-3-(2-isoquinolin-5-ylimidazo[1,2-*a*]pyridin-6-yl)phényl]méthanol ;
- {3-[2-(2,6-Difluoropyridin-4-yl)imidazo[1,2-*a*]pyridin-6-yl]-2,6-difluorophényl}méthanol ;
- (2,6-Difluoro-3-{2-[1-(3-méthylbutyl)-1*H*-pyrazol-4-yl]imidazo[1,2-*a*]pyridin-6-yl}phényl)méthanol ;
- *N*-*tert*-Butyl-5-[6-(2,4-difluoro-3-hydroxyméthylphényl)imidazo[1,2-*a*]pyridin-2-yl]nicotinamide ;

Conformément à l'invention, on peut préparer les composés de formule générale (I) selon le procédé décrit dans le schéma 1.

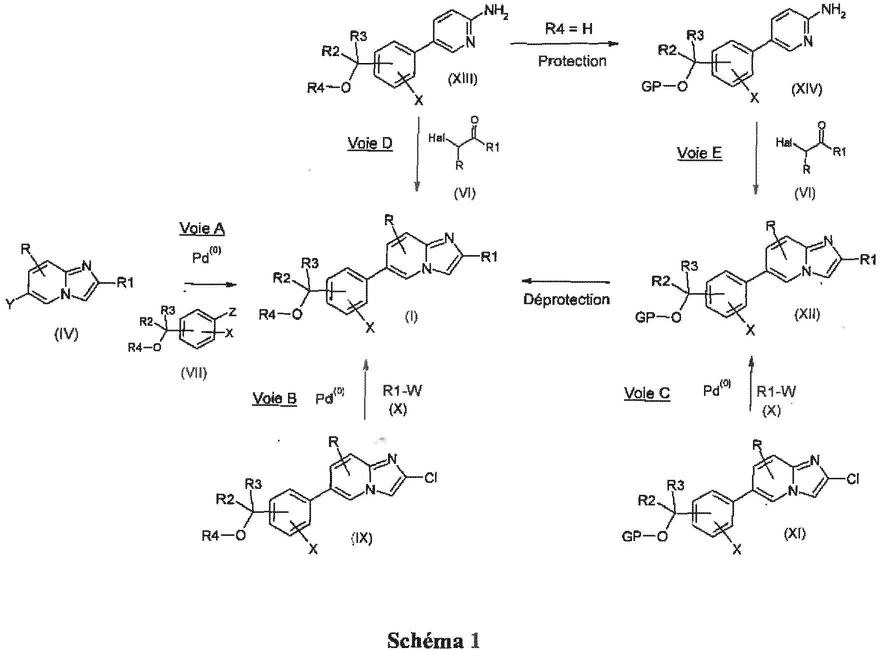

On peut préparer les composés de l'invention suivant le schéma 1 (voie A) par une réaction de couplage, catalysée par un métal tel que le palladium, entre une imidazopyridine de formule générale (IV), dans laquelle R et R1 sont définis comme précédemment et Y représente un atome d'halogène ou un dérivé de bore, et un dérivé de formule générale (VII), dans laquelle R2, R3, R4 et X sont définis comme précédemment et Z représente un dérivé de bore ou d'étain si Y représente un atome d'halogène ou un halogène si Y représente un dérivé de bore, pour obtenir les composés de formule générale (I), par exemple selon la méthode décrite par A. Gueiffier dans Helv. Chim. Acta 2001, 84, 3610-3615.

Alternativement, on peut préparer les composés de l'invention suivant le schéma 1 (voie B) par une réaction de couplage, catalysée par un métal tel que le palladium, entre une 2-chloroimidazopyridine de formule générale (IX), dans laquelle R, R2, R3, R4 et X sont définis comme précédemment, et un dérivé de formule générale (X), dans laquelle R1 est défini comme précédemment et W représente un dérivé de bore ou d'étain, pour obtenir les composés de formule générale (I), par exemple selon la méthode décrite par S. Buchwald dans J.A.C.S. 2005, 127, 4685.

Selon le schéma 1 (voie C), les dérivés de formule générale (XI), dans laquelle R, R2; R3 et X sont définis comme précédemment et PG représente un groupement protecteur de fonction hydroxyle, tel que décrit par exemple par T. Greene dans « Protective Groups in Organic Synthesis » (Wiley Interscience*)* peuvent être soumis à une réaction de couplage, catalysée par un métal tel que le palladium, avec un dérivé de formule générale (X), dans laquelle R1 est défini comme précédemment et W représente un dérivé de bore ou d'étain, pour obtenir les composés de formule générale (XII), par exemple selon la méthode décrite par S. Buchwald dans J.A.C.S. 2005, 127, 4685. Finalement, les composés de formule générale (XII) peuvent être soumis à une réaction de déprotection, telle que décrite par exemple par T. Greene dans « Protective Groups in Organic Synthesis » (Wïley Interscience*),* ou par toute autre méthode connue de l'Homme du métier pour obtenir les composés de formule générale (I).

Alternativement, on peut préparer les composés de l'invention suivant le schéma 1 (voie D) par condensation entre une aminopyridine de formule générale (XIII), dans laquelle R2, R3, R4 et X sont définis comme précédemment, R4 n'étant pas un atome d'hydrogène, et une halogénocétone de formule générale (VI), dans laquelle R et R1 sont définis comme précédemment pour obtenir les composés de formule générale (I), pour lesquels R est en position 3 du noyau imidazopyridine.

Lorsque R4 représente un atome d'hydrogène, on peut transformer les composés de formule générale (XIII) selon le schéma 1 (voie E) en dérivés de formule générale (XIV), dans laquelle R2, R3 et X sont définis comme précédemment et PG représente un groupement protecteur de fonction hydroxyle, tel que décrit par exemple par T. Greene dans « Protective Groups in Organic Synthesis » (Wiley Interscience*),* ou par toute méthode connue de l'Homme du métier. Les composés de formule générale (XIV) peuvent être condensés avec une halogénocétone de formule générale (VI), dans laquelle R et R1 sont définis comme précédemment, pour obtenir les composés de formule générale (XII), pour lesquels R est en position 3 du noyau imidazopyridine. Finalement, les composés de formule générale (XII) peuvent être soumis à une réaction de déprotection, telle que décrite par exemple par T. Greene dans « protective Groups in Organic Synthesis » (Wiley Interscience*),* ou par toute autre méthode connue de l'Homme du métier pour obtenir les composés de formule générale (I).

Conformément à l'invention, on peut préparer les intermédiaires des composés de formule générale (I) selon les procédés décrits dans le schéma 2.

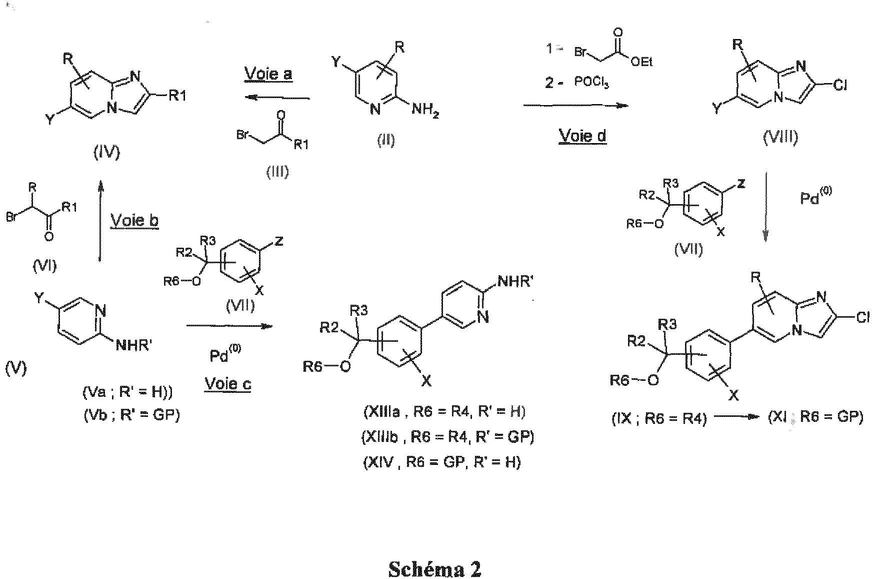

Selon le schéma 2 (voie a), les composés de formule générale (IV) peuvent être obtenus par condensation entre une aminopyridine de formule générale (II), dans laquelle R est défini comme précédemment et Y représente un atome d'halogène ou un dérivé de bore, et une halogénocétone de formule générale (III), dans laquelle R1 est défini comme précédemment par exemple selon les méthodes décrites par L. Cai dans J. Med. Chem. 2007, 50, 4746*,* pour obtenir les composés de formule générale (IV), pour lesquels R est en position 5, 7 ou 8 du noyau imidazopyridine.

Alternativement, selon le schéma 2 (voie b), les composés de formule générale (IV) peuvent être obtenus par condensation entre une aminopyridine de formule générale (V), dans laquelle Y représente un atome d'halogène ou un dérivé de bore, et une halogénocétone de formule générale (VI), dans laquelle R et R1 sont définis comme précédemment pour obtenir les composés de formule générale (IV), pour lesquels R est en position 3 du noyau imidazopyridine.

Selon le schéma 2 (voie c), les composés de formule générale (XIIIa), (XIIIb) ou (XIV) peuvent être obtenus par une réaction de couplage, catalysée par un métal tel que le palladium, entre une aminopyridine de formule générale (Va) ou (Vb) protégée par un groupe protecteur R' dans laquelle Y représente un atome d'halogène ou un dérivé de bore, et un dérivé de formule générale (VII), dans laquelle R2, R3 et X sont définis comme précédemment, R6 représente R4 (composés XIIIa et XIIIb) ou un groupe protecteur de fonction hydroxyle PG (composés XIV) et Z représente un dérivé de bore ou d'étain si Y représente un halogène ou un halogène si Y représente un dérivé de bore.

Selon le schéma 2 (voie d), les composés de formule générale (VIII) peuvent être obtenus à partir des composés de formule générale (II), par exemple selon la méthode décrite par C. Townsend dans Syn. Commun. 1997, 27, 1763-1765. Les composés de formule générale (IX) ou (XI) dans laquelle R, R2, R3 et X sont définis comme précédemment et R6 représente un groupe R4 (composés IX) ou un groupe protecteur PG (composés XI), peuvent être obtenus par une réaction de couplage, catalysée par un métal tel que le palladium, entre une imidazopyridine de formule générale (VIII), dans laquelle R est défini comme précédemment et Y représente un atome d'halogène ou un dérivé de bore, et un dérivé de formule générale (VII), dans laquelle R2, R3 et X sont définis comme précédemment, R6 représente un groupe R4 ou un groupe protecteur PG et Z représente un dérivé de bore ou d'étain si Y représente un atome d'halogène ou un halogène si Y représente un dérivé de bore, par exemple selon la méthode décrite par A. Gueiffier dans Helv. Chim. Acta 2001, 84, 3610-3615.

D'une façon générale, les intermédiaires ci-dessus peuvent être soumis, si désiré et si nécessaire, à toutes réactions de protection / déprotection connues de l'homme de l'art avant et / ou après toutes réactions décrites dans les schémas ci-dessus.

Les produits de formule (I), peuvent être soumis, si désiré et si nécessaire, à toutes réactions connues de l'homme de l'art, dans un ordre quelconque, pour être transformés en d'autres produits de formule (I),

A titre d'exemples de réactions, on peut citer : les réactions d'estérification ou d'amidification de fonction acide, les réactions de carbamoylation, les réactions d'hydrolyse de fonction ester, les réactions de transformation de fonction hydroxyle en fonction alcoxy, les réactions de couplage catalysées par un métal de transition, les réactions de protection des fonctions réactives, les réaction d'élimination des groupements protecteurs que peuvent porter les fonctions réactives protégées, les réactions de salification par un acide minéral ou organique ou par une base pour obtenir le sel correspondant, les réactions de dédoublement des formes racémiques en énantiomères, lesdits produits de formule (I) ainsi obtenus étant le cas échéant sous toutes les formes isomères possibles racémiques, énantiomères et diastéréoisomères.

Dans les schémas 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet les composés de formules (VIII-1), (IX-1), (IX-2), (XI-1) (XI-2), (XI-3), (XI-4), (XIII-1), (XIII-2), (XIV-1), (XIV-2) et (XIV-3). Ces composés sont utiles comme intermédiaires de synthèse des composés de formule (I).

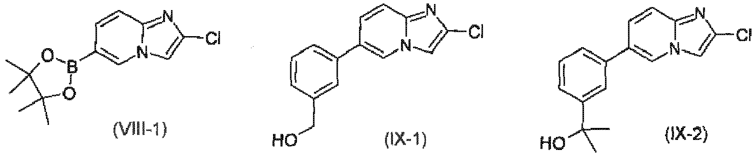

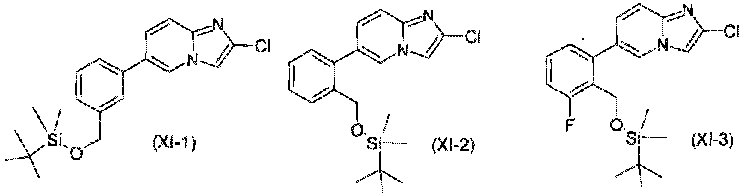

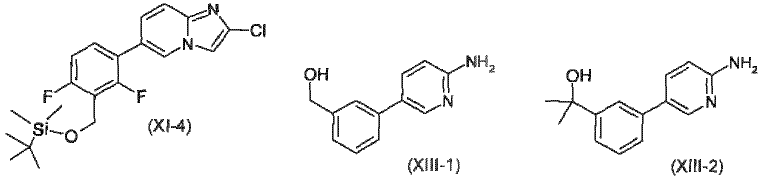

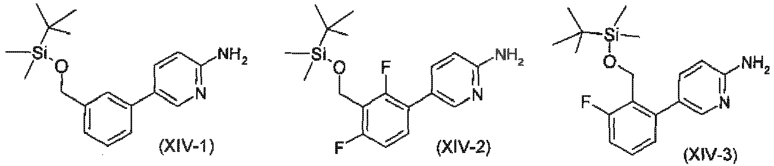

Le composé de formule (VIII-1) peut être préparé, par exemple, selon le procédé décrit dans l'exemple N° 3. Dans une première étape, on peut effectuer une condensation entre une aminopyridine substituée par un dérivé de bore telle que, par exemple, la 5-(4,4,5,5-tétraméthyl-1,3,2-dioxaborolan-2-yl)pyridin-2-ylamine et le 2-bromoacétate d'éthyle. Dans une deuxième étape, le composé est soumis à une réaction de cyclisation et de chloration en présence d'un agent chlorant tel que l'oxychlorure de phosphore qui conduit au composé (VIII-1).

Le composé de formule (IX-1) peut être préparé par une réaction de couplage, catalysée par un métal, tel que le palladium, entre la 6-bromo-2-choroimidazo[1,2-*a*]pyridine et l'acide 3-(hydroxyméthyl)phénylboronique, tel que décrit dans l'exemple N° 2.

Le composé de formule (IX-2) peut être préparé par une réaction de couplage, catalysée par un métal, tel que le palladium, entre le composé de formule (VIII-1) et le 2-(3-bromophényl)propan-2-ol, tel que décrit dans l'exemple N°7

Le composé de formule (XI-1) peut être préparé par action du *tert-*butylchlorodiméthylsilane sur le composé de formule (IX-1), en présence d'une base telle que l'imidazole et dans un solvant tel que le tétrahydrofurane, tel que décrit dans l'exemple N° 4,

Le composé de formule (XI-2) peut être préparé par une réaction de couplage, catalysée par un métal, tel que le palladium, entre le (2-bromobenzyloxy)*tert-*butyldiméthylsilane et le composé de formule (VIII-1), tel que décrit dans l'exemple N° 3.

Le composé de formule (XI-3) peut être préparé par une réaction de couplage, catalysée par un métal, tel que le palladium, entre le (2-bromo-6-fluorobenzyloxy)*tert-*butyldiméthylsilane et le composé de formule (VIII-1), tel que décrit dans l'exemple N° 13.

Le composé de formule (XI-4) peut être préparé par une réaction de couplage, catalysée par un métal, tel que le palladium, entre le (3-bromo-2,6-difluorobenzyloxy)*tert-*butyldiméthylsilane et le composé de formule (VIII-1), tel que décrit dans l'exemple N° 14.

Le composé de formule (XIII-1) peut être préparé par une réaction de couplage catalysée par un métal, tel que le palladium, entre la 5-bromo-2-aminopyridine et un dérivé d'acide boronique, par exemple l'acide 3-(hydroxyméthyl)phénylboronique, tel que décrit dans l'exemple N°6.

Le composé de formule (XIII-2) peut être préparé par une réaction de couplage, catalysée par un métal, tel que le palladium, entre la 5-(4,4,5,5-tétraméthyl-1,3,2-dioxaborolan-2-yl)pyridin-2-ylamine et le 2-(3-bromophényl)propan-2-ol, tel que décrit dans l'exemple N°8

Le composé de formule (XIV-1) peut être préparé par action du (*tert-*butylchlorodiméthyl)silane sur le composé de formule (XIII-1) dans un solvant , par exemple le tétrahydrofurane, en présence d'une base telle que l'imidazole, tel que décrit dans l'exemple N°6.

Le composé de formule (XIV-2) peut être préparé par une réaction de couplage, catalysée par un métal, tel que le palladium, entre la 5-(4,4,5,5-tétraméthyl-1,3,2-dioxaborolan-2-yl)pyridin-2-ylamine et le 3-(*tert*butyldiméthylsilyloxyméthyl)-2,4-difluorobromobenzène, tel que décrit dans l'exemple N°10

Le composé de formule (XIV-3) peut être préparé par une réaction de couplage, catalysée par un métal, tel que le palladium, entre la 5-(4,4,5,5-tétraméthyl-1,3,2-dioxaborolan-2-yl)pyridin-2-ylamine et le 2-(*tert*butyldiméthylsilyloxyméthyl)-3-fluorobromobenzène, tel que décrit dans l'exemple N°9

Les composés de formules (VIII-1), (IX-1), (XI-1), (XI-2), (XIII-1) et (XIV-1) ont été préparés à l'état de poudre ou d'huile, à l'état de base ou de sel. Le tableau 1 rassemble quelques données physicochimiques de ces intermédiaires.

**Tableau 1**

| N° | R.M.N. ¹H (DMSO-d6, δ ppm) ; M+H ; PF |
|---|---|
| (VIII-1) | 1,35 (m, 12H) ; 7,4 (d, 1H) ; 7,5 (d, 1H) ; 8,1 (s, 1H), 8,85 (s, 1H) ; M+H = 279 ; PF = 115 - 120°C. |
| (IX-1) | 4,6 (d, 2H) ; 5,3 (t, 1H) ; de 7,35 à 7,75 (m, 6H) ; 8,1 (s, 1H) ; 8,85 (s, 1H) ; M+H = 259. |
| (IX-2) | 1,5 (s, 6H) ; 5,1 (s, 1H) ; 7,45 (m, 1H) ; 7,55 (m, 2H) ; de 7,6 à 7,7 (m, 2H) ; 7,8 (s, 1H) ; 8,05 (s, 1H) ; 8,85 (s, 1H). |
| (XI-1) | 0,10 (s, 6H) ; 0,92 (s, 9H) ; 4,79 (s, 2H) ; 7,37 (d, 1H) ; de 7,55 à 7,67 (m, 5H) ; 8,05 (s, 1H) ; 8,84 (t, 1H) ; M+H = 373 ; PF = 120 - 123°C. |
| (XI-2) | (CDCl₃) : 0 (s, 6H) ; 0,85 (s, 9H) ; 4,5 (s, 2H) ; de 7,15 à 7,55 (m, 7H) ; 8,15 (s, 1H) ; M+H = 373. |
| (XI-3) | (CDCl3) : 0 (s, 6H) ; 0,85 (s, 9H) ; 4,5 (s, 2H) ; de 7,05à 7,1 (m, 2H) ; de 7,25 à 7,3 (m, 2H) ; 7,4 (s, 1H) ; 7,45 (s, 1H) ; 8,3 (s, 1H). M+H = 391. |
| (XI-4) | 0,0 (s, 6H) ; 0,8 (s, 9H) ; 4,7 (s, 2H) ; 7,15 (t, 1H) ; 7,4 (d, 1H) ; de 7,5 à 7,6 (m, 2H) ; 8,0 (s, 1H) ; 8,65 (s, 1H). M+H = 409. |
| (XIII-1) | 4,55 (d, 2H) ; 5,2 (t, 1H) ; 6,05 (s, 2H) ; 6,55 (d,1H) ; 7,2 (d, 1H) ; de 7,3 à 7,55 (m, 3H) ; 7,7 (d, 1H) ; 8,25 (s, 1H) ; M+H = 201 |
| (XIII-2) | 1,45 (s, 6H) ; 5,0 (s, 1H) ; 6,0 (s, 2H) ; 6,55 (d, 1H) ; de 7,3 à 7,4 (m, 3H) ; 7,65 (s, 1H) ; 7,7 (d, 1H) ; 8,25 (s, 1H). M+H = 229 |
| (XIV-1) | 0,09 (s, 6H) ; 0,91 (s, 9H) ; 4,75 (s, 2H) ; 6,02 (m, 2H) ; 6,52 (dd, 1H) ; 7,21 (d, 1H) ; 7,36 (t, 1H) ; 7,43 (d, 1H) ; 7,47 (s, 1H) ; 7,66 (dd, 1H) ; 8,21 (d, 1H). M+H = 315 ; PF = 82 - 84°C |
| (XIV-2) | 0 (s, 6H) ; 0,8 (s, 9H) ; 4,7 (s, 2H) ; 6,05 (s, 2H) ; 6,45 (d, 1H) ; 7,05 (t, 1H) ; de 7,35 à 7,45 (m, 2H) ; 8,0 (s, 1H). M+H = 351 |
| (XIV-3) | 0 (s, 6H) ; 0,85 (s, 9H) ; 4,5 (s, 2H) ; 6,05 (s, 2H) ; 6,45 (d, 1H) ; de 7,05 à 7,15 (m, 2H) ; de 7,3 à 7,4 (m, 1H) ; de 7,45 à 7,5 (m, 1H) ; 8,0 (d, 1H). M+H = 333 |

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

La nomenclature des composés a été établie à partir du logiciel Autonom.

### Exemple 1: Chlorhydrate (2:1) de 6-(3-tert-butoxyméthylphényl)-2-(pyridin-3-yl)imidazo[1,2-a] pyridine (composé 3 du tableau)

### 1.1 N-[5-(3-tert-Butoxyméthylphényl)pyridin-2-yl]acétamide

Sous un courant d'argon, 1,0 g de *N*-(5-bromopyridi-n-2ryl)acétamide, 967 mg d'acide 3-(*tert*-butoxyméthyl)phénylboronique et 269 mg de tétrakis(triphénylphosphine)palladium sont placés dans un ballon contenant 30 ml de diméthoxyéthane et 15 ml d'une solution 2M de carbonate de sodium préalablement dégazés. On chauffe à 90°C pendant 15 heures. Après refroidissement, le mélange réactionnel est concentré sous pression réduite. Le résidu est repris entre le dichlorométhane et l'eau, la phase organique est séparée, séchée sur sulfate de sodium et le filtrat est concentré sous pression réduite. Le résidu est ensuite purifié par chromatographie sur gel de silice en éluant avec un mélange dichlorométhane/méthanol. Le solide obtenu est trituré à l'éther diéthylique, recueilli par filtration puis séché. On obtient 1,0 g de composé.
Spectre RMN 1H (DMSO-d6, δ en ppm) : 1,4 (s, 9H) ; 2,3 (s, 3H) ; 4,55 (s, 2H) ; de 7,4 à 7,55 (m, 3H) ; 7,6 (s, 1H) ; 8,05 (m, 1H) ; de 8,2 à 8,4 (m, 2H) ; 8,55 (m, 1H). M+H = 299.

### 1.2 5-(3-tert-Bntoayméthylphényl)pyridin-2-ylamine

559 mg d'hydroxyde de sodium dans 2 ml d'éthanol et 2 ml d'eau sont placés dans un ballon contenant 695 mg de *N*-[5-(3-*tert*-butoxyméthylphényl)pyridin-2-yl]acétamide. On y ajoute 1 ml d'eau et 1 ml d'éthanol et on chauffe le mélange au reflux du solvant pendant 1 heure. Après refroidissement, le mélange réactionnel est concentré sous pression réduite. Le résidu est repris entre le dichlorométhane et l'eau puis la phase organique est séparée, séchée sur sulfate de magnésium et concentrée sous pression réduite. Le résidu est purifié par chromatographie sur gel de silice en éluant avec un mélange dichlorométhane/méthanol. On obtient 487 mg de composé.
Spectre RMN 1H (DMSO-d6, δ en ppm) : 1,25 (s, 9H) ; 4,45 (s, 2H) ; 6,05 (s, 2H) ; 6,55 (d, 1H) ; 7,25 (d, 1H) ; 7,35 (t, 1H) ; 7,45 (d, 1H) ; 7,5 (s, 1H) ; 7,7 (m, 1H) ; 8,25 (s, 1H). M+H = 257.

### 1.3 6-(3-tert-Butoxyméthylphényl)-2-pyridin-3-ylimidazo[1,2-a]pyridine

On place dans un ballon 487 mg de 5-(3-*tert*-butoxyméthylphényl)pyridin-2-ylamine (obtenu selon le protocole décrit dans l'exemple 1.2), 380 mg de 2-bromo-1-pyridin-3-yléthanone dans 15 ml de n-propanol. On ajoute 223 mg d'hydrogénocarbonate de sodium. On chauffe à 80°C durant 24 heures, laisse refroidir à température ambiante et concentre le mélange réactionnel sous pression réduite. Le résidu est repris entre l'eau et l'acétate d'éthyle. La phase organique est séparée, séchée et le filtrat est concentré sous pression réduite. Le résidu est purifié par chromatographie sur gel de silice en éluant avec un mélange dichlorométhane/méthanol. On obtient 128 mg de composé.
M+H = 358.

### 1.4 Chlorhydrate (2:1) de 6-(3-tert-butoxyméthylphényl)-2-(pyridin-3-yl)imidazo[1,2-a] pyridine

121 mg de 6-(3-*tert*-butoxyméthylphényl)-2-pyridin-3-ylimidazo[1,2-*a*]pyridine sont mis en suspension dans 5 ml de dichlorométhane ; on y ajoute, goutte à goutte et sous agitation, 7,48 ml d'une solution 0,1N d'acide chlorhydrique dans le 2-propanol et agite à température ambiante pendant 7 heures. Le mélange est concentré sous pression réduite. Le solide obtenu est trituré à l'éther diéthylique, recueilli par filtration et séché à l'étuve sous pression réduite à 40°C. Le solide est ensuite dissous à température ambiante dans un minimum d'isopropanol puis reprécipité à l'aide d'éther isopropylique. Le précipité formé (après 3h au frigo) est recueilli par filtration, lavé au pentane puis séché à l'étuve sous pression réduite à 50°C. On obtient 60 mg de composé.
PF = 220 - 222°C. Spectre RMN 1H (DMSO-d6, δ en ppm) : 1,3 (s, 9H) ; 4,5 (s, 2H) ; 7,45 (d, 1H) ; 7,55 (t, 1H) ; de 7,65 à 7,75 (m, 2H) ; de 7,85 à 8,0 (m, 2H) ; 8,05 (d, 1H) ; de 8,75 à 8,90 (m, 3H) ; 9,15 (s, 1H) ; 9,4 (s, 1H). M+H = 358.

### Exemple 2 : Chlorhydrate (2:1) de [3-(2-Quinoléin-3-ylimidazo[1,2-a]pyridin-6-yl)phényl]méthanol (composé 5 du tableau)

### 2.1 6-Bromo-3H-imidazo[1,2-a]pyridin-2-one

3,0 g de 5-bromopyridin-2-ylamine dans 5,8 ml de 2-bromoacétate d'éthyle sont placés dans un ballon et agités à température ambiante pendant 48 h. Il se forme un précipité que l'on recueille par filtration, lave à l'éther diéthylique et sèche à l'étuve sous pression réduite. Le solide est ensuite repris dans 50 ml d'éthanol et on y ajoute 2,18 g d'hydrogénocarbonate de sodium. Le mélange réactionnel est chauffé au reflux du solvant pendant 5 h, refroidi à température ambiante puis concentré sous pression réduite. Le résidu obtenu est repris entre l'eau et le dichlorométhane et la phase organique est séparée, séchée et concentrée sous pression réduite. On obtient 1,46 g de composé utilisé tel quel dans les étapes suivantes.
M+H = 214.

### 2.2 6-Bromo-2-chloroimidazo[1,2-a]pyridine

Un mélange de 1,4 g de 6-bromo-3*H*-imidazo[1,2-*a*]pyridin-2-one dans 5 ml de POCl₃ est chauffé à 105°C pendant 2 heures puis refroidi à température ambiante et concentré sous pression réduite. Le résidu est repris entre l'eau et le dichlorométhane et on ajoute une solution aqueuse à 30% de NH₄OH jusqu'à pH basique. La phase organique est ensuite séparée, séchée sur sulfate de magnésium et concentrée sous pression réduite. Puis le résidu est purifié par chromatographie sur gel de silice en éluant par un mélange dichlorométhane/méthanol. On obtient 760 mg de composé.
Spectre RMN 1H (DMSO-d6, δ en ppm) : 7,35 (d, 1H) ; 7,55 (m, 2H) ; 8,3 (s, 1H). M+H = 232.

### 2.3 [3-(2-Chloroimidazo[1,2-a]pvridin-6-yl)phényl]méthanol

Dans un ballon, on place 760 mg de 6-bromo-2-chloroimidazo[1,2-a]pyridine dans 25 ml de toluène et 8 ml d'éthanol et on dégaze à l'argon pendant 10 mn. Après addition de 230 mg de tétrakis(triphénylphosphine)palladium, on agite 5 mn à température ambiante puis on ajoute 650 mg d'acide 3-(hydroxyméthyl)phénylboronique et 8 ml d'une solution 2M de carbonate de sodium. Le mélange réactionnel est chauffé à 80°C pendant 16 h puis refroidi à température ambiante et concentré sous pression réduite. Le résidu est repris entre l'eau et l'acétate d'éthyle et la phase organique est séparée, séchée sur sulfate de magnésium et concentrée sous pression réduite. L'huile obtenue est purifiée par chromatographie sur gel de silice en éluant par un mélange heptane/acétate d'éthyle. On obtient 550 mg de composé. Spectre RMN 1H (DMSO-d6, δ en ppm) : 4,6 (d, 2H) ; 5,3 (t, 1H) ; de 7,35 à 7,75 (m, 6H) ; 8,1 (s, 1H) ; 8,85 (s, 1H). M+H = 259.

### 2.4 [3-[2-(Quinoléin-3-yl)imidazo[1,2-a]pyridin-6-yl]phényl]méthanol

Dans un réacteur à vis, on place 550 mg de [3-(2-chloroimidazo[1,2-a]pyridin-6-yl)phényl]méthanol, 550 mg d'acide 3-quinoléineboronique, 20 mg d'acétate de palladium, 70 mg de 2-dicyclohexylphosphino-2',6'-diméthoxy-1,1'-biphényl et 900 mg de K₃PO₄ dans 8 ml de toluène anhydre. Le réacteur est fermé et on chauffe à 115°C pendant 16 h. Après refroidissement, le mélange réactionnel est filtré sur célite et rincé avec du dichlorométhane. La phase organique est lavée par une solution saturée de NaCl, puis séparée, séchée et concentrée sous pression réduite. Le résidu est purifié par chromatographie sur gel de silice en éluant avec un mélange dichlorométhane/méthanol. On obtient 90 mg de composé.
M+H = 351.

### 2.5 Chlorhydrate (2:1) de [3-[2-(quinoléin-3-yl)imidazo[1,2-a]pyridin-6-yl] phényl] méthanol

Une solution de 90 mg de [3-[(2-quinoléin-3-yl)imidazo[1,2-*a*]pyridin-6-yl]phényl]méthanol dans du dichlorométhane et du méthanol est passée sur verre fritté puis on ajoute au filtrat 5,2 ml d'une solution 0,1N d'acide chlorhydrique dans l'isopropanol. Il se forme un précipité que l'on recueille par filtration et lave à l'éther diéthylique. Le solide est ensuite dissous à température ambiante par le minimum de méthanol et repris ensuite par de l'éther diéthylique. Le précipité est recueilli par filtration et séché à l'étuve sous pression réduite. On obtient 66 mg de composé.
PF = 279 - 281°C. Spectre RMN 1H (DMSO-d6, δ en ppm) : 4,65 (s, 2H) ; 7,45 (d, 1H) ; 7,55 (t, 1H) ; 7,7 (d, 1H) ; 7,8 (m, 2H) ; 7,95 (m, 1H) ; 8,0 (m, 1H) ; 8,1 (m, 1H) ; 8,2 (m, 2H) ; 8,95 (s, 1H) ; 9,15 (s, 1H) ; 9,2 (s, 1H) ; 9,6 (s, 1H). M+H = 445.

### Exemple 3 : Chlorhydrate (1:1) de [2-[2-(furan-3-yl)imidazo[1,2-a]pyridin-6-yl]phényl]méthanol (composé 12 du tableau)

### 3.1 Bromhydrate de [2-imino-5-(4,4,5,5-tetraméthyl-1,3,2-dioxaborolan-2-yl)-2H-pyridin-1-yl]acétate d'éthyle

5,0 g de 5-(4,4,5,5-tétraméthyl-1,3,2-dioxaborolan-2-yl)pyridin-2-ylamine dans 7,6 ml de 2-bromoacétate d'éthyle sont placés dans un ballon et le mélange est agité à température ambiante pendant 20 h. Il se forme un précipité que l'on recueille par filtration, lave par de l'éther diéthylique puis par de l'éthanol et sèche à l'étuve sous pression réduite. On obtient 8,78 g de composé.
Spectre RMN 1H (DMSO-d6, δ en ppm) : 1,3 (m, 15H) ; de 4,1 à 4,25 (m, 2H) ; 5,2 (s, 2H) ; 7,1 (d, 1H) ; 8,0 (d, 1H) ; 8,3 (s, 1H) ; 9,0 (s, 1H). M+H = 388.

### 3.2 2-Chloro-6-(4,4,5,5-tétraméthyl-1,3,2-dioxaborolan-2-yl)imidazo[1,2-a]pyridine

Dans un ballon on place 8,78 g de composé obtenu selon le protocole décrit en 3.1 dans 20 ml de POCl₃ Le mélange réactionnel est chauffé à 105°C pendant 16h, refroidi à température ambiante et concentré sous pression réduite. Le résidu est repris entre le dichlorométhane et l'eau à 0°C et on ajoute une solution aqueuse à 30% de NH₄OH jusqu'à pH basique. La phase organique est séparée, séchée sur sulfate de magnésium et concentrée sous pression réduite. On obtient 4,3 g de composé.
PF = 115 - 120°C. Spectre RMN 1H (DMSO-d6, δ en ppm) : 1,35 (m, 12H) ; 7,4 (d, 1H) ; 7,5 (d, 1H) ; 8,1 (s, 1H) ; 8,85 (s, 1H). M+H = 279.

### 3.3 (2-Bromobenzyloxy)tert-butyldiméthylsilane

2,0 g de (2-bromophényl)méthanol sont placés dans un ballon et dissous dans 100 ml de tétrahydrofurane. On y ajoute 1,1 g d'1*H*-imidazole puis 2,1 g de chlorure de *tert-*butyldiméthylsilane et on laisse agiter à température ambiante pendant 48 heures. Le mélange réactionnel est ensuite hydrolysé avec de l'eau et la phase organique, extraite à l'acétate d'éthyle, est séparée, séchée sur sulfate de magnésium et concentrée sous pression réduite. Le résidu obtenu est purifié par chromatographie sur gel de silice en éluant avec un mélange heptane/acétate d'éthyle. On obtient 2,66 g de composé.
Spectre RMN 1H (CDCl₃, δ en ppm) : 0 (s, 6H) ; 0,8 (s, 9H) ; 4,6 (s, 2H) ; 6,95 (m, 1H) ; 7,2 (m, 1H) ; de 7,35 à 7,45 (m, 2H). M+H = 302.

### 3.4 6-[2-[(tert-Butyldiméthylsilanyl)oxyméthyl]phényl-2-chloroimidazo[1,2-a]pyridine

450 mg de (2-bromobenzyloxy)*tert*-butyldiméthylsilane, 500 mg de 2-chloro-6-(4,4,5,5-tétraméthyl-1,3,2-dioxaborolan-2-yl)imidazo[1,2-*a*]pyridine et 1,46 g de carbonate de césium sont dissous dans 8 ml de tétrahydrofurane et 1 ml d'eau. On dégaze à l'argon pendant 10 min, on ajoute 110 mg de [1,1'-bis(diphénylphosphino)ferrocène]dichloropalladium et on chauffe au reflux du solvant pendant 16 heures. Après refroidissement, le mélange réactionnel est hydrolysé avec de l'eau et la phase organique extraite au dichlorométhane est séparée, séchée et concentrée sous pression réduite. L'huile obtenue est purifiée par chromatographie sur gel de silice en éluant avec un mélange heptane/acétate d'éthyle. On obtient 260 mg de composé.
Spectre RMN 1H (CDCl₃, δ en ppm) : 0 (s, 6H) ; 0,85 (s, 9H) ; 4,5 (s, 2H) ; de 7,15 à 7,55 (m, 7H) ; 8,15 (s, 1H). M+H = 373.

### 3.5 6-[2-[(tert-Butyldiméthylsilanyl)oxyméthyl]phényl]-2-(furan-3-yl)imidazo[1,2-a]pyridine

3 mg d'acétate de palladium, 11 mg de 2-(dicyclohexyl)phosphino-2',6'-diméthoxy-1,1'-biphényl, 270 mg de K₃PO₄, 110 mg d'acide 3-furaneboronique et quelques gouttes d'éthanol sont placés dans un réacteur contenant un mélange de 240 mg de 6-[2-[(*tert-*butyldiméthylsilanyl)oxyméthyl]phényl]-2-chloroimidazo[1,2-*a*]pyridine dans 2 ml de toluène prélablement dégazé sous courant d'argon. Le réacteur est fermé et chauffé à 115°C pendant 16 h. Après refroidissement, le mélange réactionnel est filtré sur célite, lavé au dichlorométhane puis concentré sous pression réduite. Le résidu est purifié par chromatographie sur gel de silice en éluant par un mélange dichlorométhane/méthanol. On obtient 258 mg de composé.
Spectre RMN 1H (CDCl₃, δ en ppm) : 0 (s, 6H) ; 0,85 (s, 9H) ; 4,55 (s, 2H) ; 6,75 (m, 1H) ; de 7,1 à 7,6 (m, 8H) ; 7,95 (s, 1H) ; 8,15 (s, 1H).M+H = 405.

### 3.6 [2-[2-(Furan-3-yl)imidazo[1,2-a]pyridin-6-yl]phényl]méthanol

On place dans un ballon 250 mg de 6-[2-[(*tert-*butyldiméthylsilanyloxy)méthyl]phényl]-2-(furan-3-yl)imidazo[1,2-*a*]pyridine dans 6 ml de tétrahydrofurane et on y ajoute 320 mg de fluorure de tétrabutylammonium. Le mélange réactionnel est agité à température ambiante pendant 48 h et concentré sous pression réduite. Le résidu est purifié par chromatographie sur gel de silice en éluant avec un mélange dichlorométhane/méthanol. On obtient 156 mg de composé.
M+H = 291.

### 3.7 Chlorhydrate (1:1) de [2-(2-(furan-3-yl)imidazo[1,2-a]pyridin-6-yl)phényl]méthanol

156 mg de [2-(2-furan-3-ylimidazo[1,2-*a*]pyridin-6-yl)phényl]méthanol sont mis en suspension dans du dichlorométhane ; on y ajoute, goutte à goutte, 5,4 ml d'une solution 0,1N d'acide chlorhydrique dans l'isopropanol et agite à température ambiante. Le mélange réactionnel est ensuite concentré sous pression réduite. Le solide résiduel est repris dans l'éther diéthylique, le précipité est recueilli par filtration, lavé à l'éther diéthylique et séché à l'étuve sous pression réduite.
PF = 205 - 208°C. Spectre RMN 1H (DMSO-d6, δ en ppm) : 4,45 (s, 2H) ; 7,1 (m, 1H) ; 7,4 (m, 1H) ; 7,45 (t, 1H) ; 7,5 (t, 1H) ; 7,65 (d, 1H) ; de 7,9 à 8,0 (m, 3H) ; 8,45 (s, 1H) ; 8,55 (s, 1H) ; 8,95 (s, 1H). M+H = 327.

### Exemple 4 : Chlorhydrate (1:1) de {3-[2-(1H-indol-6-yl)imidazo[1,2-a]pyridin-6-yl]phényl}méthanol (composé N°33 du tableau)

### 4.1 Bromhydrate de (5-bromo-2-imino-1H-pyridin-1-yl)acétate d'éthyle

En procédant comme dans l'exemple 3.1 et à partir de 5,0 g de 5-bromopyridin-2-ylamine et de 9,6 ml de 2-bromoacétate d'éthyle, on obtient 9,56 g de composé.
Spectre RMN 1H (DMSO-d6, δ en ppm) : 1,3 (t, 3H) ; 4,25 (q, 2H) ; 5,15 (s, 2H) ; 7,15 (d, 1H) ; 8,1 (d, 1H) ; 8,45 (s, 1H) ; 8,95 (s, 1H) ; M+H = 341.

### 4.2 6-Bromo-2-chloroimidazo[1,2,-a]pyridine

En procédant comme dans l'exemple 3.2, en partant de 9,5 g de bromhydrate de (5-bromo-2-imino-1*H*-pyridin-1-yl)acétate d'éthyle et de 30 ml de POCl₃, on obtient 6,7 g de 6-bromo-2-chloroimidazo[1,2-a]pyridine. Le composé est purifié par chromatographie sur gel de silice en éluant avec un mélange heptane/acétate d'éthyle. On obtient 5,97 g de composé.
PF = 155 -159°C. Spectre RMN 1H (CDCl₃, δ en ppm) : 7,35 (d, 1H) ; de 7,45 à 7,6 (m, 2H) ; 8,3 (s, 1H) ; M+H = 232.

### 4.3 6-[3-[(tert-butyldiméthylsilanyloxy)méthyl]phényl]-2-chloroiniidazo[1,2-a]pyridine,

On place dans un ballon 5,7 g de [3-(2-chloroimidazo[1,2-a]pyridin-6-yl)phényl]méthanol obtenu selon le protocole décrit dans l'exemple 2.3 dans 220 ml de tétrahydrofurane, puis on ajoute 1,95 g de d'1*H*-imidazole et 3,65 g de chlorure de *tert-*butyldiméthylsilane et on laisse agiter à température ambiante pendant 48 heures. Le mélange réactionnel est ensuite hydrolysé par 150 ml d'eau et la phase organique extraite à l'acétate d'éthyle est séparée, séchée sur sulfate de magnésium et concentrée sous pression réduite. Le solide résiduel est purifié par chromatographie sur gel de silice en éluant avec un mélange heptane/acétate d'éthyle. On obtient 6,1 g de composé.
Spectre RMN 1H (CDCl₃, δ en ppm) : 0,10 (s, 6H) ; 0,92 (s, 9H) ; 4,79 (s, 2H) ; 7,37 (d, 1H) ; de 7,55 à 7,67 (m, 5H) ; 8,05 (s, 1H) ; 8,84 (t, 1H). PF = 120-123°C. M+H = 373.

### 4.4 6-[3-[(tert-butyldiméthylsilanyloxy)méthyl]phényl]-2-(1H-indol-6-yl)imidazo[1,2-a]pyridine

On place dans un réacteur 200 mg de 6-[3-[(*tert-*butyldiméthylsilanyloxy)méthyl]phényl]-2-chloroimidazo[1,2-*a*]pyridine dans 2 ml de toluène et on dégaze sous courant d'argon pendant 10 mn puis on ajoute 3 mg d'acétate de palladium, 11 mg de 2-(dicyclohexyl)phosphino-2',6'-diméthoxy-1,1'-biphényle, 110 mg d'acide 6-indoleboronique, 230 mg de K₃PO₄ et quelques gouttes d'éthanol. Le réacteur est fermé et chauffé à 115°C pendant 16 h. Après refroidissement, le mélange réactionnel est concentré sous pression réduite et le résidu obtenu est ensuite purifié par chromatographie sur gel de silice en éluant par un mélange heptane/acétate d'éthyle. On obtient 180 mg de composé.
Spectre RMN 1H (CDCl₃, δ en ppm) : 0 (s, 6H) ; 0,85 (s, 9H) ; 4,7 (s, 2H) ; 6,4 (m, 1H) ; 7,1 (m, 1H) ; de 7,2 à 7,35 (m, 4H) ; 7,4 (s, 1H) ; 7,55 (m, 2H) ; 7,8 (s, 1H) ; 8,05 (s, 1H) ; 8,15 (s, 1H) 8,35 (m, 1H) 11,5 (s, 1H) ; M+H=454.

### 4.5 {3-[2-(1H-Indol-6-yl)imidazo[1,2-a]pyridin-6-yl]phényl}niéthanol

210 mg de fluorure de tétrabutylammonium sont introduits dans un ballon contenant 180 mg de 6-[3-[(*tert*-butyldiméthylsilanyloxy)méthyl]phényl]-2-(1H-indol-6-yl)imidazo[1,2-*a*]pyridine dans 4 ml de tétrahydrofurane. Le mélange réactionnel est agité à température ambiante pendant 48 h et est concentré sous pression réduite. Le résidu est purifié par chromatographie sur gel de silice en éluant avec un mélange dichlorométane/méthanol. On obtient 117 mg de composé.
Spectre RMN 1H (DMSO-d6, δ en ppm) : 4,65 (d, 2H) ; 5,3 (t, 1H) ; 6,5 (m, 1H) ; 7,4 (m, 2H) ; 7,5 (t, 1H) ; de 7,6 à 7,75 (m, 6H) ; 8,1 (s, 1H) ; 8,4 (s, 1H) ; 8,9 (s, 1H) ; 11,2 (s, 1H). M+H = 340.

### 4.6 Chlorhydrate (1:1) de {3-[2-(1H-indol-6-yl)imidazo[1,2-a]pyridin-6-yl]phényl}méthanol

110 mg de {3-[2-(1*H*-indol-6-yl)imidazo[1,2-*a*]pyridin-6-yl]phényl}méthanol sont mis en suspension dans du dichlorométhane et du méthanol; on y ajoute, goutte à goutte, 3,2 ml d'une solution 0,1N d'acide chlorhydrique dans l'isopropanol. Le mélange réactionnel est ensuite concentré sous pression réduite. Le solide résiduel est repris dans l'éther diéthylique, le précipité est recueilli par filtration et séché à l'étuve sous pression réduite.
PF = 251 - 254°C.. Spectre RMN 1H (DMSO-d6, δ en ppm) : 4,65 (s, 2H) ; 6,55 (s, 1H) ; 7,45 (d, 1H) ; 7,55 (m, 2H) ; 7,65 (d, 1H) ; 7,7 (d, il) ; 7,75 (m, 2H) ; 8,0 (d, 1H) ; 8,1 (s, 1H) ; 8,25 (d, 1H) ; 8,7 (s, 1H) ; 9,25 (s, 1H) ; 11,6 (s, 1H). M+H = 376.

### Exemple 5 : [3-(2-(Benzofuran-3-yl)imidazo[1,2-a]pyridin-6-yl)phényl]méthanol (composé 23 du tableau)

### 5.1 2-(Benzofuran-3-yl)-6-bromoimidazo[1,2-a]pyridine

Dans un tube en verre, on dissout 250 mg de 1-(benzofuran-3-yl)-2-bromoéthanone, 181 mg de 5-bromopyridin-2-ylamine et 105 mg d'hydrogénocarbonate de sodium dans 15 ml de n-propanol. Le tube est fermé et chauffé à 80°C pendant 20h. Après refroidissement, on ajoute 20 ml d'eau et le mélange réactionnel est agité 1 heure à température ambiante. Il se forme un précipité que l'on recueille par filtration, lave à l'eau puis à l'éther diisopropylique et sèche au dessicateur sous pression réduite. On obtient 250 mg de composé.
PF = 151-153°C. RMN 1H (DMSO-d6, δ en ppm) : de 7,31 à 7,43 (m, 3H) ; de 7,53 à 7,68 (m, 2H) ; 8,15 (m, 1H) ; 8,42 (s, 1H); 8,50 (s, 1H) ; 8,87 (m, 1H). M+H = 314.

### 5.2 [3- (2-(Benzofuran-3-yi)imidazo[1,2-a]pyridin-6-yl)phényl]méthanol

100 mg de 2-(benzofuran-3-yl)-6-bromoimidazo[1,2-*a*]pyridine, 73 mg d'acide 3-(hydroxyméthyl)phénylboronique et 11 mg de tétrakis(triphénylphosphine)palladium sont placés dans un tube à micro-ondes. On y ajoute 2 ml d'acétonitrile, 2 ml de toluène et 1,5 ml d'une solution 2 M de carbonate de sodium. Le tube est placé dans un appareil à micro-ondes et irradié à 150°C pendant 15 min. La phase organique est séparée, séchée puis concentrée sous pression réduite. Le résidu huileux est repris par 3 ml de dichlorométhane et trituré pendant 1 heure. Le précipité est recueilli par filtration, lavé à l'éther diisopropylique et séché au dessicateur sous pression réduite. On obtient 44 mg de composé.
PF = 150-152°C. RMN 1H (DMSO-d6, δ en ppm) : 4,6 (d, 2H) ; 5,25 (t, 1H) ; de 7,25 à 7,75 (m, 9H) ; 8,2 (m, 1H) ; 8,45 (s, 1H) ; 8,5 (s, 1H) ; 8,9 (t, 1H). M+H = 341.

### Exemple 6 : Chlorhydrate (1:1) de [3-(3-méthyl-2-thién-2-yl-imidazo[1,2-a]pyridin-6-yl)phényl]méthanol (composé 31 du tableau)

### 6.1 [3-(6-Aminopyridin-3-yl)phényl]méthanol

2,0 g de tétrakis(triphénylphosphine)palladium et 75 ml d'une solution 2M de carbonate de sodium sont ajoutés dans un ballon contenant une solution de 5,0 g de 5-bromopyridin-2-ylamine et 5,7 g d'acide 3-(hydroxyméthyl)phénylboronique dans 140 ml de toluène, et 70 ml d'éthanol préalablement dégazée. On chauffe à 80°C pendant 16h. Après refroidissement, le mélange réactionnel est concentré sous pression réduite. Le résidu est repris entre l'eau et l'acétate d'éthyle. La phase organique est séparée, séchée et concentrée sous pression réduite. Le résidu est purifié par chromatographie sur gel de silice par un mélange dichlorométhane/méthanol. On obtient 4,99 g de composé.
RMN 1H (DMSO-d6, δ en ppm) : 4,55 (d, 2H) ; 5,2 (t, 1H) ; 6,05 (s, 2H) ; 6,55 (d,1H) ; 7,2 (d, 1H) ; de 7,3 à 7,55 (m, 3H) ; 7,7 (d, 1H) ; 8,25 (s, 1H) ; M+H = 201.

### 6.2 5-[3-(tert-Butyldiméthylsilanyloxyméthyl)phényl]pyridin-2-ylamine

4,99 g de [3-(6-aminopyridin-3-yl)phényl]méthanol sont placés dans un ballon et dissous dans 240 ml de tétrahydrofurane. On y ajoute 2,2 g d'1*H*-imidazole puis 4,51 g de chlorure de tert-butyldiméthylsilane et on laisse agiter à température ambiante pendant 48 heures. Le mélange réactionnel est ensuite hydrolysé avec de l'eau et la phase organique, extraite à l'acétate d'éthyle, est séparée, séchée sur sulfate de magnésium et concentrée sous pression réduite. Le résidu obtenu est purifié par chromatographie sur gel de silice en éluant avec un mélange heptane/acétate d'éthyle. On obtient 7,0 g de composé.
RMN 1H (DMSO-d6, δ en ppm) : 0,09 (s, 6H) ; 0,91 (s, 9H) ; 4,75 (s, 2H) ; 6,02 (m, 2H) ; 6,52 (dd, 1H) ; 7,21 (d, 1H) ; 7,36 (t, 1H) ; 7,43 (d, 1H) ; 7,47 (s, 1H) ; 7,66 (dd, 1H) ; 8,21 (d, 1H). M+H = 315 ; PF = 82 - 84°C.

### 6.3 6-[3-(tert-Butyldiméthylsilanyloxyméthyl)phényl]-3-méthyl-2-thién-2-ylimidazo[1,2-a]pyridine

Dans un tube en verre, on dissout 280 mg de 5-[3-(*tert-*butyldiméthylsilanyloxyméthyl)phényl]pyridin-2-ylamine, 390 mg de 2-bromo-1-thiophèn-2-ylpropan-1-one et 190 mg d'hydrogénocarbonate de sodium dans 5 ml d'éthanol. Le tube est fermé et chauffé à 100°C pendant 20h. Après refroidissement, le mélange réactionnel est concentré sous pression réduite. Le résidu est purifié par chromatographie sur gel de silice en éluant avec un mélange heptane/acétate d'éthyle. On obtient 260 mg de composé.
Spectre RMN 1H (CDCl₃, δ en ppm) : 0 (s, 6H) ; 0,85 (s, 9H) ; 2,6 (s, 3H) ; 4,7 (s, 2H) ; 7 (m, 1H) ; de 7,2 à 7,6 (m, 8H) ; 7,45 (s, 1H) ; 7.9 (s, 1H) ; M+H = 435.

### 6.4 [3-(3-Méthyl-2-thién-2-yl-imidazo[1,2-a]pyridin-6-yl)phényl]méthanol

310 mg de fluorure de tétrabutylammonium sont introduits dans un ballon contenant 260 mg de 6-[3-(*tert*-Butyldiméthylsilanyloxyméthyl)phényl]-3-méthyl-2-thién-2-ylimidazo[1,2-*a*]pyridine dans 6 ml de tétrahydrofurane. Le mélange réactionnel est agité à température ambiante pendant 48 h et est concentré sous pression réduite. Le résidu est purifié par chromatographie sur gel de silice en éluant avec un mélange dichlorométane/méthanol. On obtient 140 mg de composé. M+H = 321

### 6.5 Chlorhydrate (1:1) de [3-(3-Méthyl-2-thién-2-yl-imidazo[1,2-a]pyridin-6-yl)phényl]méthanol

140 mg de [3-(3-méthyl-2-thién-2-yl-imidazo[1,2-*a*]pyridin-6-yl)phényl]méthanol sont mis en suspension dans du dichlorométhane et du méthanol; on y ajoute, goutte à goutte, 3,2 ml d'une solution 0,1N d'acide chlorhydrique dans l'isopropanol. Le mélange réactionnel est ensuite concentré sous pression réduite. Le solide résiduel est repris dans l'éther diéthylique, le précipité est recueilli par filtration et séché à l'étuve sous pression réduite. On obtient 150 mg de composé.
Spectre RMN 1H (DMSO-d6, δ en ppm) : 2,85 (s, 3H) ; 4,75 (s, 2H) ; 7,35 (m, 1H) ; 7,45 (d, 1H) ; 7,55 (t, 1H) ; 7,75 (d, 1H) ; 7,85 (m, 2H) ; 7,9 (d, 1H) ; 7,95 (d, 1H) ; 8,2 (d, 1H) ; 8,95 (s, 1H) ; M+H = 321 ; PF = 279 - 283°C.

### Exemple 7 : 2-{3-[2-(1H-Indol-6-yl)imidazo[1,2-a]pyridin-6-yl]phényl}propan-2-ol (composé 47 du tableau)

### 7.1 2-(3-bromophényl)propan-2-ol

Sous courant d'argon, 6,5 g de 3-bromoacétophénone sont placés dans un ballon et dissous dans 544 ml d'éther diéthylique et 272 ml de tétrahydrofurane. On refroidit à 0°C par un bain de glace et on y ajoute goutte à goutte 100 ml d'une solution 1M de bromure de méthylmagnésium dans l'éther dibutylique. On agite le mélange à 0°C pendant 1h et on ajoute 400 ml d'une solution aqueuse saturée de chlorure d'ammonium. La phase organique est séparée, séchée sur sulfate de magnésium et concentrée sous pression réduite. On obtient 10.0 g de composé. RMN 1H (DMSO-d6, δ en ppm) : 1,45 (s, 6H) ; 5,15 (s, 1H) ; 7,25 (t, 1H) ; 7,4 (d, 1H) ; 7,45 (d, 1H) ; 7,65 (s, 1H).

### 7.2 2-[3-(2-Chloro-imidazo[1,2-a]pyridin-6-yl)-phenyl]propan-2-ol

On place dans un ballon 85,5 ml de tétrahydrofurane et 9,5 ml d'eau, on dégaze à l'argon pendant 10 min et on ajoute successivement 5,8 g du composé obtenu en 7.1, 4,48 g de 2-chloro-6-(4,4,5,5,-tétraméthyl-1,2,3-dioxaborolan-2-yl)imidazo[1,2-a]pyridine obtenu en 3.2, 20,35 g de carbonate de césium et 0,85 g de de [1,1'-bis(diphénylphosphino)ferrocène]dichloropalladium. On agite le mélange 2 heures au reflux du tétrahydrofurane. Après refroidissement à température ambiante, les solvants sont évaporés sous pression réduite. Le résidu est repris entre l'eau et l'acétate d'éthyle. La phase organique lavée par 2 fois avec une solution saturée de chlorure de sodium est ensuite séchée sur sulfate de magnésium et concentrée sous pression réduite. Le résidu obtenu est purifié par chromatographie sur gel de silice en éluant avec un mélange dichlorométhanelacétate d'éthyle. Le solide obtenu est trituré à l'éther diisopropylique, recueilli par filtration, puis séché à l'étuve sous pression réduite. On obtient 2,31 g de composé. RMN 1H (DMSO-d6, δ en ppm) : 1,5 (s, 6H) ; 5,1 (s, 1H) ; 7,45 (m, 1H) ; 7,55 (m, 2H) ; de 7,6 à 7,7 (m, 2H) ; 7,8 (s, 1H) ; 8,05 (s, 1H) ; 8,85 (s, 1H).

### 7.3 2-{3-[2-(1H-Indol-6-yl)imidazo[1,2-a]pyridin-6-yl]phényl}propan-2-ol

4 mg d'acétate de palladium, 14 mg de 2-(dicyclohexyl)phosphino-2',6'-diméthoxy-1,1'-biphényle, 175 mg d'acide indole-6-boronique, 355 mg de K3PO4 et quelques gouttes d'éthanol sont placés dans un réacteur contenant un mélange de 250 mg de composé obtenu à l'étape 7.2 dans 3,1 ml de toluène anhydre préalablement dégazé sous courant d'argon. Le réacteur est fermé et chauffé à 115°C pendant 16 heures. Après refroidissement, le mélange réactionnel est filtré sur célite puis concentré sous pression réduite. Le résidu obtenu est purifié par chromatographie sur gel de silice en éluant par un mélange heptane/acétate d'éthyle. On obtient 210 mg de composé. PF = 203-204°C.
RMN 1H (DMSO-d6, δ en ppm) : 1,55 (s, 6H) ; 5,1 (s, 1H) ; 6,45 (s, 1H) ; de 7,4 à 7,75 (m, 8H).; 7,85 (s, 1H) ; 8,05 (s, 1H) ; 8,4 (s, 1H) ; 8,85 (s, 1H) ; 11,2 (s, 1H).

### Exemple 8 2-[3-(2-Thién-3-ylimidazo[1,2-a]pyridin-6-yl)phényl]propan-2-ol (composé 49 du tableau)

### 8.1 2-[3-(6-Aminopyridin-3-yl)pbényl]propan-2-ol

Sous courant d'argon, on place dans un ballon, 9,0 g de composé obtenu à l'étape 7.1, 11,05 g de 5-(4,4,5,5-tétraméthyl-1,3,2-dioxaborolan-2-yl)pyridin-2-ylamine, 83,7 ml d'une solution 2M de carbonate de sodium et 1,70 g de tétrakis(triphénylphosphine)palladium que l'on dissout dans 523 ml de *N*,*N*-diméthylformamide. Le mélange est chauffé 1h30 à 80°C. Après refroidissement à température ambiante, 1 1 d'acétate d'éthyle est ajouté au milieu réactionnel que l'on filtre sur célite. La phase organique est ensuite séparée, lavée 3 fois par une solution de chlorure de sodium saturée, séchée sur sulfate de magnésium et concentrée sous pression réduite. Le résidu est purifié par chromatographie sur gel de silice en éluant par un mélange dichlorométhane/méthanol. Le solide obtenu est trituré dans l'éther diisopropylique, recueilli par filtration puis séché à l'étuve sous pression réduite. On obtient 2,35 g de composé.
RMN 1H (DMSO-d6, δ en ppm) : 1,45 (s, 6H) ; 5,0 (s, 1H) ; 6,0 (s, 2H) ; 6,55 (d, 1H) ; de 7,3 à 7,4 (m, 3H) ; 7,65 (s, 1H) ; 7,7 (d, 1H) ; 8,25 (s, 1H).

### 8.2 2-[3-(2-Thién-3-ylimidazo[1,2-a]pyridin-6-yl)phényl]propan-2-ol (composé 49 du tableau)

58 mg (0,7 mmole) de bicarbonate de sodium sont pesés dans un tube à microondes. On y ajoute 57 mg (0,25 mmole) du composé obtenu en 8.1 en solution dans 2 ml de propan-1-ol, puis 92 mg (0,375 mmole) de 2-bromo-1-(thiényl-3-yl)éthanone.en solution dans 1 ml de propan-1-ol Le tube est scellé puis agité à 80°C pendant 16 heures. Le mélange réactionnel est refroidi à température ambiante, on y ajoute 200 mg de propanethiol sur silice (Biotage Si-Thiol) et le mélange est agité 6h à température ambiante, puis filtré. Le résidu est lavé par 2 fois 2 ml de propan-1-ol, puis le filtrat est évaporé et purifié par chromatographie. On obtient 34,2 mg de composé.
RMN 1H (DMSO-d6, δ en ppm) : 1,5 (s, 6H) ; 5,1 (s, 1H) ; 7,45 (t, 1H) ; de 7,5 à 7,55 (m, 2H) ; de 7,6 à 7,7 (m, 4H) ; 7,8 (s, 1H) ; 7,95 (d, 1H) ; 8,3 (s, 1H) ; 8,9 (s, 1H).M+H = 335.

### Exemple 9 : [2-(2-Benzothiazol-2-ylimidazo[1,2-a]pyridin-6-yl)-6-fluorophényl]méthanol (composé 69 du tableau)

### 9.1 (2-Bromo-6-fluorophényl)méthanol

20,0 g (0098 mole) de 2-bromo-6-fluorobenzaldéhyde sont mis en solution dans 500 ml de méthanol et refroidis au bain de glace; puis on y ajoute par portions 3,72 g (0.098 mole) de borohydrure de sodium. Le mélange est agité à froid pendant 1 heure puis le solvant est évaporé sous pression réduite. Le résidu est repris entre l'eau et le dichlorométhane, la phase organique est séparée, séchée et concentrée sous pression réduite. Le résidu est cristallisé dans le pentane. On obtient 18,1 g de composé.
RMN 1H (CDCl3, δ en ppm) : 2,15 (t, 1H) ; 4,95 (d, 2H) ; de 7,05 à 7,3 (m, 2H) ; 7,45 (d, 1H).

### 9.2 (2-Bromo-fluorobenzyloxy)tert-butyldiméthylsilane

Dans un ballon de 500 ml, on dissout 15,7g (0.076 mole) du composé obtenu précédemment dans 230 ml de THF et ajoute 7,8 g (0.115 mole) d'imidazole puis 13,8g (0.092 mole) de chlorure de *tert*-butyldiméthylsilane et agite le mélange réactionnel pendant 16 heures. Puis on évapore le solvant sous pression réduite, reprend le résidu entre l'eau et l'éther diéthylique, décante, lave la phase organique à l'eau et la sèche sur sulfate de sodium. Après évaporation du solvant, on recueille 25 g d'huile.
RMN 1H (CDCl3, δ en ppm) : 0,0 (s, 6H) ; 0,8 (s, 9H) ; 4,7 (s, 2H) ; de 6,8 à 7,05 (m, 2H) ; 7,25 (d, 1H).

### 9.3 5-[2-(tert-Butyldiméthylsilanyloxyméthyl)-3-fluorophényl]pyridin-2-ylamine

6,4 g du composé obtenu en 9.2, 4,40 g de 5-(4,4,5,5-tétraméthyl-1,3,2-dioxaborolan-2-yl)pyridin-2-ylamine, 30 ml d'une solution 2M de carbonate de sodium et 816 mg de [1,1'-bis(diphénylphosphino)ferrocène]dichloropalladium sont dissous dans 80 ml de *N,N-*diméthylformamide et placés dans un ballon sous courant d'argon. Le mélange est chauffé 2h à 80°C. Après refroidissement à température ambiante, on évapore les solvants sous pression réduite et reprend le résidu entre l'eau et l'acétate d'éthyle et élimine un insoluble par filtration sur célite. La phase organique est décantée, lavée avec une solution aqueuse saturée en chlorure de sodium et séchée sur sulfate de sodium. Le composé est purifié par chromatographie en éluant avec un mélange de dichlorométhane et de méthanol. On obtient 4,58 g d'huile.
RMN 1H (DMSO-d6, δ en ppm) : 0 (s, 6H) ; 0,85 (s, 9H) ; 4,5 (s, 2H) ; 6,05 (s, 2H) ; 6,45 (d, 1H) ; de 7,05 à 7,15 (m, 2H) ; de 7,3 à 7,4 (m, 1H) ; de 7,45 à 7,5 (m, 1H) ; 8,0 (d, 1H). M+H = 333

### 9.4 2-{6-(2-(tert-Butyldiméthylsilsinyloxyméthyl)-3-fluorophényl]imidazo[1,2-a]pyridin-2-yl}benzothiazole

58 mg (0,7 mmole) de bicarbonate de sodium sont pesés dans un tube à microondes. On y ajoute 83 mg (0,25 mmole) du composé obtenu en 9.3 en solution dans 2 ml de propan-1-ol, puis 0,375 mmole de 1-(benzothiazol-2-yl)-2-bromoéthanone.en solution dans 1 ml de propan-1-ol Le tube est scellé puis agité à 80°C pendant 16 heures. Le mélange réactionnel est refroidi à température ambiante, on y ajoute 200 mg de propanethiol sur silice (Biotage Si-Thiol) et le mélange est agité 6h à température ambiante, puis filtré et le filtrat est évaporé sous pression réduite. Le composé est utilisé tel quel pour l'étape suivante.

### 9.5 [2-(2-Benzothiazol-2-ylimidazo[1,2-a]pyridin-6-yl)-6-fluorophényl]méthanol

Le composé brut obtenu en 9.4 est dissous dans 5 ml de THF contenant 0,5 mmole de fluorure de tétrabutylammonium hydraté. Le mélange est agité 16h à température ambiante, puis le solvant est évaporé sous pression réduite. Le composé est purifié par chromatographie. On obtient 37,5 mg de composé.
RMN 1H (DMSO-d6, δ en ppm) : 4,45 (d, 2H) ; 5,3 (t, 1H) ; de 7,25 à 7,35 (m, 2H) ; de 7,45 à 7,5 (m, 2H) ; de 7,5 à 7,6 (m, 2H) ; 7,75 (d, 1H) ; 8,05 (d, 1H) ; 8,15 (d, 1H) ; 8,7 (s, 1H); 8,75 (s, 1H). M+H = 376.

### Exemple 10 : [2,6-Difluoro-3-(3-méthyl-2-thién-2-ylimidazo[1,2-a]pyridin-6-yl)phényl]méthanol (composé 75 du tableau)

### 10.1 (3-bromo-2,6-difluorophényl)méthanol

20 g de 3-bromo-2,6-difluorobenzaldéhyde sont mis en solution dans 450 ml de méthanol et refroidi au bain de glace; puis on y ajoute par portions 3,42 g de borohydrure de sodium. Le mélange est agité à température ambiante pendant 1 heure puis le solvant est évaporé sous pression réduite. Le résidu est repris entre l'eau et le dichlorométhane, la phase organique est séparée, séchée et concentrée sous pression réduite. Le résidu est cristallisé dans le n-pentane. On obtient 14,6 g de composé.
Spectre RMN 1H (CDCl3, δ en ppm) :.2,0 (s, 1H) ; 4,9 (s, 2H) ; de 6,85 à 7,0 (m, 1H) ; de 7,5 à 7,65 (m, 1H).

### 10.2 (3-Bromo-2,6-difluorobenzyloxy)tert-butyldiméthylsilane

11,15 g du composé obtenu en 10.1 sont dissous dans 150 ml de THF, on ajoute 5,1 g d'imidazole puis 9,04 g de chlorure de *tert*-butyldiméthylsilane et agite le mélange à température ambiante pendant 24 heures. Puis on évapore le solvant, reprend le résidu et l'eau et l'éther diéthylique, décante, lave la phase organique à l'eau et la sèche sur sulfate de sodium. On évapore le solvant sous pression réduite. On obtient 17,5 g d'huile.
Spectre RMN 1H (CDCl3, δ en ppm) :0,0 (s, 6H) ; 0,8 (s, 9H) ; 4,65 (s, 2H) ; de 6,65 à 6,7 (m, 1H) ; de 7,3 à 7,4 (m, 1H).

### 10.3 5-[3-(tert-Butyldiméthylsilanyloxyméthyl)-2,4-difluorophényl]pyridin-2-ylamine

6,7 g du composé obtenu en 10.2, 4,40 g de 5-(4,4,5,5-tétraméthyl-1,3,2-dioxaborolan-2-yl)pyridin-2-ylamine, 30 ml d'une solution 2M de carbonate de sodium et 816 mg de [1,1'-bis(diphénylphosphino)ferrocène]dichloropalladium sont dissous dans 80 ml de *N,N-*diméthylformamide et placés dans un ballon sous courant d'argon. Le mélange est chauffé 2h à 80°C. Après refroidissement à température ambiante, on évapore les solvants sous pression réduite et reprend le résidu entre l'eau et l'acétate d'éthyle et élimine un insoluble par filtration sur célite. La phase organique est décantée, lavée avec une solution aqueuse saturée en chlorure de sodium et séchée sur sulfate de sodium. Le composé est purifié par chromatographie en éluant avec un mélange de dichlorométhane et de méthanol. On obtient 4,25 g de solide blanc.
RMN 1H (DMSO-d6, δ en ppm) : 0 (s, 6H) ; 0,8 (s, 9H) ; 4,4 (s, 2H) ; 6,05 (s, 2H) ; 6,45 (d, 1H) ; 7,05 (t, 1H) ; de 7,35 à 7,45 (m, 2H) ; 8,0 (s, 1H). M+H = 351.

### 10.4 6-[3-(tert-Butyldiméthylsilanyloxyméthyl)-2,4-difluorophényl]-3-méthyl-2-thién-2-ylimidazo[1,2-a]pyridine

58,8 mg (0,7 mmole) de bicarbonate de sodium sont pesés dans un tube à microondes. On y ajoute 83 mg (0,25 mmole) du composé obtenu en 10.3 en solution dans 2 ml de propan-1-ol, puis 0,375 mmole de 2-bromo-1-(thién-2-yl)propan-1-one en solution dans 1 ml de propan-1-ol Le tube est scellé puis irradié pendant 10 mn à 180°C. Le mélange réactionnel est refroidi à température ambiante, on y ajoute 200 mg de propanethiol sur silice (Biotage Si-Thiol) et le mélange est agité 6h à température ambiante, puis filtré. Le résidu est lavé par 2 fois 2 ml de propan-1-ol, puis le filtrat est évaporé. Le composé est utilisé tel quel pour l'étape suivante.

### 10.5 [2,6-Difluoro-3-[3-méthyl-2-(thién-2-yl)imidazo[1,2-a]pyridin-6-yl]phényl]méthanol

Le composé brut obtenu en 10.4 est dissous dans 5 ml de THF contenant 0,5 mmole de fluorure de tétrabutylammonium hydraté. Le mélange est agité 16h à température ambiante, puis le solvant est évaporé sous pression réduite. Le composé est purifié par chromatographie. On obtient 21,2 mg de composé.
RMN 1H (DMSO-d6, δ en ppm) : 2,75 (s, 3H) ; 4,6 (d, 2H) ; 5,35 (t, 1H) ; 7,2 (d, 1H) ; 7,25 (t, 1H) ; 7,4 (d, 1H) ; 7,5 (s, 1H) ; 7,6 (d, 1H) ; de 7,65 à 7,75 (m, 2H) ; 8,45 (s, 1H). M+H= 357.

### Exemple 11 : 2-[3-(2-Benzo[d]isoxazol-3-ylimidazo[1,2-a]pyridin-6-yl)phényl]propan-2-ol (composé 100 du tableau)

### 11.1 [1-(3-Bromophényl)-1-(méthyl)éthyl]oxytiriméthylsilane

On place dans un ballon 3,4 g du composé préparé selon 7.1 dans 80 ml de dichlorométhane à 0°C. On ajoute 5,6 ml de triéthylamine et 4,5 ml de chlorure de triméthylsilane. Le mélange est agité une heure à 0°C et 20h à température ambiante. On ajoute 50 ml d'eau et le mélange est extrait avec 30 ml de dichlorométhane. La phase organique est lavée avec 20 ml d'une solution saturée en chlorure de sodium, séchée sur sulfate de magnésium puis concentrée sous pression réduite. Le résidu est purifié par chromatographie sur gel de silice en éluant par un mélange heptane/acétate d'éthyle. On obtient 2,5 g de composé.
RMN 1H (CDCl3, δ en ppm) : 0 (s, 9H) ; 1,45 (s, 6H) ; 7,15 à 7,25 (m, 3H) ; 7,45 (m, 1H).

### 11.2 5-[3-[1-Méthyl-1-(triméthylsilanyloxy)éthyl]phényl]pyridin-2-ylamine

0,74 g de composé obtenu en 11.1 sont placés dans 10 ml de tétrahydrofurane et 2 ml d'eau et dégazés sous courant d'argon pendant 10 mn. On y ajoute 0,6 g de 5-(4,4,5,5-tétraméthyl-1,3,2-dioxaborolan-2-yl)pyridin-2-ylamine, 180 mg de [1,1'-bis(diphénylphosphino)ferrocène]dichloropalladium et 1,8 g de carbonate de césium et on chauffe à 80°C pendant 4 heures. Après refroidissement, le mélange réactionnel est concentré sous pression réduite. Le résidu obtenu est purifié par chromatographie sur gel de silice en éluant avec un mélange heptane/acétate d'éthyle. On obtient 560 mg de composé.
RMN 1H (CDCl3, δ en ppm) : 0 (s, 9H) ; 1,5 (s , 6H) ; 4,35 (s, 2H) ; 6,45 (d, 1H) ; 7,25 (m, 3H) ; 7,5 (m, 1H) ; 7,55 (m, 1H) ; 8,2 (s, 1H). M+H = 302.

### 11.3 N-Méthoxy-N-méthylbenzo[d]isoxazole-3-carboxamide

On place dans un ballon 1,0 g d'acide benzo[*d*]isoxazole-3-carboxylique, 0,7 g de chlorhydrate de *N,O*-diméthylhydroxylamine, 2,3 g de chlorhydrate de 1-(3-diméthylaminopropyl-3-éthylcarbodiimide) et 1 ml de pyridine dans 40 ml de tétrahydrofurane. Le mélange est agité à température ambiante pendant 20h. On concentre, le résidu est repris dans 40 ml d'acétate d'éthyle et 20 ml d'eau. La phase organique est lavée avec 20 ml d'une solution 1N d'hydroxyde de sodium, 20 ml d'une solution saturée en chlorure de sodium, séchée sur sulfate de magnésium puis concentrée sous pression réduite. On obtient 1,1 g de composé.
RMN 1H (CDCl3, δ en ppm) : 3,4 (s, 3H) ; 3,8 (s, 3H) ; de 7,25 à 7,55 (m, 3H) ; 7,95 (d, 1H). M+H = 207.

### 11.4 1-(Benzo[d]isoxazol-3-yl)éthanone

On place dans un ballon 1,1 g du composé obtenu en 11.3 dans 50 ml de tétrahydrofurane à 0°C et sous argon. On ajoute goutte à goutte 5 ml de bromure de méthylmagnésium (3M dans l'éther éthylique) . Le mélange est agité deux heure à 0°C et 20h à température ambiante. On refroidit à 0°C et on ajoute 25 ml d'eau et 10 ml d'une solution saturée en chlorure d'ammonium. Le mélange est extrait avec 30 ml d'acétate d'éthyle. La phase organique est lavée avec 40 ml d'une solution saturée en chlorure de sodium, séchée sur sulfate de magnésium puis concentrée sous pression réduite. Le résidu est purifié par chromatographie sur gel de silice en éluant par un mélange heptane/acétate d'éthyle. On obtient 0,6 g de composé.
RMN 1H (CDCl3, δ en ppm) : 2,8 (s, 3H) ; 7,45 (m, 1H) ; 7,65 (m, 2H) ; 8,25 (d, 1H). M+H = 162.

### 11.5 1-(Benzo[d]isoxazol-3-yl)-2-bromoéthanone

On place dans un ballon 1,7 g de bromure de cuivre dans 75 ml d'acétate d'éthyle et on chauffe à reflux. On ajoute 0,6 g de composé obtenu en 11.4. Le mélange est agité 4 heures à reflux. On filtre sur papier puis on verse sur 100 ml d'une solution à 20% de thiosulfate de sodium. Le mélange est extrait avec 50 ml d'acétate d'éthyle. La phase organique est lavée avec 40 ml d'une solution saturée en chlorure de sodium, séchée sur sulfate de magnésium puis concentrée sous pression réduite. On obtient 0,7 g de composé.
RMN 1H (CDCl3, δ en ppm) : 4,75 (s, 2H) ; 7,45 (m, 1H) ; 7,65 (m, 2H) ; 8,25 (d, 1H).

### 11.6 2-[3-(2-Benxo[d]isoaazol-3-ylimidazo[1,2-a]pyridin-6-yl)pbényl]propan-2-ol

On place dans un ballon 0,22 g de composé obtenu en 11.2 et 0,17 g de composé obtenu en 11.5 dans 7 ml d'éthanol. On ajoute 61 mg d'hydrogénocarbonate de sodium et on chauffe à reflux pendant 20h. Après refroidissement, le mélange réactionnel est concentré sous pression réduite. Le résidu obtenu est purifié par chromatographie sur gel de silice en éluant avec un mélange heptane/acétate d'éthyle. On obtient 210 mg de composé.
RMN 1H (DMSO-d6, δ en ppm) : 1,55 (s, 6H) ; 5,15 (s, 1H) ; 7,45 (t, 1H) ; 7,55 (m, 3H) ; 7,75 (m, 2H) ; 7,85 (m, 3H) ; 8,55 (d, 1H) ; 8,75 (s, 1H) ; 9,05 (s, 1H).

### Exemple 12 : procédé de préparation des composés 102 à 111

### 12.1

0,495 mmole d'acétate de palladium, et 0,99 mmole de S-Phos sont pesés dans un ballon de 100 ml purgé à l'argon. On y ajoute 55 ml de toluène dégazé et on agite le mélange dans un bain à ultrasons jusqu'à dissolution complète.

### 12.2.

0,3 mmole d'acide hétéroarylboronique est pesée dans un tube à réaction, on y ajoute successivement 0,36 mmole de phosphate de potassium finement pulvérisé et séché, 0,5 ml d'éthanol anhydre dégazé, 0,18 mmole du composé obtenu en 7.2 en solution dans 2 ml de toluène puis le tube est purgé à l'argon. On ajoute ensuite 1 ml de la solution préparée en 12.1. Le tube est fermé et agité 16h à 75°C. On rajoute 0,5 ml de solution préparée en 12.2.1 et prolonge le chauffage pendant 10h. La solution refroidie est diluée par 5 ml d'acétate d'éthyle, on y ajoute 100 mg de silice-propanethiol (Biotage Si-Thiol) et le mélange est agité 4h à température ambiante. Le solide est séparé par filtration et lavé par 2x2 ml de THF. Le filtrat est évaporé à sec et le composé est soumis à purification par chromatographie.

### Exemple 13 : préparation des composés 112 à 125

### 13.1 6-[2-(tert-Butyldiméthylsilanyloxyméthyl)-3-fluorophényl]-2-chloroimidazo[1,2-a]pyridine

On dégaze sous courant d'argon 100 ml d'un mélange 85/15 de THF et d'eau puis ajoute 5,3 g du composé préparé en 9.2, 6,07 g de 2-chloro-6-(4,4,5,5,-tétraméthyl-1,2,3-dioxaborolan-2-yl)imidazo[1,2-a]pyridine obtenu tel qu'en 4.2, 18,6 g de carbonate de césium et 466 mg de de [1,1'-bis(diphénylphosphino)ferrocène]dichlorapalladium. On agite le mélange 2 heures dans un bain thermostaté à 80°C. Après refroidissement à température ambiante, les solvants sont.évaporés sous pression réduite. Le résidu est repris entre l'eau et l'éther diéthylique. On élimine un solide par filtration. La phase organique lavée par 2 fois avec une solution saturée de chlorure de sodium est ensuite séchée sur sulfate de sodium et concentrée sous pression réduite. Le résidu obtenu est purifié par chromatographie sur gel de silice en éluant avec un mélange dichlorométhane/méthanol. Le solide obtenu est trituré au pentane, recueilli par filtration, puis séché à l'étuve sous pression réduite. On obtient 4,74 g de composé. RMN 1H (CDCl3, δ en ppm) : 0 (s, 6H) ; 0,85 (s, 9H) ; 4,5 (s, 2H) ; de 7,05à 7,1 (m, 2H) ; de 7,25 à 7,3 (m, 2H) ; 7,4 (s, 1H) ; 7,45 (s, 1H) ; 8,3 (s, 1H). M+H = 391.

### 13.2 Procédé de préparation des composés 112 à 125

### 13.2.1

0,3 mmole d'acide hétéroarylboronique est pesée dans un tube à réaction, on y ajoute successivement 0,36 mmole de phosphate de potassium finement pulvérisé et séché, 0,5 ml d'éthanol anhydre dégazé, 0,18 mmole du composé obtenu en 13.1 en solution dans 2 ml de toluène puis le tube est purgé à l'argon. On ajoute ensuite 1 ml de la solution préparée en 12.1. Le tube est fermé et agité 16h à 75°C. On rajoute 0,5 ml de solution préparée en 12.2.1 et prolonge le chauffage pendant 10h. La solution refroidie est diluée par 5 ml d'acétate d'éthyle, on y ajoute 100 mg de silice-propanethiol (Biotage Si-Thiol) et le mélange est agité 4h à température ambiante. Le solide est séparé par filtration et lavé par 2x2 mi de THF. Le filtrat est évaporé à sec et le résidu est utilisé tel quel pour l'étape suivante.

### 13.2.2

Dans un tube à réaction, on mélange le composé obtenu en 13.2.1, 0,36 mmole de fluorure de césium en solution dans 3 ml de méthanol et 21 µl d'acide acétique. La solution est agitée 16 heures à température ambiante, les solvants sont ensuite évaporés. Le résidu est purifié par HPLC en éluant avec un mélange acétonitrile/eau.

### Exemple 14 : préparation des composés 126 à 137

### 14.1 6-[3-(tert-Butyldiméthylsilanyloxyméthyl)-2,4-difluorophényl]-2-chioroimidazo[1,2-a]pyridine

On dégaze sous courant d'argon 100 ml d'un mélange 85/15 de THF et d'eau puis ajoute 5,3 g du composé préparé en 10.2, 6,07 g de 2-chloro-6-(4,4,5,5,-tétraméthyi-1,2,3-dioxaborolan-2-yl)imidazo[1,2-a]pyridine obtenu tel qu'en 4.2, 18,6 g de carbonate de césium et 466 mg de de [1,1'-bis(diphénylphosphino)ferrocène]dichloropalladium. On agite le mélange 2 heures dans un bain thermostaté à 80°C. Après refroidissement à température ambiante, les solvants sont évaporés sous pression réduite. Le résidu est repris entre l'eau et l'éther diéthylique. On élimine un solide par filtration. La phase organique lavée par 2 fois avec une solution saturée de chlorure de sodium est ensuite séchée sur sulfate de sodium et concentrée sous pression réduite. Le résidu obtenu est purifié par chromatographie sur gel de silice en éluant avec un mélange dichlorométhane/méthanol. Le solide obtenu est trituré au pentane, recueilli par filtration, puis séché à l'étuve sous pression réduite. On obtient 4,74 g de composé. RMN 1H (DMSO-d6, δ en ppm) : 0,0 (s, 6H) ; 0,8 (s, 9H) ; 4,7 (s, 2H) ; 7,15 (t, 1H) ; 7,4 (d, 1H) ; de 7,5 à 7,6 (m, 2H) ; 8,0 (s, 1H) ; 8,65 (s, 1H). M+H = 409.

### 14.2 Préparation des composés 126 à 137

### 14.2.1

En procédant comme décrit en 13.2.1, et en partant de 0,3 mmole d'acide hétéroarylboronique et de 0,18 mmole du composé préparé en 14.1 on obtient le composé brut utilisé ten quel pour l'étape suivante.

### 14.2.2

En procédant comme décrit en 13.2.2, et en partant du composé obtenu en 14.2.1, on obtient le composé attendu qui est purifié par HPLC en éluant avec un mélange acétonitrile/eau.

Le tableau 2 ci-après illustre les structures chimiques de formule générale (I) ; le tableau 3 ci-après illustre les caractéristiques physicochimiques de quelques exemples de composés selon l'invention. Dans ces tableaux :
- la colonne « Pos. » renseigne sur la position de substitution du groupe 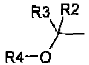 sur le noyau phényle ;
- dans la colonne « Sel/base », « - » représente un composé sous forme de base libre, alors que « HCl » ou « oxalate » représente respectivement un composé sous forme de chlorhydrate ou d'oxalate et le rapport entre parenthèses est le rapport (acide:base) ;
- Dans le tableau 3 ,la colonne « PF » renseigne les points de fusion des produits en degrés Celsius (°C) ou, lorsque les produits ont été isolés sous la forme de solide amorphe ou d'huile, ils sont caractérisés par leur masse [M+H] ;
- « Ph » signifie phényle; « Cl » signifie chlore, « F » signifie fluor ; « Me » signifie méthyle ; « MeO » signifie méthoxy ; « (F₂CH)O » signifie difluorométhoxy ; « *t*-Bu » signifie *tert*-butyle ;
- dans la colonne R, le chiffre devant le substituant renseigne la position de substitution du groupe R sur le noyau phényle ;
- dans la colonne X, le chiffre devant le substituant renseigne la position de substitution du groupe X sur le noyau imidazo[1,2-*a*]pyridine ;
- N.D. signifie non déterminé ;

**Tableau 2**

| | | | | | | | | |
|---|---|---|---|---|---|---|---|---|
| 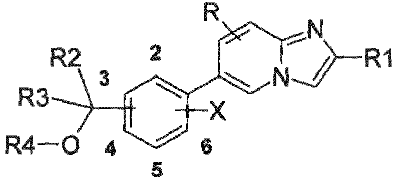 | | | | | | | | |

| **Ex** | **R₁** | **Pos.** | **R₂** | **R₃** | **R₄** | **X** | **R** | **sel** |
|---|---|---|---|---|---|---|---|---|
| **1** | 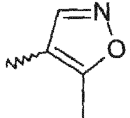 | 3 | H | H | H | H | H | - |
| **2** | 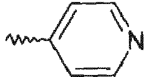 | 3 | H | H | H | H | H | HCl (2:1) |
| **3** | 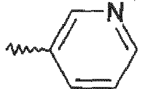 | 3 | H | H | t-Bu | H | H | HCl (1:1) |
| **4** | 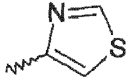 | 3 | H | H | H | H | H | - |
| **5** | 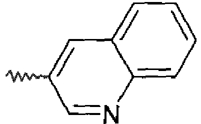 | 3 | H | H | H | H | H | HCl (2:1) |
| **6** | 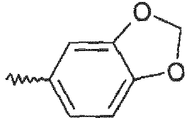 | 3 | H | H | H | H | H | HCl (1:1) |
| **7** | 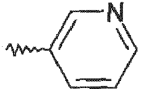 | 3 | H | H | H | H | H | HCl (2;1) |
| **8** | 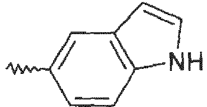 | 3 | H | H | H | H | H | HCl (1:1) |
| **9** | 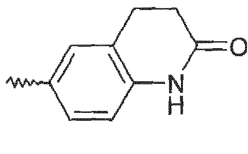 | 3 | H | H | H | H | H | HCl (1:1) |
| **10** | 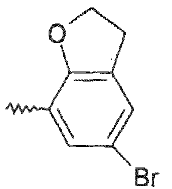 | 3 | H | H | *t*-Bu | H | H | - |
| **11** | 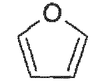 | 3 | H | H | H | H | H | HCl (1:1) |
| **12** | 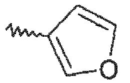 | 2 | H | H | H | H | H | HCl (1:1) |
| **13** | 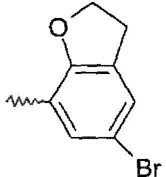 | 3 | H | H | H | H | H | - |
| **14** | 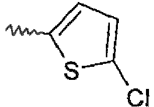 | 3 | H | H | H | H | H | HCl (1:1) |
| **15** | 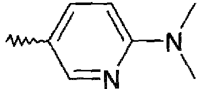 | 3 | H | H | H | H | H | HCl (2:1) |
| **16** | 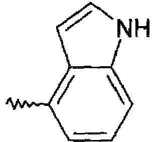 | 3 | H | H | H | H | H | HCl (1:1) |
| **17** | 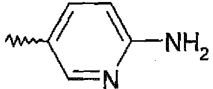 | 3 | H | H | H | H | H | HCl (2:1) |
| **18** | 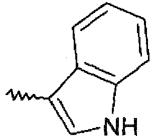 | 3 | H | H | H | H | H | HCl (1:1) |
| **19** | 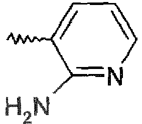 | 3 | H | H | H | H | H | HCl (2:1) |
| **20** | 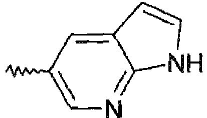 | 3 | H | H | H | H | H | HCl (2:1) |
| **21** | 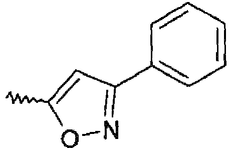 | 3 | H | H | H | H | H | - |
| **22** | 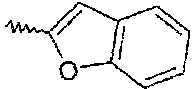 | 3 | H | H | H | H | H | - |
| **23** | 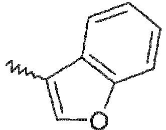 | 3 | H | H | H | H | H | - |
| **24** | 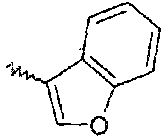 | 4 | H | H | H | H | H | - |
| **25** | 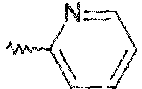 | 3 | H | H | H | H | H | - |
| **26** | 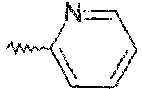 | 4 | H | H | H | H | H | - |
| **27** | 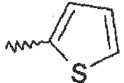 | 3 | H | H | H | H | H | - |
| **28** | 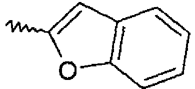 | 3 | H | H | (CH₂)₂OCH₃ | H | H | - |
| **29** | 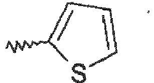 | 3 | H | H | (CH₂)₂OCH₃ | H | H | Oxalate (1:1) |
| **30** | 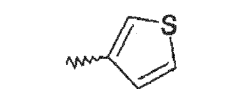 | 3 | H | H | H | H | H | - |
| **31** | 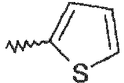 | 3 | H | H | H | H | 3-Me | HCl (1:1) |
| **32** | 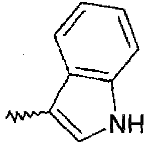 | 3 | H | H | H | H | 3-Me | HCl (1:1) |
| **33** | 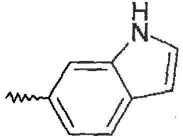 | 3 | H | H | H | H | H | HCl (1:1) |
| **34** | 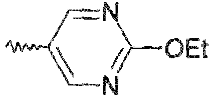 | 3 | H | H | H | H | H | HCl (1:1) |
| **35** | 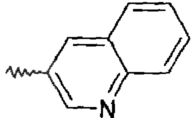 | 2 | H | H | H | H | H | HCl (1:1) |
| **36** | 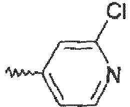 | 3 | H | H | H | H | H | HCl (1:1) |
| **37** | 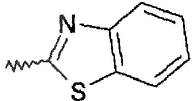 | 3 | H | H | H | H | H | HCl (1:1) |
| **38** | 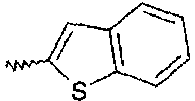 | 3 | H | H | H | H | H | HCl (1:1) |
| **39** | 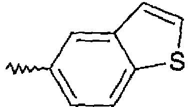 | 3 | H | H | H | H | H | HCl (1:1) |
| **40** | 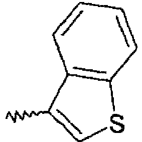 | 3 | H | H | H | H | H | HCl (1:1) |
| **41** | 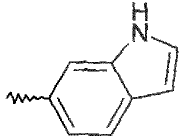 | 2 | H | H | H | H | H | HCl (1:1) |
| **42** | 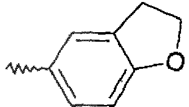 | 3 | H | H | H | H | H | HCl (1:1) |
| **43** | 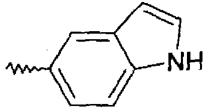 | 2 | H | H | H | H | H | HCl (1:1) |
| **44** | 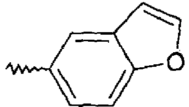 | 3 | H | H | H | H | H | - |
| **45** | 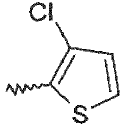 | 3 | H | H | H | H | H | - |
| **46** | 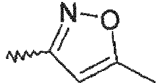 | 2 | H | H | H | 3-F | H | - |
| **47** | 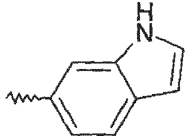 | 3 | Me | Me | H | H | H | - |
| **48** | 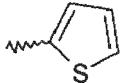 | 3 | Me | Me | H | H | 3-Me | - |
| **49** | 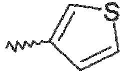 | 3 | Me | Me | H | H | H | - |
| **50** | 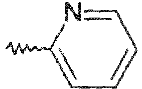 | 3 | Me | Me | H | H | H | - |
| **51** | 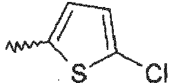 | 3 | Me | Me | H | H | H | - |
| **52** | 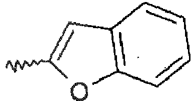 | 3 | Me | Me | H | H | H | - |
| **53** | 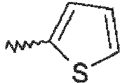 | 3 | Me | Me | H | H | H | - |
| **54** | 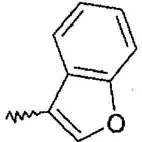 | 3 | Me | Me | H | H | H | - |
| **55** | 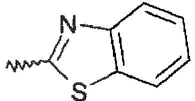 | 3 | Me | Me | H | H | H | - |
| **56** | 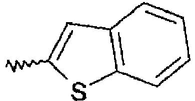 | 3 | Me | Me | H | H | H | - |
| **57** | 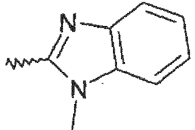 | 3 | Me | Me | H | H | H | - |
| **58** | 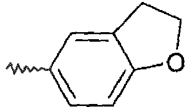 | 3 | Me | Me | H | H | H | - |
| **59** | 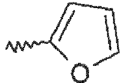 | 3 | Me | Me | H | H | H | - |
| **60** | 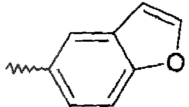 | 3 | Me | Me | H | H | H | - |
| **61** | 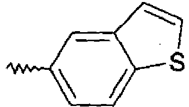 | 3 | Me | Me | H | H | H | - |
| **62** | 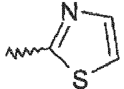 | 3 | Me | Me | H | H | H | - |
| **63** | 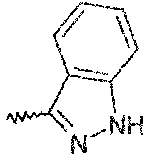 | 2 | H | H | H | 3-F | H | HCl (1:1) |
| **64** | 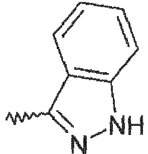 | 3 | Me | Me | H | H | H | HCl (1:1) |
| **65** | 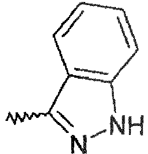 | 2 | H | H | H | 2,4-diF | H | HCl (1:1) |
| **66** | 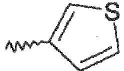 | 2 | H | H | H | 3-F | H | - |
| **67** | 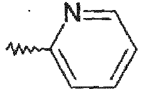 | 2 | H | H | H | 3-F | H | - |
| **68** | 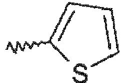 | 2 | H | H | H | 3-F | H | - |
| **69** | 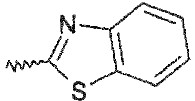 | 2 | H | H | H | 3-F | H | - |
| **70** | 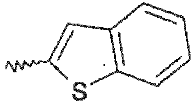 | 2 | H | H | H | 3-F | H | - |
| **71** | 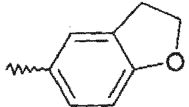 | 2 | H | H | H | 3-F | H | - |
| **72** | 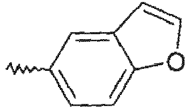 | 2 | H | H | H | 3-F | H | - |
| **73** | 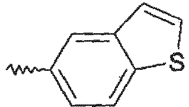 | 2 | H | H | H | 3-F | H | - |
| **74** | 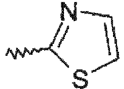 | 2 | H | H | H | 3-F | H | - |
| **75** | 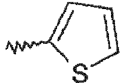 | 3 | H | H | H | 2,4-diF | 3-Me | - |
| **76** | 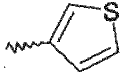 | 3 | H | H | H | 2,4-diF | H | - |
| **77** | 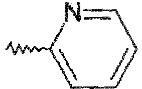 | 3 | H | H | H | 2,4-diF | H | - |
| **78** | 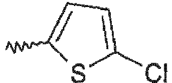 | 3 | H | H | H | 2,4-diF | H | - |
| **79** | 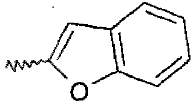 | 3 | H | H | H | 2,4-diF | H | - |
| **80** | 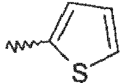 | 3 | H | H | H | 2,4-diF | H | - |
| **81** | 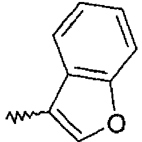 | 3 | H | H | H | 2,4-diF | H | - |
| **82** | 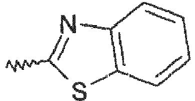 | 3 | H | H | H | 2,4-diF | H | - |
| **83** | 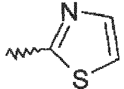 | 3 | H | H | H | 2,4-diF | H | - |
| **84** | 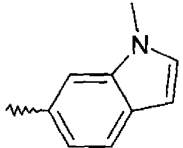 | 3 | H | H | Me | H | H | HCl (1:1) |
| **85** | 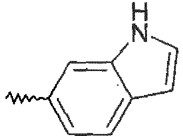 | 3 | H | H | Me | H | H | HCl (1:1) |
| **86** | 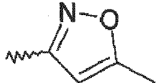 | 3 | Me | Me | H | H | H | - |
| **87** | 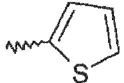 | 2 | H | H | H | 3-F | 3-Me | - |
| **88** | 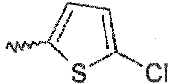 | 2 | H | H | H | 3-F | H | - |
| **89** | 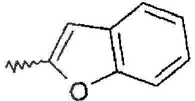 | 2 | H | H | H | 3-F | H | - |
| **90** | 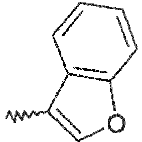 | 2 | H | H | H | 3-F | H | - |
| **91** | 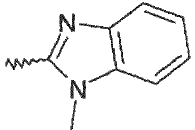 | 2 | H | H | H | 3-F | H | - |
| **92** | 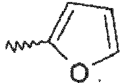 | 2 | H | H | H | 3-F | H | - |
| **93** | 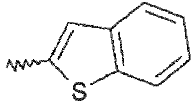 | 3 | H | H | H | 2,4-diF | H | - |
| **94** | 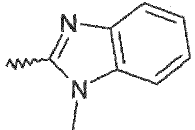 | 3 | H | H | H | 2,4-diF | H | - |
| **95** | 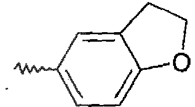 | 3 | H | H | H | 2,4-diF | H | - |
| **96** | 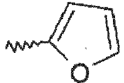 | 3 | H | H | H | 2,4-diF | H | - |
| **97** | 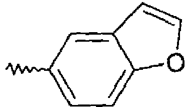 | 3 | H | H | H | 2,4-diF | H | - |
| **98** | 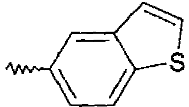 | 3 | H | H | H | 2,4-diF | H | - |
| **99** | 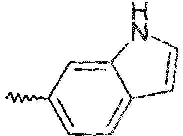 | 3 | H | H | (CH₂)₂OCH₃ | H | H | - |
| **100** | 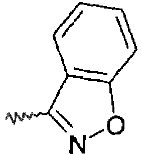 | 3 | Me | Me | H | H | H | - |
| **101** | 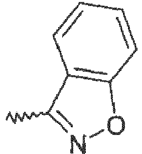 | 2 | H | H | H | 3-F | H | - |
| **102** | 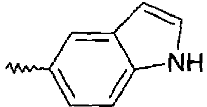 | 3 | Me | Me | H | H | H | - |
| **103** | 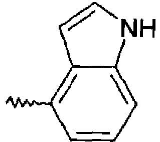 | 3 | Me | Me | H | H | H | - |
| **104** | 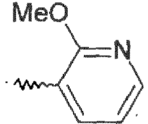 | 3 | Me | Me | H | H | H | - |
| **105** | 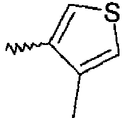 | 3 | Me | Me | H | H | H | - |
| **106** | 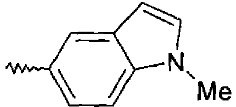 | 3 | Me | Me | H | H | H | - |
| **107** | 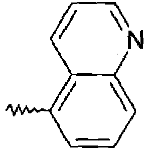 | 3 | Me | Me | H | H | H | - |
| **108** | 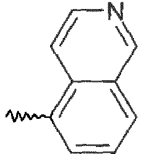 | 3 | Me | Me | H | H | H | - |
| **109** | 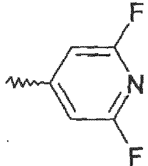 | 3 | Me | Me | H | H | H | - |
| **110** | 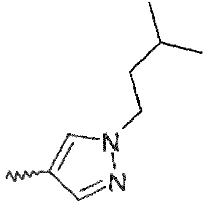 | 3 | Me | Me | H | H | H | - |
| **111** | 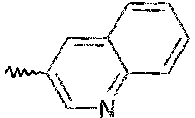 | 3 | Me | Me | H | H | H | - |
| **112** | 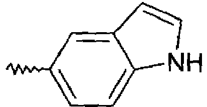 | 2 | H | H | H | 3-F | H | - |
| **113** | 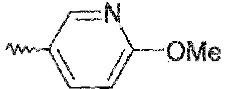 | 2 | H | H | H | 3-F | H | - |
| **114** | 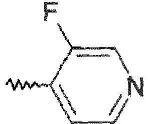 | 2 | H | H | H | 3-F | H | - |
| **115** | 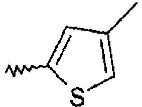 | 2 | H | H | H | 3-F | H | - |
| **116** | 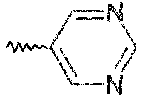 | 2 | H | H | H | 3-F | H | - |
| **117** | 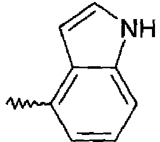 | 2 | H | H | H | 3-F | H | - |
| **118** | 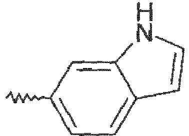 | 2 | H | H | H | 3-F | H | - |
| **119** | 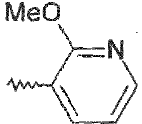 | 2 | H | H | H | 3-F | H | - |
| **120** | 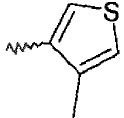 | 2 | H | H | H | 3-F | H | - |
| **121** | 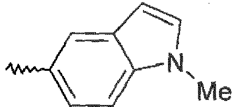 | 2 | H | H | H | 3-F | H | - |
| **122** | 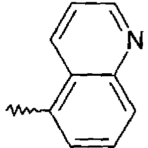 | 2 | H | H | H | 3-F | H | - |
| **123** | 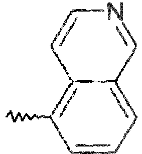 | 2 | H | H | H | 3-F | H | - |
| **124** | 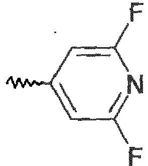 | 2 | H | H | H | 3-F | H | - |
| **125** | 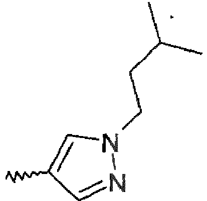 | 2 | H | H | H | 3-F | H | - |
| **126** | 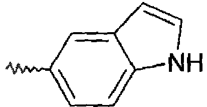 | 3 | H | H | H | 2,4-diF | H | - |
| **127** | 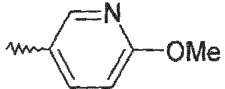 | 3 | H | H | H | 2,4-diF | H | - |
| **128** | 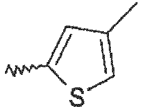 | 3 | H | H | H | 2,4-diF | H | - |
| **129** | 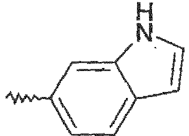 | 3 | H | H | H | 2,4-diF | H | - |
| **130** | 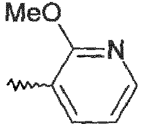 | 3 | H | H | H | 2,4-diF | H | - |
| **131** | 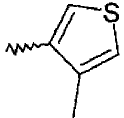 | 3 | H | H | H | 2,4-diF | H | - |
| **132** | 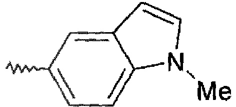 | 3 | H | H | H | 2,4-diF | H | - |
| **133** | 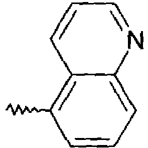 | 3 | H | H | H | 2,4-diF | H | - |
| **134** | 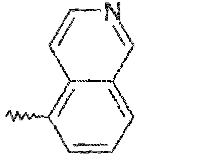 | 3 | H | H | H | 2,4-diF | H | - |
| **135** | 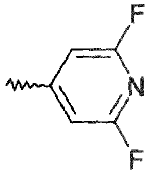 | 3 | H | H | H | 2,4-diF | H | - |
| **136** | 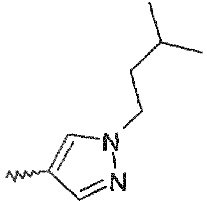 | 3 | H | H | H | 2,4-diF | H | - |
| **137** | 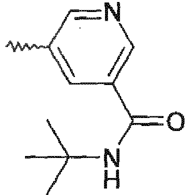 | 3 | H | H | H | 2,4-diF | H | - |

**Tableau 3**

| **Ex** | **PF ou[M+H]** | **RMN** Spectre RMN ¹H (DMSO-d6, δ en ppm) |
|---|---|---|
| **1** | 176-178 | 2,8 (s, 3H) ; 4,6 (d, 2H) ; 5,3 (t, 1H) ; 7,4 (d, 1H) ; 7,5 (t, 1H) ; de 7,6 à 7,75 (m, 4H) ; 8,3 (s, 1H) ; 8,9 (s, 1H) ; 9,0 (s, 1H). |
| **2** | 325-330 | 4,6 (s, 2H) ; 7,4 (d, 1H) ; 7,5 (m, 1H) ; 7,65 (d, 1H) ; 7,75 (s, 1H) ; 7,85 (m, 2H) ; 8,55 (d, 2H), 8,85 (d, 2H) ; 9,05 (m, 2H). |
| **3** | 220-222 | 1,3 (s, 9H) ; 4,5 (s, 2H) ; 7,45 (d, 1H) ; 7,55 (t, 1H) ; de 7,65 à 7,75 (m, 2H) ; de 7,85 à 8,0 (m, 2H) ; 8,05 (d, 1H) ; de 8,75 à 8,90 (m, 3H) ; 9,15 (s, 1H) ; 9,4 (s, 1H). |
| **4** | 201-203 | 4,6 (d, 2H) ; 5,25 (t, 1H) ; 7,4 (d, 1H) ; 7,5 (t, 1H) ; de 7,55 à 7,75 (m, 4H) ; 8,1 (s, 1H) ; 8,35 (s, 1H) ; 8,95 (s, 1H) ; 9,25 (s, 1H). |
| **5** | 279-281 | 4,65 (s, 2H) ; 7,45 (d, 1H) ; 7,55 (t, 1H) ; 7,7 (d, 1H) ; 7,8 (m, 2H) ; 7,95. (m, 1H) ; 8,0 (m, 1H) ; 8,1 (m, 1H) ; 8,2 (m, 2H) ; 8,95 (s, 1H) ; 9,15 (s, 1H) ; 9,2 (s, 1H) ; 9,6 (s, 1H). |
| **6** | 264-266 | 4,65 (s, 2H) ; 6,15 (s, 2H) ; 7,15 (d, 1H) ; 7,45 (d, 1H) ; 7,55 (t, 1H) ; 7,6 (d, 1H) ; 7,7 (m, 2H) ; 7,75 (s, 1H) ; 8,0 (d, 1H) ; 8,2 (d, 1H) ; 8,65 (s, 1H) ; 9,2 (s, 1H). |
| **7** | 260-265 | 4,65 (s, 2H) ; 7,45 (d, 1H) ; 7,55 (t, 1H) ; 7,65 (d, 1H) ; 7,75 (s, 1H) ; de 7,85 à 8,0 (m, 2H) ; 8,05 (m, 1H) ; de 8,75 à 8,9 (m, 3H) ; 9,2 (s, 1H) ; 9,4 (s, 1H). |
| **8** | 270-272 | 4,65 (s, 2H) ; 6,6 (s, 1H) ; 7,45 (d, 1H) ; de 7,5 à 7,6 (m , 2H) ; 7,65 (d, 1H) ; 7,7 (d, 1H) ; de 7,75 à 7,85 (m, 2H) ; 8,05 (d, 1H) ; 8,2 (d, 1H) ; 8,3 (s, 1H) ; 8,65 (s, 1H) ; 9,2 (s, 1H) ; 11,5 (s, 1H). |
| **9** | 340-345 | 2,55 (m, 2H) ; 3,0 (t, 2H) ; 4,65 (s, 2H) ; 7,05 (d, 1H) ; 7,45 (d, 1H) ; 7,55 (t, 1H) ; 7,65 (d, 1H) ; 7,75(s, 1H) ; de 7,8 à 7,9 (m, 2H) ; 8,0 (d, 1H) ; 8,2 (d, 1 H) ; 8,65 (s, 1 H) ; 9,2 (s, 1H). |
| **10** | 277-279 | 1,3 (s, 9H) ; de 3,25 à 3,35 (m, 2H) ; 4,5 (s, 2H) ; 4,9 (t, 2H) ; de 7,3 à 7,4 (m, 2H) ; 7,5 (t, 1H) ; de 7,55 à 7,70 (m, 4H) ; 8,1 (s, 1H) ; 8,45 (s, 1H) ; 9,0 (s, 1H). |
| **11** | 340-345 | 4,65 (s, 2H) ; 7,35 (d, 1H) ; 7,45 (d, 1H) ; 7,55 (t, 1H) ; 7,65 (d, 1H) ; 7,75 (s, 1H) ; 7,85 (d, 1H) ; de 7,9 à 8,0 (m, 2H) ; 8,15(d, 1H) ; 8,65 (s, 1H) ; 9,15 (s, 1H) ; 12,0 (s, 1H). |
| **12** | 205-208 | 4,45 (s, 2H) ; 7,1 (m, 1H) ; 7,4 (m, 1H) ; 7,45 (t, 1H) ; 7,5 (t, 1H) ; 7,65 (d, 1H) ; de 7,9 à 8,0 (m, 3H) ; 8,45 (s, 1H) ; 8,55 (s, 1H) ; 8,95 (s, 1H). |
| **13** | 239-240 | 3,3 (m, 2H) ; 4,6 (d, 2H) ; 4,8 (t, 2H) ; 5,25 (t, 1H) ; de 7,35 à 7,45 (m, 2H) ; 7,5 (t, 1H) ; de 7,55 à 7,7 (m, 4H) ; 8,1 (s, 1H) ; 8,45 (s, 1H) ; 9,0 (s, 1H). |
| **14** | 232-236 | 4,65 (s, 2H) ; 7,25 (d, 1H) ; 7,4 (m, 1H) ; 7,5 (t, 1H) ; 7,65 (m, 2H) ; 7,75 (s, 1H) ; 7,85 (d, 1H) ; 7,95 (d, 1H) ; 8,5 (s, 1H) ; 9,05 (s, 1H). |
| **15** | 280-285 | 3,2 (s, 6H) ; 4,6 (s, 2H) ; 7,15 (m, 1H) ; 7,45 (d, 1H) ; 7,55 (t, 1H) ; 7,65 (d, 1H) ; 7,75 (s, 1H) ; 8,0 (d, 1H) ; 8,15 (m, 1H) ; 8,35 (m, 1H); 8,7 (s, 1H) ; 8,8 (s, 1H) ; 9,20 (s, 1H). |
| **16** | 285-290 | 4,65 (s, 2H) ; 6,9 (m, 1H) ; 7,35 (t, 1H) ; 7,45 (d, 1H) ; 7,55 (t, 1H) ; de 7,65 à 7,75 (m, 4H) ; 7,8 (s, 1H) ; 8,05 (d, 1H) ; 8,25 (d, 1H) ; 8,9 (s, 1H) ; 9,25 (s, 1H) ; 11,65 (s, 1H). |
| **17** | 375-380 | 4,65 (s, 2H) ; 7.15 (d, 1H) , 7,45 (d, 1H) ; 7,55 (t, 1H) ; 7,65 (d, 1H) ; 7,75 (s, 1H) , 7,9 (d, 1H) ; 8,0 (d, 1H) ; 8,35 (m, 2H) ; 8,5 (m, 1H) ; 8,6 (s, 1H) ; 8,7 (s, 1H) ; 9,15 (s, 1H) |
| **18** | 250-254 | 4,65 (s, 2H) ; 7,1 (m, 1H) ; 7,25 (m, 2H) ; de 7,4 à 7,6 (m, 3H) ; 7,65 (m, 2H) ; 7,8 (s, 1H) ; 7,95 (d, 1H) ; 8,15 (d, 1H) ; 8,6 (s, 1H) ; 9,35 (s, 1H) ; 12,15 (s, 1H). |
| **19** | 340-345 | 4,6 (s, 2H) ; 7,05 (t, 1H) ; 7,4 (d, 1H) ; 7,5 (t, 1H) ; 7,65 (d, 1H) ; 7,7 (s, 1H), 7,8 (m, 2H) ; 8,05 (d, 1H) ; 8,55 (d, 1H) ; 8,7 (s, 1H) ; 9,05 (s, 1H). |
| **20** | 380-385 | 4,65 (s, 2H) ; 6,65 (d, 1H) ; 7,45 (d, 1H) ; 7,55 (t, 1H), 7,65 (s, 1H) ; 7,7. (d, 1H) ; 7,8 (s, 1H) ; 8,05 (d, 1H) ; 8,3 (d, 1H) ; 8,7 (s, 1H) ; 8,8 (s, 1H) ; 8,95 (s, 1H) ; 9,3 (s, 1H) ; 12,05 (s, 1H). |
| **21** | 285-287 | 4,6 (d, 2H) ; 5,25 (t, 1H) ; de 7,3 à 7,75 (m, 10H) ; 7,95 (m, 2H) ; 8,5 (s, 1H) ; 8,95 (t, 1H). |
| **22** | 144-146 | 4,6 (d, 2H) ; 5,25 (t, 1H), de 7,15 à 7,75 (m, 11H) ; 8,40 (s, 1H) ; 8,9 (t, 1H). |
| **23** | 150-152 | 4,6 (d, 2H) ; 5,25 (t, 1H) ; de 7,25 à 7,75 (m, 9H) ; 8,2 (m, 1H) ; 8,45 (s, 1H) ; 8,5 (s, 1H); 8,9 (t, 1H). |
| **24** | 178-180 | 4,55 (d, 2H) ; 5,2 (t, 1H) ; de 7,3 à 7,5 (m, 4H) ; de 7,55 à 7,75 (m, 5H) ; 8,2 (m, 1H) ; 8,45 (s, 1H) ; 8,5 (s, 1H) , 8,9 (m, 1H) |
| **25** | 208-210 | 4,6 (d, 2H) ; 5,25 (t, 1H) , de 7,25 à 7,4 (m, 2H) ; 7,45 (t, 1H) ; de 7,5 à 7,7 (m, 4H) ; 7,85 (t, 1H) ; 8,1 (d, 1H) ; 8,5 (s, 1H) ; 8,6 (m, 1H) ; 8,9 (m, 1H). |
| **26** | 226-228 | 4,55 (d, 2H) ; 5,2 (t 1H) ; 7,3 (m, 1H) ; 7,4 (m, 2H) ; 7,7 (m, 4H) ; 7,85. (t, 1H) , 8,1 (d, 1H) ; 8,45 (s, 1H) ; 8,6 (m, 1H) ; 8,95 (s, 1H). |
| **27** | 162-164 . | 4,6 (d, 2H) ; 5,25 (t, 1H) ; 7,1 (m, 1H) , de 7,25 à 7,65 (m, 8H) ; 8,25 (s, 1H) , 8,85 (t, 1H) |
| **28** | 81-83 | 3,25 (s, 3H) ; 3,55 (m, 4H) ; 4,55 (s, 2H) ; de 7,2 à 7,4 (m, 4H) ; 7,5 (t, 1H) ; de 7,68 à 7,75 (m, 6H) ; 8,4 (s, 1H) ; 8,95 (t, 1H). |
| **29** | 126-128 . | 3,25 (s, 3H) ; 3,55 (m, 4H) ; 4,55 (s, 2H) ; 7,1 (m, 1H) ; de 7,3 à 7,7 (m, 8H) ; 8,3 (s, 1H) ; 8,85 (t, 1H). |
| **30** | 173-175 | 4,6 (d, 2H) ; 5,2 (t, 1H) ; 7,3 (d, 1H) ; 7,45 (t, 1H) ; de 7,5 à 7,65 (m, 6H) ; 7,9 (m, 1H) ; 8,25 (s, 1H) ; 8,85 (t, 1H). |
| **31** | 279-283 | 2,85 (s, 3H) ; 4,65 (s, 2H) ; 7,35 (m, 1H) ; 7,45 (d, 1H) ; 7,55 (t, 1H) ; 7,75 (d, 1H) ; 8,8 (m, 2H) ; 8,9 (m, 1H) ; 8,95 (d, 1H) ; 8,15 (d, 1H) ; 8,95 (s, 1H). |
| **32** | 255-258 | 3,75 (s, 3H) ; 4,65 (s, 2H) ; 7,2 (t, 1H) ; 7,3 (t, 1H) ; 7,45 (d, 1H) ; de 7,55 à 7,65 (m, 2H) ; de 7,75 à 7,9 (m, 3H) ; 7,95 (d, 1H) ; 8,05 (d, 1H) ; 8,25 (d, 1H) ; 9,0 (s, 1H) ; 11,95 (s, 1H) ; 14,55 (m, 1H). |
| **33** | 251-254 | 4,65 (s, 2H) ; 6,55 (s, 1H) ; 7,45 (d, 1H) ; 7,55 (m, 2H) ; 7,65 (d, 1H) ; 7,7 (d, 1H) ; 7,75 (m, 2H) ; 8,0 (d, 1H) ; 8,1 (s, 1H) ; 8,25 (d, 1H) ; 8,7 (s, 1H) ; 9,25 (s, 1H) ; 11,6 (s, 1 H). |
| **34** | 204-208 | 1,4 (t, 3H) ; 4,45 (q, 2H) , 4,65 (s, 2H) ; 7,45 (d, 1H) ; 7,55(t, 1H) ; 7,65 (d, 1H) ; 7,75 (s, 1H) ; 7,95 (d, 1H) ;8,05 (d, 1H) ; 8,65 (s, 1H) ; 9,2 (s, 1H) ; 9,25 (s, 2H). |
| **35** | 290-294 | 4,5 (s, 2H) ; 7,4 à 7,55 (m, 3H) ; 7,65 (d, 1H) ; 7,8 (t, 1H) ; 7,95 (m, 2H) 8,0 (d, 1H) ; 8,2 (m, 2H) ; 9,0 (m, 2H) ; 9,2 (s, 1H) ; 9,65 (s, 1H). |
| **36** | 244-248 | 4,6 (s, 2H) ; 7,4 (d, 1H) ; 7,5 (t, 1H) ; 7,65 (d, 1H) ; 7,75 (s, 1H) ; 7,9 (d, 1H) ; 7,95 (d, 1H) ; 8,05 (m, 1H) ; 8,15 (s, 1H) ; 8,55 (d, 1H) ; 8,85 (s, 1H) ; 9,05 (s, 1H). |
| **37** | 220-224 | 4,6 (s, 2H) ; 7,4 (d, 1H) ; 7,45 à 7,65 (m, 4H) ; 7,75 (s, 1H) ; 7,8 (m, 2H) 8,1 (d, 1H) ; 8,2 (d, 1H) ; 8,75 (s, 1H) ; 9,05 (s, 1H). |
| **38** | 261-263 | 4,65 (s, 2H) ; 7,4 à 7,5 (m, 3H) ; 7,55 (t, 1H) ; 7,65 (d, 1H) ; 7,75 (s, 1H) ; 7,9 (d, 1H) ; 7,95 (d, 1H) ; 8,05 à 8,15 (m, 3H) ; 8,65 (s, 1H) ; 9,1 (s, 1H). |
| **39** | 277-279 | 4,6 (s, 2H) ; 7,4 (d, 1H) ; 7,45 (t, 1H) ; 7,55 (d, 1H) 7,65 (d, 1H) ; 7,7 (s, 1H) ; 7,85 (d, 1H) ; 7,95 (m, 2H) ; 8,15 (d, 1H) ; 8,2 (d, 1H) ; 8,5 (s, 1H) ; 8,7 (s, 1H) ; 9,15 (s, 1H). |
| **40** | 285-288 | 4,55 (s, 2H) ; 7,35 (d, 1H) ; 7,45 (m, 2H) ; 7,55 (m, 1H) ;7,6 (d, 1H) ; 7,7 (s, 1H) ; 7,95 (d, 1H) ; 8,15 (m, 2H) ; 8,25 (d, 1H) ; 8,45 (s, 1H) ; 8,8 (s, 1H) ; 9,15 (s, 1H). |
| **41** | 310-315 | 4,5 (s, 2H) ; 6,55 (s, 1H) ; 7,4 à 7,55 (m, 4H) ; 7,65 (m, 2H) ; 7,75 (d, 1H) ; 7,95 (m, 2H) ; 8,1 (s, 1H) ; 8,7 (s, 1H) ; 8,95 (s, 1H). |
| **42** | 270-271 | 3,35 (t, 2H) ; de4,6 à 4,7 (m, 4H) ; 7,0 (d, 1H) ; 7,5 (d, 1H) ; 7,55 (t, 1H) 7,7 (d, 1H) ; 7,75 (s, 1H) ; 7,85 (d, 1H) ; 7,95 (s, 1H) ; 8,0 (d, 1H) ; 8,2 (d, 1H) ; 8,6 (s, 1H) ; 8,2 (s, 1H). |
| **43** | 310-315 | 4,5 (s, 2H) ; 6,6 (s, 1H) ; 7,4 à 7,55 (m, 4H) ; 7,65 (m, 2H) ; 7,75 (d, 1H) 8,0 (m, 2H) ; 8,3 (s, 1H) ; 8,7 (s, 1H) ; 8,95 (s, 1H). |
| **44** | 181-183 | 4,65 (d, 2H) ; 5,3 (t, 1H) ; 7,1 (s, 1H) ; 7,4 (d, 1H) ; 7,55 (t, 1H) ; 7,65 (m, 1H) ; 7,75 (m, 4H) ; 8,0 (d, 1H) ; 8,1 (s, 1H) ; 8,35 (s, 1H) ; 8,5 (s, 1H) ; 8,95 (s, 1H). |
| **45** | 178-180 | 4,65 (d, 2H) ; 5,3 (t, 1H) ; 7,2 (s, 1H) ; 7,4 (d, 1H) ; 7,5 (t, 1H) ; 7,65 (d, 1H) ; 7,75 (m, 4H) ; 8,65 (s, 1H) ; 9,05 (s, 1H). |
| **46** | 277-279 | 2,5 (s, 3H) ; 4,45 (d, 2H) ; 5,3 (t, 1H) ; 6,75 (s, 1H) ; 7,3 (m, 2H) ; 7,5 (m, 2H) ; 7,75 (d, 1H) ; 8,5 (s, 1H) ; 8,75 (s, 1H). |
| **47** | 203-204 | 1,55 (s, 6H) ; 5,1 (s, 1H) ; 6,45 (s, 1H) ; de 7,4 à 7,75 (m, 8H).; 7,85 (s, 1H) ; 8,05 (s, 1H) ; 8,4 (s, 1H) ; 8,85 (s, 1H) ; 11,2 (s, 1H). |
| **48** | [349] | 1,5 (s, 6H) ; 2,8 (s, 3H) ; 5,1 (s, 1H) ; 7,2 (d, 1H) ; 7,4s (t, 1H) ; 7,55 (d, 2H) ; de 7,6 à 7,7 (m, 4H) ; 7,85 (s, 1H) ; 8,55 (s, 1H). |
| **49** | [335] | 1,5 (s, 6H) ; 5,1 (s, 1H) ; 7,45 (t, 1H) ; de 7,5 à 7,55 (m, 2H) ; de 7,6 à 7,7 (m, 4H) ; 7,8 (s, 1H) ; 7,95 (d, 1H) ; 8,3 (s, 1H) ; 8,9 (s, 1H). |
| **50** | [330] | 1,5 (s, 6H) ; 5,1 (s, 1H) ; de 7,3 à 7,35 (m, 1H) ; 7,45 (t, 1H) ; 7,5 (d, 1H), 7,55 (d, 1H) ; 7,65 (d, 1H) ; 7,7 (d, 1H) ; 7,8 (s, 1H) ; 7,9 (t, 1H) ; 8,15 (d, 1H) ; 8,55 (s, 1H) ; 8,65 (d, 1H) ; 9,0 (s, 1H). |
| **51** | [369] | 1,5 (s, 6H) ; 5,1 (s, 1H) ; 7,15 (d, 1H) ; de 7,4 à 7,45 (m, 2H) ; de 7,5 à 7,55 (m, 2H) ; de 7,6 à 7,65 (m, 2H) ; 7,8 (s, 1H) ; 8,35 (s, 1H) ; 8,9 (s, 1H). |
| **52** | [369] | 1,5 (s, 6H) ; 5,15 (s, 1H) ; de 7,25 à 7,4 (m, 3H) ; 7,45 (t, 1H) ; 7,5 (d, 1H) ; 7,55 (d, 1H) ; de 7,65 à 7,75 (m, 4H) ; 7,85 (s, 1H) ; 8,45 (s, 1H) 8,95 (s, 1H). |
| **53** | [335] | 1,5 (s, 6H) ; 5,15 (s, 1H) ; 7,15 (d, 1H) ; de 7,5 à 7,6 (m, 4H) ; de 7,6 à 7,65 (m, 2H) ; 7,8 (s, 1H) ; 8,3 (s, 1H) ; 8,85 (s, 1H). |
| **54** | [369] | 1,5 (s, 6H) ; 5,1 (s, 1H) ; de 7,4 à 7,45 (m, 3H) ; 7,5 (d, 1H) ; 7,55 (d, 1H) ; de 7,65 à 7,75 (m, 3H) ; 7,85 (s, 1H) ; 8,2 (m, 1H) ; 8,55 (s, 2H) 8,98 (s, 1H). |
| **55** | [386] | 1,5 (s, 6H) ; 5,15 (s, 1H) ; 7,45 (t, 2H) ; de 7,5 à 7,6 (m, 3H) ; 7,65 (d, 1H) ; 7,7 (d, 1H) ; 7,85 (s, 1H) ; 8,05 (d, 1H) ; 8,15 (d, 1H) ; 8,7 (s, 1H) ; 9,0 (s, 1H). |
| **56** | [385] | 1,5 (s, 6H) ; 5,15 (s, 1H) ; de 7,35 à 7,45 (m, 3H) ; 7,5 (d, 1H) ; 7,55 (d, 1H) ; 7,65 (d, 1H) ; 7,7 (d, 1H) ; 7,8 (s, 1H) ; de 7,85 à 7,9 (m, 2H) ; 8,0 (d, 1H) ; 8,55 (s, 1 H) ; 8,9 (s, 1H). |
| **57** | [383] | 1,5 (s, 6H) ; 4,35 (s, 3H) ; 5,15 (s, 1H) ; de 7,25 à 7,35 (m, 2H) ; 7,45 (t, 1H) ; 7,5 (d, 1H) ; 7,6 (d, 1H) ; de 7,65 à 7,75 (m, 3H) ; 7,8 (d, 1H) ; 7,85 (s, 1H) ; 8,7 (s, 1H), 9,05 (s, 1H). |
| **58** | [371] | 1,5 (s, 6H) ; de 3,2 à 3,3 (m, 2H) ; de 4,55 à 4,6 (m, 2H) ; 5,1 (s, 1H) ; 6,85 (d, 1H) ; de 7.4 à 7,55 (m, 4H) ; 7,6 (d, 1H) ; 7,7 (d, 1H) ; 7,8 (s, 1H) ; 7,85 (s, 1H) ; 8,3 (s, 1H) ; 8,85 (s, 1H). |
| **59** | [319] | 1,5 (s, 6H) ; 5,15 (s, 1H) ; 6,6 (d, 1H) ; 6,85 (d, 1H) ; 7,4 (t, 1H) ; de 7,45 à 7,55 (m, 2H) ; 7,65 (m, 2H) ; 7,75 (s, 1H) ; 7,8 (s, 1H) ; 8,2 (s, 1H) ; 8,9 (s, 1H). |
| **60** | [369] | 1,5 (s, 6H) ; 5,15 (s, 1H) ; 7,05 (s, 1H) ; 7,45 (t, 1H) ;.7,5 (d, 1H) ; 7,55 (d, 1H) ; 7,6 (d, 1H) ; de7,6 à 7,7 (d, 2H) ; 7,8 (s, 1H) ; 7,95 (d, 1H) ; 8,0 (s, 1H) ; 8,3 (s, 1H) ; 8,45 (s, 1H) ; 8,9 (s, 1H). |
| **61** | [385] | 1,5 (s, 6H) ; 5,15 (s, 1H) ; de 7,45 à 7,5 (m, 2H) ; de 7,55 à 7,6 (m, 2H) ; 7,65 (d, 1H) ; 7,7 (d, 1H) ; de 7,8 à 7,85 (m, 2H) ; 8,0 (d, 1H) ; 8,05 (d, 1H) ; 8,5 (s, 1H) ; 8,55 (s, 1H) ; 8,9 (s, 1H). |
| **62** | [336] | 1,5 (s, 6H) ; 5,15 (s, 1H) ; 7,45 (t, 1H) ; de 7,45 à 7,55 (m, 2H) ; de 7,65 à 7,7 (m, 2H) ; 7,75 (d, 1H) ; 7,8 (s, 1H) ; 7,9 (sd, 1H) ; 8,5 (s, 1H) ; 8,95 (s, 1H). |
| **63** | 330-335 | 4,5 (s, 2H) ; 7,4 (m, 3H) ; 7,55 (m, 2H) ; 7,75 (d, 1H) ; 8,05 (m, 2H) ; 8,3 (d, 1H) ; 9,0 (s, 1H) ; 9,15 (s, 1H) ; 14,0 (s, 1H). |
| **64** | 190-195 | 1,55 (s, 6H) ; 7,4 (t, 1H) ; 7,6 (m, 4H) ; 7,75 (d, 1H) ; 7,9 (s, 1H) ; 8,0 (d, 1H) ; 8,25 (m, 2H) ; 9,05 (s, 1H) ; 9,2 (s, 1H) ; 14,0 (s, 1H) |
| **65** | 350-355 | 4,65 (s, 2H) ; 7,35 (m, 2H) ; 7,55 (t, 1H) ; 7,75 (m, 2H) ; 8,05 (m, 2H) ; 8,25 (d, 1H) ; 9,05 (s, 1H) ; 9,1 (s, 1H) ; 13,9 (s, 1H). |
| **66** | [325] | 4,45 (d, 2H) ; 5,3 (t, 1H) ; de 7,25 à 7,3 (m, 2H) ; de 7,4 à 7,5 (m, 2H) ; de 7,5 à 7,55 (m, 3H) ; 7,95 (s, 1H) ; 8,3 (s, 1H) ; 8,65 (s, 1H). |
| **67** | [320] | 4,45 (s, 2H) ; 5,25 (s, 1H) ; de 7,25 à 7,35 (m, 3H) ; 7,45 (m, 2H) ;7,7 (d, 1H) ; 7,9 (t, 1H) ; 8,25 (d, 1H) ; 8,5 (s, 1H) ; 8,65 (s, 1H) ; 8,75 (s, 1H). |
| **68** | [325] | 4,45 (d, 2H) ; 5,3 (t, 1H) ; 7,15 (m, 1H) ; de 7,2 à 7,3 (m, 2H) ; de 7,4 à 7,5 (m, 2H) ; de 7,5 à 7,6 (m, 2H) ; 7,65 (d, 1H) ; 8,3 (s, 1H) ; 8,65 (s, 1H). |
| **69** | [376] | 4,45 (d, 2H) ; 5,3 (t, 1H) ; de 7,25 à 7,35 (m, 2H) ; de 7,45 à 7,5 (m, 2H) ; de 7,5 à 7,6 (m, 2H) ; 7,75 (d, 1H) ; 8,05 (d, 1H) ; 8,15 (d, 1H) ; 8,7 (s, 1H) ; 8,75 (s, 1H). |
| **70** | [375] | 4,45 (d, 2H) ; 5,3 (t, 1H) ; de 7,25 à 7,4 (m, 4H) ; de 7,45 à 7,5 (m, 2H) ; 7,7 (d, 1H) ; 7,85 (d, 1H) ; 7,9 (s, 1H) ; 8,0 (d, 1H) ; 8,5 (s, 1H) ; 8,7 (s, 1H). |
| **71** | [361] | 3,25 (t, 2H) ; 4,45 (d, 2H) ; 4,55 (t, 2H) ; 5,3 (t, 1H) ; 6,85 (d, 1H); 7,25 (m, 2H) ; 7,4 (d, 1H) ; 7,45 (m, 1H) ; 7,6 (d, 1H) ; 7,75 (d, 1H) ; 7,85 (s, 1H) ; 8,3 (s, 1H) ; 8,65 (s, 1H). |
| **72** | [359] | 4,45 (d, 2H) ; 5,3 (t, 1H) ; 7,05 (s, 1H) ; 7,3 (m, 2H) ; de 7,4 à 7,5 (m, 2H) ; 7,65 (d, 2H) ; 7,95 (d, 1H) ; 8,05 (s, 1H) ; 8,3 (s, 1H) ; 8,45 (s, 1H). ; 8,65 (s, 1H). |
| **73** | [375] | 4,45 (d, 2H) ; 5,35 (t, 1H) ; 7,3 (m, 2H) ; de 7,4 à 7,5 (m, 2H) ; 7,55 (d, 1H) ; 7,65 (d, 1H) ; 7,8 (d, 1H) ; 8,0 (d, 1H) ; 8,05 (d, 1H) ; 8,5 (s, 1H) ; 8,55 (s, 1H) ; 8,7 (s, 1H). |
| **74** | [326] | 4,45 (d, 2H) ; 5,3 (t, 1H) ; de 7,25 à 7,35 (m, 2H) ; de 7,45 à 7,55 (m, 2H) ; 7,7 (d, 1H) ; 7,75 (s, 1H) ; 7,95 (s, 1H) ; 8,5 (s, 1H) ; 8,75 (s, 1H). |
| **75** | [357] | 2,75 (s, 3H) ; 4,6 (d, 2H) ; 5,35 (t, 1H) ; 7,2 (d, 1H) ; 7,25 (t, 1H) ; 7,4 (d, 1H) ; 7,5 (s, 1H) ; 7,6 (d, 1H) ; de 7,65 à 7,75 (m, 2H) ; 8,45 (s, 1H). |
| **76** | [343] | 4,6 (d, 2H) ; 5,35 (t, 1H) ; 7,25 (t, 1H) ; 7,4 (d, 1H) ; de 7,55 à 7,65 (m, 4H) ; 7,95 (s, 1H) ; 8,35 (s, 1H) ; 8,75 (s, 1H). |
| **77** | [338] | 4,6 (s, 2H) ; 5,35 (s, 1H) ; 7,25 (t, 1H) ; 7,35 (m, 1H) ; 7,45 (d, 1H) ; 7,6 (m, 1H) ; 7,75 (d, 1H) ; 7,9 (t, 1H) ; 8,15 (d, 1H); 8,55 (s, 1H) ;.8,65 (d, 1H) ; 8,85 (s, 1H). |
| **78** | [377] | 4,6 (d, 2H) ; 5,35 (t, 1H) ; 7,15 (s, 1H) ; 7,25 (t, 1H) ; de 7,4 à 7,45 (m, 2H) ; de 7,55 à 7,65 (m, 2H) ; 8,35 (s, 1H) ; 8,75 (s, 1H). |
| **79** | [377] | 4,6 (d, 2H) ; 5,35 (t, 1H) ; de 7,2 à 7,3 (m, 2H) ; 7,35 (m, 2H) ; 7,5 (d, 1H) ; de 7,6 à 7,75 (m, 4H) ; 8,5 (s, 1H) ; 8,85 (s, 1H). |
| **80** | [343] | 4,6 (s, 2H) ; 5,35 (s, 1H); 7,15 (d, 1H) ; 7,35 (t, 1H) ; 7,45 (d, 1H) ; 7,55. (m, 2H) ; de 7,6 à 7,7 (m, 2H) ; 8,35 (s, 1H) ; 8,75 (s, 1H). |
| **81** | [377] | 4,6 (d, 2H) ; 5,35 (t, 1H) ; 7,25 (t, 1H) ; de 7,4 à 7,5 (m, 3H) ; de 7,6 à 7,75 (m, 3H) ; 8,25 (m, 1H) ; 8,55 (d, 2H) ; 8,8 (s, 1H). |
| **82** | [394] | 4,6 (d, 2H) ; 5,35 (t, 1H) ; 7,25 (t, 1H) ; 7,45 (t, 1H) ; 7,55 (m, 2H) ; 7,65 (m, 1H) ; 7,8 (d, 1H) ; 8,05 (d, 1H) ; 8,15 (d, 1H) ; 8,75 (s, 1H) ; 8,9 (s, 1H). |
| **83** | [344] | 4,6 (s, 2H) ; 5,35 (s, 1H) ; de 7,25 à 7,3 (m, 2H) ; de 7,4 à 7,5 (m, 2H) ; de 7,5 à 7,55 (m, 3H) ; 7,95 (s, 1H) ; 8,3 (s, 1H) ; 8,65 (s, 1H). |
| **84** | 247-252 | 3,4 (s, 3H) ; 3,9 (s, 3H) ; 4,55 (s, 2H) ; 6,55 (s, 1H) ; de 7,45 à 7,8 (m, 7H) ; 8,0 (d, 1H) ; 8,25 (m, 2H) ; 8,7 (s, 1H) ; 9,25 (s, 1H). |
| **85** | 88-92 | 3,35 (s, 3H) ; 4,55 (s, 2H) ; 6,55 (s, 1H) ; 7,45 (d, 1H) ; de 7,55 à 7,65 (m, 3H) ; 7,75 (m, 3H) ; 8,0 (d, 1H) ; 8,1 (s, 1H) ; 8,25 (d, 1H) ; 8,7 (s, 1H) ; 9,25 (s, 1H) ; 11,6 (s, 1H). |
| **86** | 205-208 | 1,5 (s, 6H) ; 2,5 (s, 3H) ; 5,1 (s, 1H) ; 6,7 (s, 1H) ; 7,45 (m, 1H) ; 7,55 (m, 2H) ; 7,7 (m, 2H) ; 7,8 (s, 1H) ; 8,45 (s, 1H) ; 8,95 (s, 1H). |
| **87** | [339] | 2,70 (s, 3H) ; 4,45 (d, 2H) ; 5,35 (t, 1H) ; 7,20 (m, 1H) ; 7,32 (m, 2H) ; 7,47 (m, 3H) ; 7,58 (m, 1H) ; 7,64 (d, 1H) ; 8,50 (s, 1H). |
| **88** | [359] | 4,43 (d, 2H) ; 5,30 (t, 1H) ; 7,18 (s, 1H) ; 7,30 (m, 2H) ; 7,46 (m, 3H) ; 7,65 (d, 1H) ; 8,39 (s, 1H) ; 8,68 (s, 1H). |
| **89** | [359] | 4,45 (d, 2H) ; 5,30 (t, 1H) ; 7,32 (m, 5H) ; 7,48 (m, 2H) ; 7,65 (d, 1H) ; 7,70 (m, 2H) ; 8,47 (s, 1H) ; 8,72 (s, 1H). |
| **90** | [359] | 4,45 (d, 2H) ; 5,32 (t, 1H) ; 7,30 (m, 2H) ; 7,41 (m, 2H) ; 7,48 (m, 2H) ; 7,70 (m, 2H) ; 8,25 (d, 1H) ; 8,54 (m, 2H) ; 8,72 (s, 1H). |
| **91** | [373] | 4,35 (s, 3H) ; 4,47 (d, 2H) ; 5,31 (t, 1H) ; 7,28 (m, 4H) ; 7,49 (m, 1H) ; 7,55 (d, 1H) ; 7,65 (dd 2H) ; 7,78 (d, 1H) ; 8,66 (s, 1H) ; 8,78 (s, 1H). |
| **92** | [309] | 4,45 (d, 2H) ; 5,30 (t, 1H) ; 6,63 (s, 1H) ; 6,88 (s, 1H) ; 7,29 (m, 2H) ; 7,47 (m, 2H) ; 7,62 (d, 1H) ; 7,78 (s, 1H) ; 8,23 (s, 1H) ; 8,69 (s, 1H). |
| **93** | [393] | 4,61 (d, 2H) ; 5,34 (t, 1H) ; 7,25 (t, 1H) ; 7,40 (m, 2H) ; 7,48 (d, 1H) ; 7,65 (m, 1H) ; 7,72 (d, 1H) ; 7,88 (d, 1H) ; 7,91 (s, 1H) ; 8,00 (d, 1H) ; 8,52 (s, 1H) ; 8,81 (s, 1H). |
| **94** | [391] | 4,35 (s, 3H) ; 4,62 (d, 2H) ; 5,32 (t, 1H) ; 7,29 (m, 3H) ; 7,53 (d, 1H) ; 7,67 (m, 3H) ; 7,82 (d, 1H) ; 8,70 (s, 1H) ; 8,94 (s, 1H). |
| **95** | [379] | 3,25 (t, 2H) ; 4,58 (m, 4H) ; 5,34 (t, 1H) ; 6,83 (d, 1H) ; 7,25 (t, 1H) ; 7,40 (d, 1H) ; 7,62 (m, 2H) ; 7,74 (d, 1H) ; 7,86 (s, 1H) ; 8,30 (s, 1H) ; 8,74 (s, 1H). |
| **96** | [327] | 4,60 (d, 2H) ; 5,33 (t, 1H) ; 6,62 (s, 1H) ; 6,88 (s, 1H) ; 7,25 (t, 1H) ; 7,42 (d, 1H) ; 7,63 (m, 2H) ; 7,79 (s, 1H) ; 8,24 (s, 1H) ; 8,80 (s, 1H). |
| **97** | [377] | 4,62 (d, 2H) ; 5,35 (t, 1H) ; 7,05 (s, 1H) ; 7,25 (t, 1H) ; 7,41 (d, 1H) ; 7,68 (m, 3H) ; 7,97 (d, 1H) ; 8,05 (s, 1H) ; 8,30 (s, 1H) ; 8,47 (s, 1H) ; 8,78 (s, 1H). |
| **98** | [393] | 4,61 (d, 2H) ; 5,34 (t, 1H) ; 7,25 (t, 1H) ; 7,43 (d, 1H) ; 7,57 (d, 1H) ; 7,64 (m, 1H) ; 7,72 (d, 1H) ; 7,81 (d, 1H) ; 7,99 (d, 1H) ; 8,08 (d, 1H) ; 8,52 (d, 2H) ; 8,79 (s, 1H). |
| **99** | 67-70 | 3,3 (s, 3H) ; 3,55 (m, 2H) ; 3,65 (m, 2H) ; 4,6 (s, 2H) ; 6,45 (m, 1H) ; 7,4 (m, 2H) ; 7,5 (t, 1H) ; de 7,55 à 7,7 (m, 6H) ; 8,05 (s, 1H) ; 8,35 (s, 1H) ; 8,9 (s, 1H) ; 11,15 (s, 1H). |
| **100** | 206-210 | 1,55 (s, 6H) ; 5,15 (s, 1H) ; 7,45 (t, 1H) ; 7,55 (m, 3H) ; 7,75 (m, 2H) ; 7,85 (m, 3H) ; 8,55 (d, 1H) ; 8,75 (s, 1H) ; 9,05 (s, 1H). |
| **101** | 275-277 | 4.45 (d, 2H) ; 5,35 (t, 1H) ; 7,35 (m, 2H) ; 7,45 à 7,6 (m, 3H) ; 7,75 à 7,9 (m, 3H) ; 8,55 (d, 1H) ; 8,8 (m, 2H). |
| **102** | [368] | 1.5 (s, 6H) ; 5,15 (s, 1H) ; 6,5 (m, 1H) ; 7,5 (m, 7H) ; 7,72 (d, 1H) ; 7,8 (s, 1H) ; 8,19 (s, 1H) ; 8,32 (s, 1H) ; 8,85 (s, 1H) ; 11,2 (s, 1H). |
| **103** | [368] | 1,5 (s, 6H) ; 5,15 (s, 1H) ; 6,4 (m, 1H) ; 7,0 (m, 1H) ; 7,05 (m, 2H) ; 7,2 (m, 1H) ; 7,35 (m, 1H) ; 7,5 (m, 3H) ; 7,75 (m, 1H) ; 7,85 (s, 1H) ; 8,6 (s, 1H) ; 8,95 (s, 1H) ; 11,0 (s, 1H). |
| **104** | [360] | 1.5 (s, 6H), 4.1 (s, 3H), 5.1 (s, 1H), 7.15 (m, 1 H), 7.45 (m, 1H), 7.55 (m, 2H), 7.7 (s, 2H), 7.8 (s, 1H), 8.2 (d, 1H), 8.55 (s, 1H), 8.6 (d, 1H) , 9.0 (s, 1H) |
| **105** | [346] | 1.5 (s, 6H), 2,5 (s, 3H) ; 5.15 (s, PH), 7.3 (m, 1H), 7,45 (m, 1H), 7.5 (m, 2H), 7.65 (m, 2H), 7.8 (s, 1H), 7.9 (d, 1H), 8.25 (s, 1H), 8.9 (s, 1H) |
| **106** | [382] | 1.5 (s, 6H), 3.85 (s, 3H), 5.1 (s, 1H), 6.5 (d, 1H), 7.3 (d, 1H), 7.45 (m, 1H), 7.55 (m, 3H), 7.65 (m, 2H), 7.8 (m, 2H), 8.2 (s, 1H), 8.4 (s, 1H), 8.9 (s, 1H) |
| **107** | [380] | 1,5 (s, 6H) ; 5,15 (m, 1H) ; 7,45 (m, 1H) ; 7,6 (m, 2H) ; 7,8 (m, 4H) ; 8,0 (d, 1H) ; 8,2 (d, 1H) ; 8,5 (s, 1H) ; 8,95 (m, 1H) ; 9,0 (m, 1H) ; 9,05 (s, 1H) ; 9,35 (d, 1H). |
| **108** | [380] | 1,5 (s, 6H) ; 5,15 (s, 1H) ; 7,6 (m, 3H) ; 7,85 (m, 4H) ; 8,2 (m, 2H) ; 8,5 (s, 1H) ; 8,6 (d, 1H) ; 8,8 (d; 1H) ; 9,0 (s, 1H) ; 9,4 (s, 1H). |
| **109** | [357] | 1.5 (s, 6H), 5.1 (s, 1H) , 7.45 (t, 1H) , 7.55 (d, 1H) , 7.6 (d, 1H) , 7.7 (s, 2H), 7.75 (m, 1H) , 7.8 (m, 2H), 8.75 (s, 1H) , 8.95 (s, 1H) |
| **110** | [366] | 0.95 (d, 6H) , 1.5 (m, 7H) , 1.7 (m, 2H) , 4.2 (t, 2H), 5.1 (s, 1H) , 7.45 (m, 1H), 7.55 (m, 2H), 7.65 (m, 2H), 7.8 (s, 1H), 7.9 (s, 1H) , 8.15 (s, 1H), 8.25 (s, 1H), 8.9 (s, 1H) |
| **111** | [380] | 1.5 (s, 6H), 5.15 (s, 1H), 7.65 (m, 8H), 8.05 (m, 1H), 8.1 (m, 1H), 8.7 (s, 1H), 8.9 (m, 1H), 8.95 (s, 1H), 9.95 (s, 1H) |
| **112** | [358] | 4,45 (d, 2H) ; 5,30 (t, 1H) ; 6,5 (s, 1H) ; 7,3 (m, 2H) ; 7,4 (m, 1H) ; 7,45 (m, 3H) ; 7,65 (d, 1H) ; 7,75 (d, 1H) ; 8,2 (s, 1H) ; 8,4 (s, 1H) ; 8,7 (s, 1H); 11,2 (s, 1H) ; |
| **113** | [350] | 3,9 (s, 3H) ; 4,4 (s, 2H) ; 5,35 (s, 1H) ; 6,9 (d, 1H, ) ;7,4 (m, 3H) 7,65 (d, 2H); 8,25 (d, 1H) ; 8,45 (s 1H) ; 8,70 (s, 1H ) ; 8,75 (s, 1H) ; |
| **114** | [338] | 4,45 (m, 2H) ; 5,3 (m, 1H) ; 7,3 (m, 2H) ; 7,5 (m, 2H) ; 7,75 (d, 1H) ; 8,25 (m, 1H) ; 8,55 (d, 1H) ; 8,6 (d, 1H) ; 8,7 (m, 1H) ; 8,75 (m, 1H). |
| **115** | [339] | 2,25 (s, 3H) ; 4,44 (s, 2H) ; 5,3 (s, 1H) ; 7,1 (s, 1H) ; 7,3 (m, 2H) ; 7,45 (m, 3H) ; 7,6 (d, 1H) ; 8,3 (s, 1H) ; 8,65 (s, 1H). |
| **116** | [321] | 4,45 (d, 2H) ; 5,35 (t, 1H) ; 7,3 (m, 2H) ; 7,5 (m, 2H) ; 7,75 (d, 1H) ; 8,7 (s, 1H) ; 8,78 (s, 1H) ; 9,18 (s, 1H) ; 9,4 (s, 2H). |
| **117** | [358] | 4,45 (m, 2H) ; 5,25 (m, 1H) ; 7,05 (s, 1H) ; 7,2 (m, 1H) ; 7,25 (m, 2H) ; 7,45 (m, 4H) ; 7,75 (m, 2H) ; 8,6 (s, 1H) ; 8,75 (s, 1H) ; 11,0 (s, 1H). |
| **118** | [358] | 4,45 (m, 2H) ; 5,3 (m, 1H) ; 6,45 (s, 1H) ; 7,3 (m, 2H) ; 7,45 (m, 4H) ; 7,6 (m, 2H) ; 8,05 (s, 1H) ; 8,4 (s, 1H) ; 8,7 (s, 1H) ; 11,2 (s, 1H). |
| **119** | [350] | 4,1 (s, 3H) ; 4,45 (s, 2H) ; 5,3 (m, 1H) ; 7,2 (m, 1H) ; 7,3 (d, 2H) ; 7,5 (m, 2H) ; 7,7 (d, 1H) ; 8,2 (m, 1H) ; 8,5 (s, 1H) ; 8,6 (d, 1H) ; 8,75 (s, 1H). |
| **120** | [339] | 3,15 (s, 3H) ; 4,45 (m, 2H) ; 5,3 (m, 1H) ; 7,3 (m, 3H) ; 7,45 (m, 2H) ; 7,65 (d, 1H) ; 7,9 (d, 1H) ; 8,25 (s, 1H) ; 8,7 (s, 1H). |
| **121** | [372] | 3,8 (s, 3H) ; 4,45 (m, 2H) ; 5,35 (m, 1H) ; 6,55 (d, 1H) ; 7,3 (d, 2H) ; 7,4 (d, 1H) ; 7,5 (m, 1H) ; 7,6 (m, 2H) ; 7,75 (d, 1H) ; 7,8 (d, 1H) ; 8,2 (s, 1H) ; 8,5 (s, 1H) ; 8,75 (s, 1H). |
| **122** | [370] | 4,45 (m, 2H) ; 5,3 (m, 1H) ; 7,3 (m, 2H) ; 7,5 (m, 2H) ; 7,65 (m, 1H) ; 7,75 (d, 1H) ; 7,85 (m, 1H) ; 8,0 (d, 1H) ; 8,1 (m, 1H) ; 8,45 (s, 1H) ; 8,8 (s, 1H) ; 8,95 (m, 1H) ; 9,35 (m, 1H). |
| **123** | [370] | 4,45 (s, 2H) ;5,3 (m, 1H) ; 7,3 (m, 2H) ; 7,55 (m, 2H) ; 7,9 (m, 3H) ; 8,25 (m, 2H) ; 8,55 (s, 1H) ; 8,6 (d, 1H) ; 8,85 (d, 1H) ; 9,45 (s, 1H). |
| **124** | [356] | 4,45 (m, 2H) ; 5,35 (m, 1H) ; 7,3 (d, 2H) ; 7,5 (m, 2H) ; 7,7 (m, 3H) ; 8,75 (s, 1H) ; 8,85 (s, 1H). |
| **125** | [379] | 0,9 (d, 6H) ; 1,5 (m, 1H) ; 1,7 (m, 2H) ; 4,2 (t, 2H) ; 4,4 (s, 2H] ; 5,25 (s, 1H) ; 7,25 (m, 3H) ; 7,5 (m, 3H) ; 7,6 (d, 1H) ; 8,1 (s, 1H) ; 8,65 (s, 1H). |
| **126** | [376] | 4.6 (s, 2H) ; 5,35 (s, 1H) ; 6,5 (s, 1H) ; 7,25 (t, 1H) ; 7,4 (s, 1H) ; 7,45 (m, 2H) ; 7,65 (m, 3H] ; 8.2 (s, 1H) ; 8,4 (s, 1H) ; 8,75 (s, 1H) ; 11,2 (s, 1H). |
| **127** | [368] | 3,9 (s, 3H) ; 4.6 (d,2H) ; 5,3 (t, 1H) ; 6,9 (d, 1H) ; 7,2 (t, 1H) ; 7,4 (m, 1H) ; 7,6 (m, 1H) ; 7,7 (d, 1H) ; 8,25 (d, 1H) ; 8,4 (s, 1H) ; 8,75 (d, 2H). |
| **128** | [389] | 2,25 (s, 3H) ; 4,6 (s, 2H) ; 5,3 (s, 1H) ; 7,1 (s, 1H) ; 7,25 (t, 1H) ; 7,4 (m, 2H) ; 7,6 (m, 2H) ; 8,3 (s, 1H) ; 8,7 (s, 1H). |
| **129** | [376] | 4.6 (d, 2H) ; 5,35 (t, 1H) ; 6,45 (s, 1H) ; 7,25 (m, 1H) ; 7,45 (m, 2H) ; 7,65 (m, 4H) ; 8.05 (s, 1H) ; 8,45 (s, 1H) ; 8,75 (s, 1H) ; 11,2 (s, 1H). |
| **130** | [368] | 4,1 (s, 3H) ; 4,6 (d, 2H) ; 5,3 (t, 1H) ; 7,15 (m, 1H) ; 7,25 (t, 1H) ; 7,45 (m, 1H) ; 7,65 (m, 2H) ; 8,15 (m, 1H) ; 8,55 (s, 1H) ; 8,6 (d, 1H) ; 8,85 (s, 1H). |
| **131** | [357] | 2,45 (s, 3H) ; 4,6 (s, 2H) ; 5,35 (s, 1H) ; 7,25 (m, 2H) ; 7,4 (m, 1H) ; 7,65 (m, 2H) ; 7,9 (d, 1H) ; 8,25 (s, 1H) ; 8,8 (s, 1H). |
| **132** | [390] | 3,85 (s, 3H) ; 4,6 (d, 2H) ; 5,35 (t, 1H) ; 6,5 (d, 1H) ;7,25 (t, 1H) ; 7,45 (d, 1H) ; 7,5 (m, 2H) ; 7,65 (m, 2H) ; 7,8 (d, 1H) ; 8,2 (s, 1H) ; 8,45 (s, 1H) ; 8,8 (s, 1H). |
| **133** | [388] | 4,6 (d, 2H) ; 5,35 (t, 1H) ; 7,25 (t, 1H) ; 7,5 (d, 1H) ; 7,65 (m, 2H) ; 7,85 (m, 2H) ; 8,0 (d, 1H) ; 8,1 (m, 1H) ; 8,5 (s, 1H) ; 8,85 (s, 1H) ; 9,0 (m, 1H) ; 9,35 (d, 1H). |
| **134** | [388] | 4,6 (s, 2H) ; 5,35 (s, 1H) ; 7,25 (m, 1H) ; 7,7 (m, 4H) ; 8,2 (m, 2H) ; 8,55 (s, 1H) ;8,6 (d, 1H) ;8,8 (d 1H) ; 8,85 (s, 1H) ; 9,45 (s, 1H) |
| **135** | [374] | 455 (s, 2H) ; 5,35 (s, 1H) ; 7,25 (t, 1H) ; 7,50 (d, 1H) ; 7,65 (m, 1H) ; 7,7 (s, 2H) ; 7,75 (d, 1H) ; 8,80 (d, 1H) ; 8,85 (s, 1H) |
| **136** | [397] | 0,9 (d, 6H) ; 1,5 (m, 1H) ; 1,7 (m, 2H) ; 4,15 (t, 2H) ; 4,6 (s, 2H) ; 5,35 (s, 1H) ; 7,25 (t, 1H) ; 7,45 (m, 1H) ; 7,55 (m, 2H) ; 7,9 (s, 1H) ; 8,15 (s, 1H) ; 8,25 (s, 1 H) ; 8,8 (s, 1H) ; |
| **137** | [437] | 1,4 (s, 9H) ; 4,6 (m, 2H) ; 5,35 (m, 1H) ; 7,75 (d, 1H); 8,15 (s, 1H) ; 8,2 (s, 1H) ; 8,55 (m, 1H) ; 8,65 (m, 1H) ; 8,8 (m, 1H) ; 8,9 (m, 1H) ; 9,0 (s, 1H) ; 9,15 (s, 1H); 9,3 (s, 1H). |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 10 µM. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Par exemple, les composés n° 6, 7, 24, 50, 68, 79 et 86 ont montré une CE₅₀ de respectivement 0,9 ; 1,6 ; 52 ; 202 ; 20 ; 5 et 71 nM.II apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcereuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable,

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 g |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composés de formule (I) : 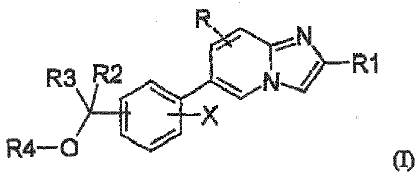
R₁ représente un groupe isoxazolyle, pyridinyle, thiazolyle, quinoléinyle, benzo[1,3]dioxolyle, indolyle, 1,2,3,4-tétrahydroquinoléinyle, benzofuranyle, dihydrobenzofuranyle, dihydrobenzoxazolyle, furyle, thiényle, pyrrolo[2,3-*b*]pyridinyle, pyrimidinyle, benzothiazolyle, benzothiophényle, benzimidazome, indazolyle, benzisoxazolyle, isoquinoléinyle, pyrazolyle ;
ces groupes pouvant éventuellement être substitués par un ou plusieurs atomes ou groupes choisis indépendamment les uns des autres parmi halogène, (C₁-C₁₀)alkyle, oxo, NRaRb ; (C₁-C₁₀)alcoxy, aryle, CONRaRb ;
Ra et Rb représentent, indépendamment l'un de l'autre, un atome d'hydrogène ou un groupe (C₁-C₁₀)alkyle.
X représente 1 ou 2 atomes d'hydrogène ou d'halogène.
R représente en position 3 de l'imidazo[1,2-*a*]pyridine un atome d'hydrogène, ou un groupe (C₁-C₁₀)alkyle.
R₂ et R₃ représentent, indépendamment l'un de l'autre un atome d'hydrogène ou un groupe (C₁-C₁₀)alkyle.
R₄ représente un atome d'hydrogène, ou un groupe (C₁-C₁₀)alkyle, éventuellement substitué par un groupe Rf ;
Rf représente un groupe (C₁-C₁₀)alcoxy,
à l'état de base ou de sel d'addition à un acide.

2. Composés de formule (I) selon l'une quelconque des revendications précédentes, **caractérisé en ce que** :
le groupe 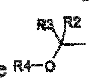 est en position 2, 3 ou 4 du phényle qui le porte.

3. Composés de formule (I) selon la revendication 1, **caractérisé en ce que**
R₁ représente un groupe isoxazolyle, pyridinyle, thiazolyle, quinoléinyle, benzo[1,3]dioxolyle, indolyle, 1,2,3,4 tétrahydroquinoléinyle, benzofuranyle, dihydrobenzofuranyle, dihydrobenzoxazolyle, furyle, thiényle, pyrrolo[2,3-b]pyridinyle, ces groupes pouvant éventuellement être substitués par un ou plusieurs atomes ou groupes choisis indépendamment les uns des autres parmi halogène, (C₁-C₁₀)alkyle, oxo, NRaRb, aryle ;
X représente un hydrogène,
R représente un hydrogène, ou un groupe (C₁-C₁₀)alkyle ;
R₂ et R₃ représentent, indépendamment l'un de l'autre, un atome d'hydrogène ;
R₄ représente un atome d'hydrogène, un groupe (C₁-C₁₀)alkyle, ce groupe étant éventuellement substitué par un groupe Rf ;
Ra et Rb représentent, indépendamment l'un de l'autre, un atome d'hydrogène ou un groupe (C₁-C₁₀)alkyle ;
Rf représente un groupe (C₁-C₁₀)alcoxy ;
à l'état de base ou de sel d'addition à un acide.

4. Composés :
• {3-[2-(5-Méthylisoxazol-4-yl)imidazo[1,2-*a*]pyridin-6-yl]phényl}méthanol ;
• [3-[2-(pyridin-4-yl)imidazo[1,2-*a*]pyridin-6-yl]phényl]méthanol et son chlorhydrate;
• Chlorhydrate (2:1) de 6-(3-*tert*butoxyméthylphényl)-2-(pyridin-3-yl)imidazo[1,2-*a*]pyridine ;
• [3-[2-(Thiazol-4-yl)imidazo[1,2-*a*]pyridin-6-yl]phényl]méthanol
• [3-[2-(quinoléin-3-yl)imidazo[1,2-*a*]pyridin-6-yl]phényl]méthanol et son chlorhydrate ;
• {3-[2-(1,3-benzodioxol-5-yl)imidazo[1,2-*a*]pyridin-6-yl]phényl} méthanol et son chlorhydrate;
• [3-]2-(pyridin-3-yl)imidazo[1,2-*a*]pyridin-6-yl]phényl]méthanol et son chlorhydrate ;
• {3-[2-[(1*H*-indol-5-yl)imidazo[1,2-*a*]pyridin-6-yl]phényl} méthanol et son chlorhydrate ;
• 6-[6-(3-hydroxymethylphényl)imidazo[1,2-*a*]pyridin-2-yl]-3,4-dihydro-1*H-*quinoléin-2-one et son chlorhydrate ;
• 2-(5-Bromo-2,3-dihydrobenzofuran-7-yl)-6-(3-*tert-*butoxyméthylphényl)imidazo[1,2-*a*]pyridine ;
• 6-[6-(3-Hydroxyméthylphényl)imidazo[1,2-*a*]pyridin-2-yl]-3*H*-benzoxazol-2-one et son chlorhydrate ;
• Chlorhydrate (1:1) de [2-(2-furan-3-ylimidazo[1,2-*a*]pyridin-6-yl)phényl]méthanol ;
• {3-[2-(5-Bromo-2,3-dihydrobenzofuran-7-yl)imidazo[1,2-*a*]pyridin-6-yl]phényl}méthanol ;
• {3-[2-(5-Chlorothién-2-yl)imidazo[1,2-*a*]pyridin-6-yl] phenyl}méthanol et son chlorhydrate ;
• {3-[2-(6-Diméthylaminopyridin-3-yl)imidazo[1,2-*a*]pyridin-6-yl]phényl}méthanol et son chlorhydrate ;
• {3-[2-(1*H*-indol-4-yl)imidazo[1,2-*a*]pyridin-6-yl]phényl} méthanol et son chlorhydrate ;
• {3-[2-(6-Aminopyridin-3-yl)imidazo[1,2-*a*]pyridin-6-yl]phényl} méthanol et son chlorhydrate ;
• {3-[2-(1*H*-indol-3-yl)imidazo[1,2-*a*]pyridin-6-yl]phényl} méthanol et son chlorhydrate ;
• {3-[2-(2-Aminopyridin-3-yl)imidazo[1,2-*a*]pyridin-6-yl]phényl} méthanol et son chlorhydrate ;
• {3-[2-(1*H*-pyrrolo[2,3-*b*]pyridin-5-yl)imidazo[1,2-*a*]pyridin-6-yl]phényl}méthanol et son chlorhydrate ;
• {3-[2-(3Phénylisoxazol-5-yl)imidazo[1,2-*a*]pyridin-6-yl]phényl}méthanol ;
• [3-[2-(Benzofuran-2-yl)imidazo[1,2-*a*]pyridin-6-yl]phényl]méthanol ;
• [3-[2-(Benzofuran-3-y)limidazo[1,2-*a*]pyridin-6-yl)phényl]méthanol;
• [4-[2-(Benzofuran-3-yl)imidazo[1,2-*a*]pyridin-6-yl]phényl]méthanol ;
• [3-[2-(Pyridin-2-yl)imidazo[1,2-*a*]pyridin-6-yl]phényl]méthanol;
• [4-[2-(Pyridin-2-yl]imidazo[1,2-*a*]pyridin-6-yl]phényl]méthanol ;
• [3-[2-(Thién-2-yl)imidazo[1,2-*a*]pyridin-6-yl]phényl]méthanol ;
• 2-(Benzofuran-2-yl)-6-[3-(2-méthoxyéthoxyméthyl)phényl]imidazo[1,2-*a*]pyridine ;
• 6-[3-(2-Méthoxyéthoxyméthyl)phényl]-2-(thién-2-yl)imidazo[1,2-*a*]pyridine et son oxalate ;
• [3-(2-Thién-3-ylimidazo[1,2-*a*]pyridin-6-yl)phényl]méthanol ;
• [3-(3-méthyl-2-thién-2-yl-imidazo[1,2-*a*]pyridin-6-yl)phényl]méthanol et son chlorhydrate ; ;
• {3-[2-(1*H*-indol-3-yl)-3-méthylimidazo[1,2-*a*]pyridin-6-yl]phényl}méthanol et son chlorhydrate ;
• {3-[2-(1*H*)-indol-6-yl)imidazo[1,2-*a*]pyridin-6-yl]phényl}méthanol et son chlorhydrate ;
• {3-[2-(2-Ethoxypyrimidin-5-yl)imidazo[1,2-*a*]pyridin-6-yl]phényl}méthanol et son chlorhydrate ;
• [2-(2-Quinoléin-3-ylimidazo[1,2-*a*]pyridin-6-yl)phényl]méthanol et son chlorhydrate;
• {3-[2-(2-Chloro-pyridin-4-yl)imidazo[1,2-*a*]pyridin-6-yl]phényl}méthanol et son chlorhydrate ;
• [3-(2-Benzothiazol-2-ylimldazo[1,2-*a*]pyridin-6-yl)phényl]méthanol et son chlorhydrate ;
• [3-{2-Benzo[b]thiophèn-2-ylimidazo[1,2-*a*]pyridin-6-yl)phényl]méthanol et son chlorhydrate ;
• [3-(2-Benzo[b]thiophèn-5-ylimidazo[1,2-*a*]pyridin-6-yl)phényl]méthanol et son chlorhydrate ;
• 3-(2-Benzo[b]thiophèn-3-ylimidazo[1,2-*a*]pyridin-6-yl)phényl]méthanol et son chlorhydrate ;
• {2-[2-(1*H*-Indol-6-yl)imidazo[1,2-*a*]pyridin-6-yl]phényl}méthanol et son chlorhydrate ;
• {3-[2-(2,3-Dihydrobenzofuran-5-yl)imidazo[1,2-*a*]pyridin-6-yl]phényl}méthanol et son chlorhydrate ;
• {2-[2-(1H-Indol-5-yl)imidazo[1,2-*a*]pyridin-6-yl]phényl}méthanol et son chlorhydrate ;
• [3-(2-Benzofuran-5-ylimidazo[1,2-*a*]pyridin-6-yl)phényl]méthanol ;
• {3-[2-(3-Chlorothién-2-yl)imidazo[1,2-*a*]pyridin-6-yl]phényl}méthanol;
• {2-Fluoro-6-[2-(5-méthylisoxazol-3-yl)imidazo[1,2-*a*]pyridin-6-yl]phényl}méthanol;
• 2-{3-[2-(1H-Indol-6-yl)imidazo[1,2-*a*]pyridin-6-yl]phényl}propan-2-ol ;
• 2-[3-(3-Méthyl-2-thién-2-ylimidazo[1,2-*a*]pyridin-6-yl)phényl]propan-2-ol ;
• 2-[3-(2-Thién-3-ylimidazo[1,2-*a*]pyridin-6-yl)phény]propan-2-ol :
• 2-[3-(2-Pyridin-2-ylimidazo[1,2-*a*]pyridin-6-yl)phényl]propan-2-ol ;
• 2-{3-[2-(5-Chlorothién-2-yl)imidazo[1,2-*a*]pyridin-6-yl]phényl}propan-2-ol ;
• 2-[3-(2-Benzofuran-2-ylimidazo[1,2-*a*]pyridin-6-yl)phényl]propan-2-ol ;
• 2-[3-(2-Thién-2-ylimidazo[1,2-*a*]pyridin-6-yl)phényl]propan-2-ol ;
• 2-[3-{2-Benzofuran-3-ylimidazo[1,2-*a*]pyridin-6-yl)phényl]propan-2-ol ;
• 2-[3-(2-Benzothiazol-2-ylimidazo[1,2,*a*]pyridin-6-yl)phényl]propan-2-ol;
• 2-{3-(2-Benzo[*b*]thiényl-2-ylimidazo[1,2-*a*]pyridin-6-yl)phényl]propan-2-ol ;
• 2-{3-[2-(1-Méthyl-1H-benzimidazol-2-yl)imidazo[1,2-*a*]pyridin-6-yl]phényl}propan-2-ol ;
• 2-{3-[2-(2,3-Dihydrobenzofuran-5-yl)imidazo[1,2-*a*]pyridin-6-yl]phényl}propan-2-ol ;
• 2-[3-(2-Furan-2-ylimidazo[1,2-*a*]pyridin-6-yl)phényl]propan-2-ol ;
• 2-[3-(2-Benzofuran-5-ylimidazo[1,2-*a*]pyridin-6-yl)phényl]propan-2-ol ;
• 2-[3-(2-Benzo[*b*]thiényl-5-ylimidazo[1,2-a]pyridin-6-yl)phényl]propan-2-ol ;
• 2-[3-(2-Thiazol-2-ylimidazo[1,2-*a*]pyridin-6-yl)phényl]propan-2-ol ;
• {2-Fluoro-6-[2-(1*H*-indazol-3-yl)imidazo[1,2-a]pyridin-6-yl]phényl}méthanol et son chlorhydrate ;
• 2-{3-[2-(1*H*-Indazol-3-yl)imidazo[1,2-*a*]pyridin-6-yl]phényl}propan-2-ol et son chlorhydrate ;
• {2,6-Difluoro-3-[2-(1*H*-indazol-3-yl)imidazo[1,2-*a*]pyridin-6-yl]phényl}méthanol et son chlorhydrate ;
• [2-Fluoro-6-(2-thién-3-ylimidazo[1,2-*a*]pyridin-6-yl)phényl]méthanol ;
• [2-Fluoro-6-(2-pyridin-2-ylimidazo[1,2-*a*]pyridin-6-yl)phényl]méthanol ;
• [2-Fluoro-6-(2-thién-2-ylimidazo[1,2-*a*]pyridin-6-yl)phényl]méthanol ;
• [2-(2-Benzothiazol-2-yl-imidazo[1,2-*a*]pyridin-6-yl)-6-fluorophényl]méthanol ;
• [2-(2-Benzo[b]thiophén-2-ylimidazo[1,2-*a*]pyridin-6-yl)-6-fluorophényl]méthanol;
• {2-[2-(2,3-Dihydrobenzofuran-5-yl)imidazo[1,2-*a*]pyridin-6-yl]-6-fluorophényl}méthanol ;
• [2-(2-Benzofuran-5-yl-imidazo[1,2-*a*]pyridin-6-yl)-6-fluorophényl]méthanol ;
• [2-(2-Benzo[b]thiényl-5-ylimidazo[1,2-*a*]pyridin-6-yl)-6-fluorophényl]méthanol ;
• [2-Fluoro-6-(2-thiazol-2-ylimidazo[1,2-*a*]pyridin-6-yl)phényl]méthanol ;
• (2,6-Difluoro-3-(3-méthyl-2-thién-2-ylimidazo[1,2-*a*]pyridin-6-yl)phényl]méthanol ;
• [2,6-Difluoro-3-(2-thién-3-ylimidazo[1,2-*a*]pyridin-6-yl)phényl]méthanol ;
• [2,6-Difluoro-3-(2-pyridin-2-ylimidazo[1,2-*a*]pyridin-6-yl)phényl]méthanol ;
• {3-[2-(5-Chlorothién-2-yl)imidazo[1,2-*a*]pyridin-6-yl]-2,6-difluorophényl}méthanol ;
• [3-(2-Benzofuran-2-ylimidazo[1,2-*a*]pyridin-6-yl)-2,6-difluorophényl]méthanol ;
• [2,6-Difluoro-3-(2-thién-2-ylimidazo[1,2-*a*]pyridin-6-yl)phényl]méthanol ;
• [3-(2-Benzofuran-3-ylimidazo[1,2-*a*]pyridin-5-yl)-2-6-difluorophényl]méthanol ;
• [3-(2-Benzothiazol-2-ylimidazo[1,2-*a*]pyridin-6-yl)-2,6-difiuorophényl]méthanol ;
• [2,6-Difluoro-3-(2-thiazol-2-ylimidazo[1,2-*a*]pyridin-6-yl)phényl]méthanol ;
• 6-(3-Méthoxyméthylphényl)-2-(1-méthyl-1*H*-indol-6-yl)imidazo[1,2-*a*]pyridine et son chlorhydrate ;
• 2-(*1H*-Indol-6-yl)-6-(3-méthoxyméthylphényl)imidazo[1,2-*a*]pyridine et son chlorhydrate ;
• 2-{3-[2-(5-Méthylisoxazol-3-yl)imidazo[1,2-*a*]pyridin-6-yl]phényl}propan-2-ol ;
• [2-Fluoro-6-(3-méthyl-2-thiényl-2-ylimidazo[1,2-*a*]pyridin-6-yl)phényl]méthanol ;
• {2-[2-(5-Chlorothiophén-2-yl)imidazo[1,2-*a*]pyridin-6-yl]-6-fluorophényl}méthanol ;
• [2-(2-Benzofuran-2-ylimidazo[1,2-*a*]pyridin-6-yl)-6-fluorophényl]méthanol ;
• [2-(2-Benzofuran-3-ylimidazo[1,2-*a*]pyridin-6-yl)-6-fluorophényl]méthanol ;
• {2-Fluoro-6-[2-(1-méthyl-1*H*-benzimidazol-2-yl)imidazo[1,2-*a*]pyridin-6-yl]phényl}méthanol ;
• [2-Fluoro-6-(2-furan-2-ylimidazo[1,2-*a*]pyridin-6-yl)phényl]méthanol ;
• [3-(2-Benzo[*b*]thiényl-2-ylimidazo[1,2-*a*]pyridin-6-yl)-2,6-difluorophényl]méthanol ;
• {2,6-Difluoro-3-[2-(1-méthyl-1*H*-benzimidazol-2-yl)imidazo[1,2-*a*]pyridin-6-yl]phényl}méthanol ;
• {3-[2-(2,3-Dihydrobenzofuran-5-yl)-imidazo[1,2-*a*]pyridin-6-yl]-2,6-difluorophényl}méthanol ;
• [2,6-Difluoro-3-(2-furan-2-ylimidazo[1,2-*a*]pyridin-6-yl)phényl]méthanol ;
• [3-(2-Benzofuran-5-ylimidazo[1,2-*a*]pyridin-6-yl)-2,6-difluorophényl]méthanol ;
• [3-(2-Benzo[b]thiényl-5-ylimidazo[1,2-*a*]pyridin-6-yl)-2,6-difluorophényl]méthanol ;
• 2-(1*H*-Indol-6-yl)-6-[3-[2-(méthoxyéthyl)oxyméthyl]phényl]imidazo[1,2-*a*]pyridine ;
• 2-[3-(2-Benzo[d]isoxazol-3-ylimidazo[1,2-*a*]pyridin-6-yl)phényl]propan-2-ol;
• [2-(2-Benzo[d]isoxazol-3-ylimidazo[1,2-*a*]pyridin-6-yl)-6-fluorophényl]méthanol ;
• 2-{3-[2-(1*H*-Indol-5-yl)imidazo[1,2-*a*]pyridin-6-yl]phényl}propan-2-ol ;
• 2-{3-[2-(1*H*-Indol-4-yl)imidazo[1,2-*a*]pyridin-6-yl]phényl}propan-2-ol ;
• 2-{3-[2-(2-Méthoxypyridin-3-yl)imidazo[1,2-*a*]pyridin-6-yl]phényl}propan-2-ol ;
• 2-{3-[2-(4-Méthylthién-3-yl)imidazo[1,2,*a*]pyridin-6-yl]phényl}propan-2-ol ;
• 2-{3-[2-(1-Méthyl-1*H*-indol-5-yl)imidazo[1,2-*a*]pyridin-6-yl]phényl}propan-2-ol ;
• 2-[3-(2-Quinolin-5-ylimidazo[1,2-*a*]pyridin-6-yl)phényl]propan-2-ol ;
• 2-[3-(2-Isoquinolin-5-ylimidazo[1,2-*a*]pyridin-6-yl)phényl]propan-2-ol ;
• 2-{3-[2-(2,6-Difluoropyridin-4-yl)imidazo[1,2-*a*]pyridin-6-yl]phényl}-propan-2-ol ;
• 2-(3-{2-[1-(3-Méthylbutyl)-1*H*-pyrazol-4-yl]imidazo[1,2-*a*]pyridin-6-yl}phényl)propan-2-ol ;
• 2-[3-(2-Quinolin-3-yl-imidazo[1,2-*a*]pyridin-6-yl)phényl]propan-2-ol ;
• {2-Fluoro-6-[2-(1*H*-indol-5-yl)imidazo[1,2-*a*]pyridin-6-yl]phényl}méthanol ;
• {2-Fluoro-6-[2-(6-méthoxypyridin-3-yl)imidazo[1,2-*a*]pyridin-6-yl]phényl} méthanol
• {2-Fluoro-6-[2-(3-fluoropyridin-4-yl)imidazo[1,2-*a*]pyridin-6-yl]phényl}méthanol ;
• {2-Fluoro-6-[2-(4-méthylthién-2-yl)imidazo[1,2-*a*]pyridin-6-yl]phényl}méthanol ;
• [2-Fluoro-6-(2-pyrimidin-5-ylimidazo[1,2-*a*]pyridin-6-yl)phényl]méthanol ;
• {2-Fluoro-6-[2-(1*H*-indol-4-yl)imidazo[1,2-*a*]pyridin-6-yl]phényl}méthanol;
• {2-Fluoro-6-[2-(1*H*-indol-6-yl)imidazo[1,2-*a*]pyridin-6-yl]phényl}méthanol ;
• {2-Fluoro-6-[2-(2-méthoxypyridin-3-yl)imidazo[1,2-*a*]pyridin-6-yl]phényl}méthanol ;
• {2-Fluoro-6-[2-(4-méthylthién-3-yl)imidazo[1,2-*a*]pyridin-6-yl]phényl}méthanol ;
• {2-Fluoro-6-[2-(1-méthyl-1*H*-indol-5-yl)imidazo[1,2-*a*]pyridin-6-yl]phényl}méthanol ;
• [2-Fluoro-6-(2-quinolin-5-ylimidazo[1,2-*a*]pyridin-6-yl)phényl]méthanol ;
• [2-Fluoro-6-(2-isoquinolin-5-ylimidazo[1,2-*a*]pyridin-6-yl)phényl]méthanol ;
• {2-[2-(2,6-Difluoropyridin-4-yl)imidazo[1,2-*a*]pyridin-6-yl]-6-fluorophényl}méthanol ;
• (2-Fluoro-6-{2-[1-(3-méthylbutyl)-1*H*-pyrazol-4-yl]imidazo[1,2-*a*]pyridin-6-yl}phenyl)méthanol ;
• {2,6-Difluoro-3-[2-(1*H*-indol-5-yl)imidazo[1,2-*a*]pyridin-6-yl]phényl}méthanol ;
• {2,6-Difluoro-3-[2-(6-méthoxypyridin-3-yl)imidazo[1,2-*a*]pyridin-6-yl]phényl}méthanol ;
• {2,6-Difluoro-3-[2-(4-méthylthién-2-yl)imidazo[1,2-*a*]pyridin-6-yl]phényl}méthanol ;
• {2,6-Difluoro-3-[2-(1*H*-indol-6-yl)imidazo[1,2-*a*]pyridin-6-yl]phényl}méthanol;
• {2,6-Difluoro-3-[2-(2-méthoxypyridin-3-yl)imidazo[1,2-*a*]pyridin-6-yl]phényl}méthanol ;
• {2,6-Difluoro-3-[2-(4-méthylthién-3-yl)imidazo[1,2-*a*]pyridin-6-yl]phényl}méthanol ;
• {2,6-Difluoro-3-[2-(1-méthyl-1*H*-indol-5-yl)imidazo[1,2-*a*]pyridin-6-yl]phényl}méthanol;
• [2,6-Difluoro-3-(2-quinolin-5-ylimidazo[1,2-*a*]pyridin-6-yl)phényl]méthanol ;
• [2,6-Difluoro-3-(2-isoquinolin-5-ylimidazo[1,2-*a*]pyridin-6-yl)phényl]méthanol ;
• {3-[2-(2,6-Difluoropyridin-4-yl)imidazo[1,2-*a*]pyridin-6-yl]-1,6-difluorophényl}méthanol ;
• (2,6-Difluoro-3-{2-[1-(3-méthylbutyl)-1*H*-pyrazol-4-yl]imidazo[1,2-*a*]pyridin-6-yl}phényl)méthanol ;
• *N*-*tert*-Butyl-5-[6-(2,4-difluoro-3-hydroxyméthylphényl)imidazo[1,2-*a*]pyridin-2-yl]nicotinamide.

5. Médicament, **caractérisé en ce qu'**il comprend un composé de formule (I) selon l'une quelconque des revendications 1 à 4, ou un sel d'addition de ce composé à un acide pharmaceutiquemmt acceptable.

6. Composition pharmaceutique, **caractérisée en ce qu'**elle comprend un composé de formule (I) selon l'une quelconque des revendications 1 à 4, ou un sel pharmaceutiquement acceptable, de ce composé, ainsi qu'au moins un excipient pharmaceutiquement acceptable.

7. Utilisation d'un composé de formule (I) selon l'une quelconque des revendications 1 à 4 pour la préparation d'un médicament destiné au traitement et à la prévention des maladies neurodégénératives.

8. Utilisation d'un composé de formule (I) selon l'une quelconque des revendications 1 à 4 pour la préparation d'un médicament destiné au traitement et à la prévention des traumatismes cérébraux et de l'épilepsie.

9. Utilisation d'un composé de formule (I) selon l'une quelconque des revendications 1 à 4 pour la préparation d'un médicament destiné au traitement et à la prévention des maladies psychiatriques.

10. Utilisation d'un composé de formule (I) selon l'une quelconque des revendications 1 à 4 pour la préparation d'un médicament destiné au traitement et à la prévention des maladies inflammatoires.

11. Utilisation d'un composé de formule (I) selon l'une quelconque des revendications 1 à 4 pour la préparation d'un médicament destiné au traitement et à la prévention de l'ostéoporose et les cancers.

12. Utilisation d'un composé de formule (I) selon l'une quelconque des revendications 1 à 4 pour la préparation d'un médicament destiné au traitement et à la prévention de la maladie de Parkinson, d'Alzheimer, des tauopathies, de la sclérose en plaque.

13. Utilisation d'un composé de formule (I) selon l'une quelconque des revendications 1 à 4 pour la preparation d'un médicament destiné au traitement et à la prévention de la schizophrénie, la dépression, la dépendance à une substance, les troubles du déficit de l'attention et de l'hyperactivité.

14. Procédé de synthèse des composés de formule (I) selon la revendication 1,par réaction de composés de formule (XI) 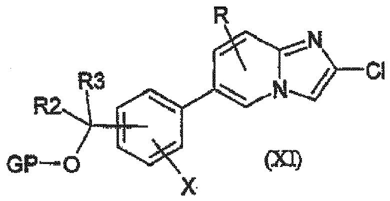 dans laquelle R, X, R3 et R2 sont tels que définis dans la revendication 1 et GP est un groupement protection de la fonction hydroxyle, avec R1-W avec R1 est tel que défini dans la revendication 1 et W est un dérivé de bore ou d'étain puis le produit obtenu est déprotégé.

15. Procédé de synthèse des composés de formule (I) selon la revendication 1, par réaction de composés de formule (IX) 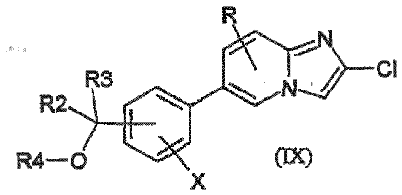 dans laquelle R, X, R3, R4 et R2 sont tels que définis dans la revendication 1 avec R1-W avec R1 est tel que défini dans la revendication 1 et W est un dérivé de bore ou d'étain.

16. Procédé de synthèse des composés de formule (I) selon la revendication 1, par réaction de composés de formule (VIII) 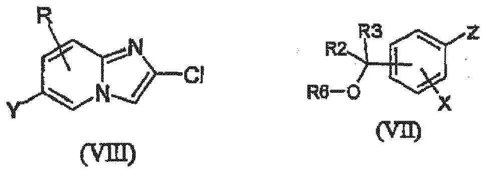 dans laquelle R est tel que défini dans la revendication 1 et Y représente un dérivé de bore avec un composé de formule (VII) dans laquelle R6 représente R4 et R2, R3, R4 et X sont tel que défini dans la revendication 1 et Z est un halogène

17. Procédé de synthèse des composés de formule (I) selon la revendication 1, par réaction de composés de formule (XII) 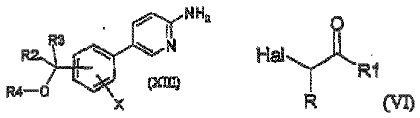 dans laquelle R2, R3, R4 et X sont tel que défini dans la revendication 1 avec un composé de formula (VI) R1, R sont tel que défini dans la revendication 1 et Hal est un halogène.

18. Procédé de synthèse des composés de formule (I) selon la revendication 1, par réaction de composés de formule (XIV) 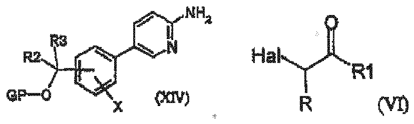 dans laquelle R2, R3, R4 et X sont tel que défini dans la revendication 1 et GP est un groupement protection de l'a fonction hydroxyle avec un composé de formule (VI) R1, R sont tel que défini dans la revendication 1 et Hal est un halogène puis le produit obtenu est déprotégé.

19. Composés intermédiaires

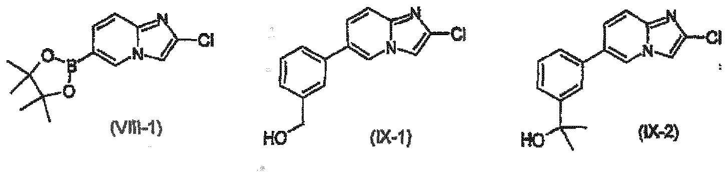

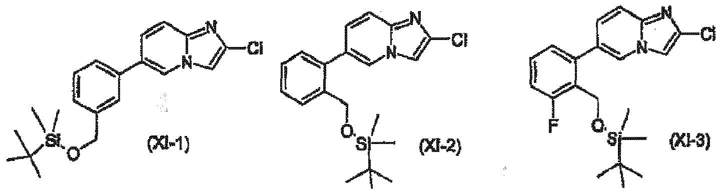

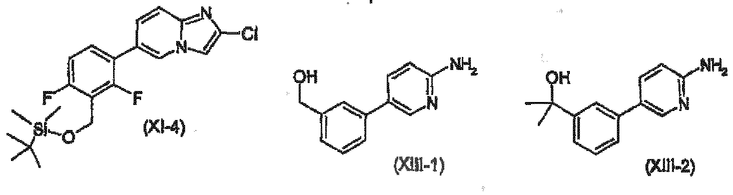

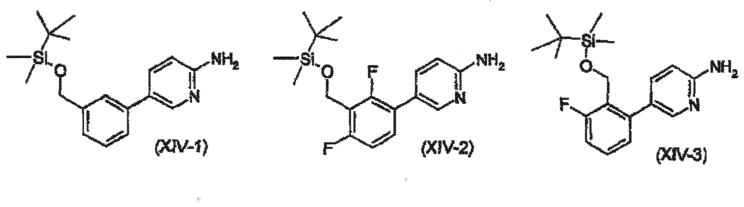

20. Procédé de synthèse selon l'une quelconque des revendications 14 à 18 où les composés de formules générales (VIII), (IX), (XI), (XII), et (XIV) sont les composés de formules générales (VIII-1), (IX-1), (IX-2), (XI-1), (XI-2), (XI-3), (XI-4), (XIII-1), (XIII-2), (XIV-1), (XIV-2) et (XIV-3).

## Patentansprüche

1. Verbindungen der Formel (I): 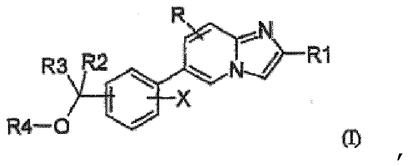 worin:
R₁ für eine Isoxazolyl-, Pyridinyl-, Thiazolyl-, Chinolinyl-, Benzo[1,3]dioxolyl-, Indolyl-, 1,2,3,4-Tetrahydrochinolinyl-, Benzofuranyl-, Dihydrobenzofuranyl-, Dihydrobenzoxazolyl-, Furyl-, Thienyl-, Pyrrolo[2,3-b]pyridinyl-, Pyrimidinyl-, Benzothiazolyl-, Benzothiophenyl-, Benzimidazolyl-, Indazolyl-, Benzisoxazolyl-, Isochinolinyl-, Pyrazolylgruppe steht,
wobei diese Gruppen gegebenenfalls mit einem oder mehreren Atomen oder Gruppen substituiert sein können, die unabhängig voneinander aus Halogen, (C₁-C₁₀)Alkyl, Oxo, NRaRb, ((C₁-C₁₀)Alkoxy, Aryl, CONRaRb ausgewählt werden, und
Ra und Rb unabhängig voneinander ein Wasserstoffatom oder eine (C₁-C₁₀)Alkylgruppe darstellen,
X für 1 oder 2 Wasserstoff- oder Halogenatome steht,
R an der Position 3 des Imidazo[1,2-a]pyridins für ein Wasserstoffatom oder eine (C₁-C₁₀)Alkylgruppe steht,
R2 und R3 unabhängig voneinander ein Wasserstoffatom oder eine (C₁-C₁₀)Alkylgruppe darstellen,
R4 für ein Wasserstoffatom oder eine (C₁-C₁₀)Alkylgruppe, die gegebenenfalls mit einer Gruppe Rf substituiert ist, steht und
Rf für eine (C₁-C₁₀)Alkoxygruppe steht,
im Zustand einer Base oder eines Säureadditionssalzes.

2. Verbindungen der Formel (I) nach einem der vorhergehenden Ansprüche, **dadurch gekennzeichnet, dass**:
die Gruppe 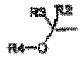 sich an Position 2, 3 oder 4 des Phenyls, das sie trägt, befindet.

3. Verbindungen der Formel (I) nach Anspruch 1, **dadurch gekennzeichnet, dass**
R₁ für eine Isoxazolyl-, Pyridinyl-, Thiazolyl-, Chinolinyl-, Benzo[1,3]dioxolyl-, Indolyl-, 1,2,3,4-Tetrahydrochinolinyl-, Benzofuranyl-, Dihydrobenzofuranyl-, Dihydrobenzoxazolyl-, Furyl-, Thienyl-, Pyrrolo[2,3-b]pyridinylgruppe steht, wobei diese Gruppen gegebenenfalls mit einem oder mehreren Atomen oder Gruppen substituiert sein können, die unabhängig voneinander aus Halogen, (C₁-C₁₀)Alkyl, Oxo, NRaRb, Aryl ausgewählt werden,
X für ein Wasserstoffatom steht,
R für ein Wasserstoffatom oder eine (C₁-C₁₀)Alkylgruppe steht,
R2 und R3 unabhängig voneinander ein Wasserstoffatom darstellen,
R4 ein Wasserstoffatom, eine (C₁-C₁₀)Alkylgruppe darstellt, wobei diese Gruppe gegebenenfalls mit einer Gruppe Rf substituiert ist,
Ra und Rb unabhängig voneinander ein Wasserstoffatom oder eine (C₁-C₁₀)Alkylgruppe darstellen,
Rf für eine (C₁-C₁₀)Alkoxygruppe steht,
im Zustand einer Base oder eines Säureadditionssalzes.

4. Verbindungen:
• {3-[2-(5-Methylisoxazol-4-yl)imidazo[1,2-a]pyridin-6-yl]phenyl}methanol,
• [3-[2-(Pyridin-4-yl)imidazo[1,2-a]pyridin-6-yl]phenyl]methanol und dessen Hydrochlorid,
• 6-(3-tert.-Butoxymethylphenyl)-2-(pyridin-3-yl)imidazo[1,2-a]pyridin-Hydrochlorid (2:1),
• [3-[2-(Thiazol-4-yl)imidazo[1,2-a]pyridin-6-yl]phenyl]methanol,
• [3-[2-(Chinolin-3-yl)imidazo[1,2-a]pyridin-6-yl]phenyl]methanol und dessen Hydrochlorid,
• {3-[2-(1,3-Benzodioxol-5-yl)imidazo[1,2-a]pyridin-6-yl]phenyl}methanol und dessen Hydrochlorid,
• [3-[2-(Pyridin-3-yl)imidazo[1,2-a]pyridin-6-yl]phenyl]methanol und dessen Hydrochlorid,
• 13-[2-[(1H-Indol-5-yl)imidazo[1,2-a]pyridin-6-yl]phenyl}methanol und dessen Hydrochlorid,
• 6-[6-(3-Hydroxymethylphenyl)imidazo[1,2-a]pyridin-2-yl]-3,4-dihydro-1H-chinolin-2-on und dessen Hydrochlorid,
• 2-(5-Brom-2,3-dihydrobenzofuran-7-yl)-6-(3-tert.-butoxymethylphenyl)imidazo[1,2-a]pyridin,
• 6-[6-(3-Hydroxymethylphenyl)imidazo[1,2-a]pyridin-2-yl]-3H-benzoxazol-2-on und dessen Hydrochlorid,
• [2-(2-Furan-3-ylimidazo[1,2-a]pyridin-6-yl)phenyl]methanol-Hydrochlorid (1:1),
• {3-[2-(5-Brom-2,3-dihydrobenzofuran-7-yl)imidazo[1,2-a]pyridin-6-yl]phenyl}methanol,
• {3[2-(5-Chlorothien-2-yl)imidazo[1,2-a]pyridin-6-yl]phenyl}methanol und dessen Hydrochlorid,
• {3-[2-(6-Dimethylaminopyridin-3-yl)imidazo[1,2-a]pyridin-6-yl]phenyl}methanol und dessen Hydrochlorid,
• {3-[2-(1H-Indol-4-yl)imidazo[1,2-a]pyridin-6-yl]phenyl}methanol und dessen Hydrochlorid,
• {3-[2-(6-Aminopyridin-3-yl)imidazo[1,2-a]pyridin-6-yl]phenyl}methanol und dessen Hydrochlorid,
• {3-[2-(1H-indol-3-yl)imidazo[1,2-a]pyridin-6-yl]phenyl}methanol und dessen Hydrochlorid,
• {3-[2-(2-Aminopyridin-3-yl)imidazo[1,2-a]pyridin-6-yl]phenyl}methanol und dessen Hydrochlorid,
• {3-[2-(1H-Pyrrolo[2,3-b]pyridin-5-yl)imidazo[1,2-a]pyridin-6-yl]phenyl}methanol und dessen Hydrochlorid,
• {3-[2-(3-Phenylisoxazol-5-yl)imidazo[1,2-a]pyridin-6-yl]phenyl}methanol,
• [3-[2-(Benzofuran-2-yl)imidazo[1,2-a]pyridin-6-yl]phenyl]methanol,
• [3-[2-(Benzofuran-3-yl)imidazo[1,2-a]pyridin-6-yl)phenyl]methanol,
• [4-[2-(Benzofuran-3-yl)imidazo[1,2-a]pyridin-6-yl]phenyl]methanol,
• [3-[2-(Pyridin-2-yl)imidazo[1,2-a]pyridin-6-yl]phenyl]methanol,
• [4-[2-(Pyridin-2-yl]imidazo[1,2-a]pyridin-6-yl]phenyl]methanol,
• [3-[2-(Thien-2-yl)imidazo[1,2-a]pyridin-6-yl]phenyl]methanol,
• 2-(Benzofuran-2-yl)-6-[3-(2-methoxyethoxymethyl)phenyl]imidazo[1,2-a]pyridin,
• 6-[3-(2-Methoxyethoxymethyl)phenyl]-2-(thien-2-yl)imidazo[1,2-a]pyridin und dessen Oxalat,
• [3-(2-Thien-3-ylimidazo[1,2-a]pyridin-6-yl)phenyl]methanol,
• [3-(3-Methyl-2-thien-2-ylimidazo[1,2-a]pyridin-6-yl)phenyl]methanol und dessen Hydrochlorid,
• {3-[2-(1H-Indol-3-yl)-3-methylimidazo[1,2-a]pyridin-6-yl]phenyl}methanol und dessen Hydrochlorid,
• {3-[2-(1H-Indol-6-yl)imidazo[1,2-a]pyridin-6-yl]phenyl}methanol und dessen Hydrochlorid,
• {3-[2-(2-Ethoxypyrimidin-5-yl)imidazo[1,2-a]pyridin-6-yl]phenyl}methanol und dessen Hydrochlorid,
• [2-(2-Chinolin-3-ylimidazo[1,2-a]pyridin-6-yl)phenyl]methanol und dessen Hydrochlorid,
• {3-[2-(2-Chlorpyridin-4-yl)imidazo[1,2-a]pyridin-6-yl]phenyl}methanol und dessen Hydrochlorid,
• [3-(2-Benzothiazol-2-ylimidazo[1,2-a]pyridin-6-yl)phenyl]methanol und dessen Hydrochlorid,
• [3-(2-Benzo[b]thiophen-2-ylimidazo[1,2-a]pyridin-6-yl)phenyl]methanol und dessen Hydrochlorid,
• [3-(2-Benzo[b]thiophen-5-ylimidazo[1,2-a]pyridin-6-yl)phenyl]methanol und dessen Hydrochlorid,
• 3-(2-Benzo[b]thiophen-3-ylimidazo[1,2-a]pyridin-6-yl)phenyl]methanol und dessen Hydrochlorid,
• {2-[2-(1H-Indol-6-yl)imidazo[1,2-a]pyridin-6-yl]phenyl}methanol und dessen Hydrochlorid,
• {3-[2-(2,3-Dihydrobenzofuran-5-yl)imidazo[1,2-a]pyridin-6-yl]phenyl}methanol und dessen Hydrochlorid,
• {2-[2-(1H-Indol-5-yl)imidazo[1,2-a]pyridin-6-yl]phenyl}methanol und dessen Hydrochlorid,
• [3-(2-Benzofuran-5-ylimidazo[1,2-a]pyridin-6-yl)phenyl]methanol,
• {3-[2-(3-Chlorthien-2-yl)imidazo[1,2-a]pyridin-6-yl]phenyl}methanol,
• {2-Fluor-6-[2-(5-methylisoxazol-3-yl)imidazo[1,2-a]pyridin-6-yl]phenyl}methanol,
• 2-{3-[2-(1H-Indol-6-yl)imidazo[1,2-a]pyridin-6-yllphenyllpropan-2-ol,
• 2-[3-(3-Methyl-2-thien-2-ylimidazo[1,2-a]pyridin-6-yl)phenyl]propan-2-ol,
• 2-[3-(2-Thien-3-ylimidazo[1,2-a]pyridin-6-yl)phenyl]propan-2-ol,
• 2-[3-(2-Pyridin-2-ylimidazo[1,2-a]pyridin-6-yl)phenyl]propan-2-ol,
• 2-{3-[2(5-Chlorthien-2-yl)imidazo[1,2-a]pyridin-6-yllphenyllpropan-2-ol,
• 2-[3-(2-Benzofuran-2-ylimidazo[1,2-a]pyridin-6-yl)phenyl]propan-2-ol,
• 2-[3-(2-Thien-2-ylimidazo[1,2-a]pyridin-6-yl)phenyl]propan-2-ol,
• 2-[3-(2-Benzofuran-3-ylimidazo[1,2-a]pyridin-6-yl)phenyl]propan-2-ol,
• 2-[3-(2-Benzothiazol-2-ylimidazo[1,2-a]pyridin-6-yl)phenyl]propan-2-ol,
• 2-[3-(2-Benzo[b]thienyl-2-ylimidazo[1,2-a]pyridin-6-yl)phenyl]propan-2-ol,
• 2-13-[2-(1-Methyl-1H-benzimidazol-2-yl)imidazo[1,2-a]pyridin-6-yl]phenyl}propan-2-ol,
• 2-{3-[2-(2,3-Dihydrobenzofuran-5-yl)imidazo[1,2-a]pyridin-6-yl]phenyl}propan-2-ol,
• 2-[3-(2-Furan-2-ylimidazo[1,2-alpyridin-6-yl)phenyl]propan-2-ol,
• 2-[3-(2-Benzofuran-5-ylimidazo[1,2-a]pyridin-6-yl)phenyl]propan-2-ol,
• 2-[3-(2-Benzo[b]thienyl-5-ylimidazo[1,2-a]pyridin-6-yl)phenyl]propan-2-ol,
• 2-[3-(2-Thiazol-2-ylimidazo[1,2-a]pyridin-6-yl)phenyl]propan-2-ol,
• {2-Fluor-6-[2-(1H-indazol-3-yl)imidazo[1,2-a]pyridin-6-yl]phenyl}methanol und dessen Hydrochlorid,
• 2-{3-[2-(1H-Indazol-3-yl)imidazo[1,2-a]pyridin-6-yl]phenyl}propan-2-ol und dessen Hydrochlorid,
• {2,6-Difluor-3-[2-(1H-indazol-3-yl)imidazo[1,2-a]pyridin-6-yl]phenyl}methanol und dessen Hydrochlorid,
• [2-Fluor-6-(2-thien-3-ylimidazo[1,2-a]pyridin-6-yl)phenyl]methanol,
• [2-Fluor-6-(2-pyridin-2-ylimidazo[1,2-a]pyridin-6-yl)phenyl]methanol,
• [2-Fluor-6-(2-thien-2-ylimidazo[1,2-a]pyridin-6-yl)phenyl]methanol,
• [2-(2-Benzothiazol-2-ylimidazo[1,2-a]pyridin-6-yl)-6-fluorphenyl]methanol,
• [2-(2-Benzo[b]thiophen-2-ylimidazo[1,2-a]pyridin-6-yl)-6-fluorphenyl]methanol,
• {2-[2-(2,3-Dihydrobenzofuran-5-yl)imidazo[1,2-a]pyridin-6-yl]-6-fluorphenyl}methanol,
• [2-(2-Benzofuran-5-ylimidazo[1,2-a]pyridin-6-yl)-6-fluorphenyl]methanol,
• [2-(2-Benzo[b]thienyl-5-ylimidazo[1,2-a]pyridin-6-yl)-6-fluorphenyl]methanol,
• [2-Fluor-6-(2-thiazol-2-ylimidazo[1,2-a]pyridin-6-yl)phenyl]methanol,
• [2,6-Difluor-3-(3-methyl-2-thien-2-ylimidazo[1,2-a]pyridin-6-yl)phenyl]methanol,
• [2,6-Difluor-3-(2-thien-3-ylimidazo[1,2-a]pyridin-6-yl)phenyl]methanol,
• [2,6-Difluor-3-(2-pyridin-2-ylimidazo[1,2-a]pyridin-6-yl)phenyl]methanol,
• {3-[2-(5-Chlorthien-2-yl)imidazo[1,2-a]pyridin-6-yl]-2,6-difluorphenyl}methanol,
• [3-(2-Benzofuran-2-ylimidazo[1,2-a]pyridin-6-yl)-2,6-difluorphenyl]methanol,
• [2,6-Difluor-3-(2-thien-2-ylimidazo[1,2-a]pyridin-6-yl)phenyl]methanol,
• [3-(2-Benzofuran-3-ylimidazo[1,2-a]pyridin-6-yl)-2,6-difluorphenyl]methanol,
• [3-(2-Benzothiazol-2-ylimidazo[1,2-a]pyridin-6-yl)-2,6-difluorphenyl]methanol,
• [2,6-Difluor-3-(2-thiazol-2-ylimidazo[1,2-a]pyridin-6-yl)phenyl]methanol,
• 6-(3-Methoxymethylphenyl)-2-(1-methyl-1H-indol-6-yl)imidazo[1,2-a]pyridin und dessen Hydrochlorid,
• 2-(1H-Indol-6-yl)-6-(3-methoxymethylphenyl)imidazo[1,2-a]pyridin und dessen Hydrochlorid,
• 2-{3-[2-(5-Methylisoxazol-3-yl)imidazo[1,2-a]pyridin-6-yl]phenyl}propan-2-ol,
• [2-Fluor-6-(3-methyl-2-thienyl-2-ylimidazo[1,2-a]pyridin-6-yl)phenyl]methanol,
• {2-[2-(5-Chlorthiophen-2-yl)imidazo[1,2-a]pyridin-6-yl]-6-fluorphenyl}methanol,
• [2-(2-Benzofuran-2-ylimidazo[1,2-a]pyridin-6-yl)-6-fluorphenyl]methanol,
• [2-(2-Benzofuran-3-ylimidazo[1,2-a]pyridin-6-yl)-6-fluorphenyl]methanol,
• {2-Fluor-6-[2-(1-methyl-1H-benzimidazol-2-yl)imidazo[1,2-a]pyridin-6-yl]phenyl}methanol,
• [2-Fluor-6-(2-furan-2-ylimidazo[1,2-a]pyridin-6-yl)phenyl]methanol,
• [3-(2-Benzo[b]thienyl-2-ylimidazo[1,2-a]pyridin-6-yl)-2,6-difluorphenyl]methanol,
• {2,6-Difluor-3-[2-(1-methyl-1H-benzimidazol-2-yl)imidazo[1,2-a]pyridin-6-yl]phenyl}methanol,
• {3-[2-(2,3-Dihydrobenzofuran-5-yl)imidazo[1,2-a]pyridin-6-yl]-2,6-difluorphenyl}methanol,
• [2,6-Difluor-3-(2-furan-2-ylimidazo[1,2-a]pyridin-6-yl)phenyl]methanol,
• [3-(2-Benzofuran-5-ylimidazo[1,2-a]pyridin-6-yl)-2,6-difluorphenyl]methanol,
• [3-(2-Benzo[b]thienyl-5-ylimidazo[1,2-a]pyridin-6-yl)-2,6-difluorphenyl]methanol,
• 2-(1H-Indol-6-yl)-6-[3-[2-(methoxyethyl)oxymethyl]phenyl]imidazo[1,2-a]pyridin,
• 2-[3-(2-Benzo[d]isoxazol-3-ylimidazo[1,2-a]pyridin-6-yl)phenyl]propan-2-ol,
• [2-(2-Benzo[d]isoxazol-3-ylimidazo[1,2-a]pyridin-6-yl)-6-fluorphenyl]methanol,
• 2-{3-[2-(1H-Indol-5-yl)imidazo[1,2-a]pyridin-6-yl]phenyl}propan-2-ol,
• 2-{3-[2-(1H-Indol-4-yl)imidazo[1,2-a]pyridin-6-yl]phenyl}propan-2-ol,
• 2-{3-[2-(2-Methoxypyridin-3-yl)imidazo[1,2-a]pyridin-6-yl]phenyl}propan-2-ol,
• 2-{3-[2-(4-Methylthien-3-yl)imidazo[1,2-a]pyridin-6-yl]phenyl}propan-2-ol,
• 2-{3-[2-(1-Methyl-1H-indol-5-yl)imidazo[1,2-a]pyridin-6-yl]phenyl}propan-2-ol,
• 2-[3-(2-Chinolin-5-ylimidazo[1,2-a]pyridin-6-yl)phenyl]propan-2-ol,
• 2-[3-(2-Isochinolin-5-ylimidazo[1,2-a]pyridin-6-yl)phenyl]propan-2-ol,
• 2-{3-[2-(2,6-Difluorpyridin-4-yl)imidazo[1,2-a]pyridin-6-yl]phenyl}propan-2-ol,
• 2-(3-{2-[1-(3-Methylbutyl)-1H-pyrazol-4-yl]imidazo[1,2-alpyridin-6-yllphenyl)propan-2-ol,
• 2-[3-(2-Chinolin-3-ylimidazo[1,2-a]pyridin-6-yl)phenyl]propan-2-ol,
• {2-Fluor-6-[2-(1H-indol-5-yl)imidazo[1,2-a]pyridin-6-yl]phenyl}methanol,
• {2-Fluor-6-[2-(6-methoxypyridin-3-yl)imidazo[1,2-a]pyridin-6-yl]phenyl}methanol,
• {2-Fluor-6-[2-(3-fluorpyridin-4-yl)imidazo[1,2-a]pyridin-6-yl]phenyl}methanol,
• {2-Fluor-6-[2-(4-methylthien-2-yl)imidazo[1,2-a]pyridin-6-yl]phenyl}methanol,
• [2-Fluor-6-(2-pyrimidin-5-ylimidazo[1,2-a]pyridin-6-yl)phenyl]methanol,
• {2-Fluor-6-[2-(1H-indol-4-yl)imidazo[1,2-a]pyridin-6-yl]phenyl}methanol,
• {2-Fluor-6-[2-(1H-indol-6-yl)imidazo[1,2-a]pyridin-6-yl]phenyl}methanol,
• {2-Fluor-6-[2-(2-methoxypyridin-3-yl)imidazo[1,2-a]pyridin-6-yl]phenyl}methanol,
• {2-Fluor-6-[2-(4-methylthien-3-yl)imidazo[1,2-a]pyridin-6-yl]phenyl}methanol,
• {2-Fluor-6-[2-(1-methyl-1H-indol-5-yl)imidazo[1,2-a]pyridin-6-yl]phenyl}methanol,
• [2-Fluor-6-(2-chinolin-5-ylimidazo[1,2-a]pyridin-6-yl)phenyl]methanol,
• [2-Fluor-6-(2-isochinolin-5-ylimidazo[1,2-a]pyridin-6-yl)phenyl]methanol,
• {2-[2-(2,6-Difluorpyridin-4-yl)imidazo[1,2-a]pyridin-6-yl]-6-fluorphenyl}methanol,
• (2-Fluor-6-{2-[l-(3-methylbutyl)-lH-pyrazol-4-yl]imidazo[1,2-a]pyridin-6-yl}phenyl)methanol,
• {2,6-Difluor-3-[2-(1H-indol-5-yl)imidazo[1,2-a]pyridin-6-yl]phenyl}methanol,
• {2,6-Difluor-3-[2-(6-methoxypyridin-3-yl)imidazo[1,2-alpyridin-6-yllphenyllmethanol,
• {2,6-Difluor-3-[2-(4-methylthien-2-yl)imidazo[1,2-a]pyridin-6-yl]phenyl}methanol,
• {2,6-Difluor-3-[2-(1H-indol-6-yl)imidazo[1,2-a]pyridin-6-yl]phenyl}methanol,
• {2,6-Difluor-3-[2-(2-methoxypyridin-3-yl)imidazo[1,2-alpyridin-6-yllphenyllmethanol,
• {2,6-Difluor-3-[2-(4-methylthien-3-yl)imidazo[1,2-a]pyridin-6-yl]phenyl}methanol,
• {2,6-Difluor-3-[2-(1-methyl-1H-indol-5-yl)imidazo[1,2-alpyridin-6-yllphenyllmethanol,
• [2,6-Difluor-3-(2-chinolin-5-ylimidazo[1,2-a]pyridin-6-yl)phenyl]methanol,
• [2,6-Difluor-3-(2-isochinolin-5-ylimidazo[1,2-a]pyridin-6-yl)phenyl]methanol,
• {3-[2-(2,6-Difluorpyridin-4-yl)imidazo[1,2-a]pyridin-6-yl]-2,6-difluorphenyl}methanol,
• (2,6-Difluor-3-12-[1-(3-methylbutyl)-1H-pyrazol-4-yl]imidazo[1,2-a]pyridin-6-yl}phenyl)methanol,
• N-tert.-Butyl-5-[6-(2,4-difluor-3-hydroxymethylphenyl)imidazo[1,2-a]pyridin-2-yl]nicotinamid.

5. Medikament, **dadurch gekennzeichnet, dass** es eine Verbindung der Formel (I) nach einem der Ansprüche 1 bis 4 oder ein Additionssalz dieser Verbindung mit einer pharmazeutisch annehmbaren Säure umfasst.

6. Pharmazeutische Zusammensetzung, **dadurch gekennzeichnet, dass** sie eine Verbindung der Formel (I) nach einem der Ansprüche 1 bis 4 oder ein pharmazeutisch annehmbares Additionssalz dieser Verbindung sowie mindestens einen pharmazeutisch annehmbaren Excipienten umfasst.

7. Verwendung einer Verbindung der Formel (I) nach einem der Ansprüche 1 bis 4 zur Herstellung eines Medikaments zur Behandlung und Vorbeugung von neurodegenerativen Erkrankungen.

8. Verwendung einer Verbindung der Formel (I) nach einem der Ansprüche 1 bis 4 zur Herstellung eines Medikaments zur Behandlung und Vorbeugung von Hirntraumen und Epilepsie.

9. Verwendung einer Verbindung der Formel (I) nach einem der Ansprüche 1 bis 4 zur Herstellung eines Medikaments zur Behandlung und Vorbeugung von psychiatrischen Erkrankungen.

10. Verwendung einer Verbindung der Formel (I) nach einem der Ansprüche 1 bis 4 zur Herstellung eines Medikaments zur Behandlung und Vorbeugung von entzündlichen Erkrankungen.

11. Verwendung einer Verbindung der Formel (I) nach einem der Ansprüche 1 bis 4 zur Herstellung eines Medikaments zur Behandlung und Vorbeugung von Osteoporose und Krebserkrankungen.

12. Verwendung einer Verbindung der Formel (I) nach einem der Ansprüche 1 bis 4 zur Herstellung eines Medikaments zur Behandlung und Vorbeugung von Parkinson-Krankheit, Alzheimer-Krankheit, Tauopathien, Multipler Sklerose.

13. Verwendung einer Verbindung der Formel (I) nach einem der Ansprüche 1 bis 4 zur Herstellung eines Medikaments zur Behandlung und Vorbeugung von Schizophrenie, Depression, Abhängigkeit von einer Substanz, Aufmerksamkeitsdefizit- und Hyperaktivitätsstörungen.

14. Verfahren zur Synthese der Verbindungen der Formel (I) nach Anspruch 1 durch Umsetzen von Verbindungen der Formel (XI) 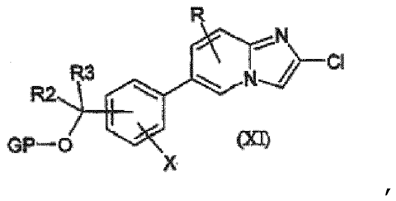 worin R, X, R3 und R2 wie in Anspruch 1 definiert sind und GP eine Schutzgruppe für die Hydroxylfunktion ist, mit R1-W, worin R1 wie in Anspruch 1 definiert ist und W für ein Bor- oder Zinnderivat steht, und anschließendes Entfernen der Schutzgruppe von dem erhaltenen Produkt.

15. Verfahren zur Synthese der Verbindungen der Formel (I) nach Anspruch 1 durch Umsetzen von Verbindungen der Formel (IX) 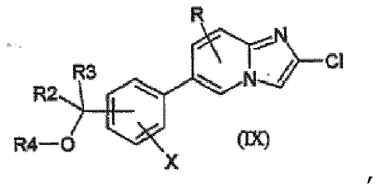 worin R, X, R3, R4 und R2 wie in Anspruch 1 definiert sind, mit R1-W, worin R1 wie in Anspruch 1 definiert ist und W für ein Bor- oder Zinnderivat steht.

16. Verfahren zur Synthese der Verbindungen der Formel (I) nach Anspruch 1 durch Umsetzen von Verbindungen der Formel (VIII) 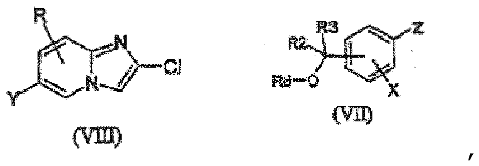 worin R wie in Anspruch 1 definiert ist und Y für ein Borderivat steht, mit einer Verbindung der Formel (VII), worin R6 für R4 steht und R2, R3, R4 und X wie in Anspruch 1 definiert sind und Z ein Halogen ist.

17. Verfahren zur Synthese der Verbindungen der Formel (I) nach Anspruch 1 durch Umsetzen von Verbindungen der Formel (XIII) 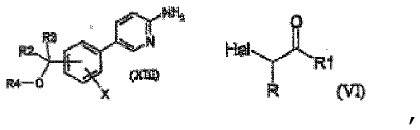 worin R2, R3, R4 und X wie in Anspruch 1 definiert sind, mit einer Verbindung der Formel (VI), R1, R wie in Anspruch 1 definiert sind und Hal für ein Halogen steht.

18. Verfahren zur Synthese der Verbindungen der Formel (I) nach Anspruch 1 durch Umsetzen von Verbindungen der Formel (XIV) 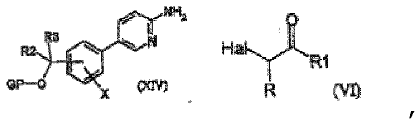 worin R2, R3, R4 und X wie in Anspruch 1 definiert sind und GP eine Schutzgruppe für die Hydroxylfunktion ist, mit einer Verbindung der Formel (VI), R1, R wie in Anspruch 1 definiert sind und Hal für ein Halogen steht, und anschließendes Entfernen der Schutzgruppe von dem erhaltenen Produkt.

19. Zwischenprodukte

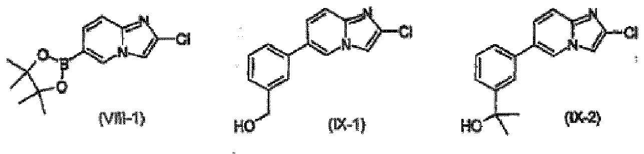

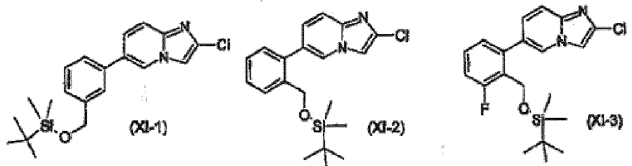

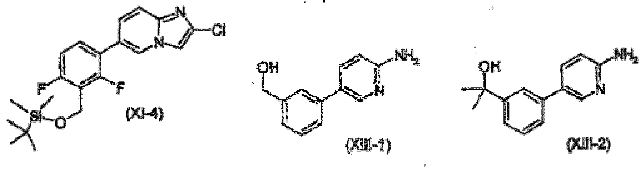

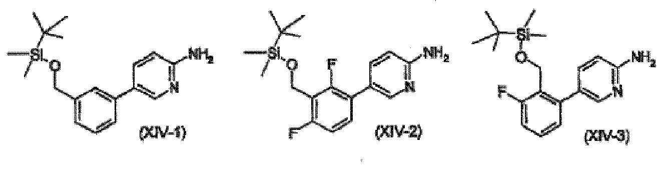

20. Verfahren zur Synthese nach einem der Ansprüche 14 bis 18, wobei es sich bei den Verbindungen der allgemeinen Formeln (VIII), (IX), (XI), (XIII) und (XIV) um die Verbindungen der allgemeinen Formeln (VIII-1), (IX-1), (IX-2), (XI-1), (XI-2), (XI-3), (XI-4), (XIII-1), (XIII-2), (XIV-1), (XIV-2) und (XIV-3) handelt.

## Claims

1. Compounds of formula (I): 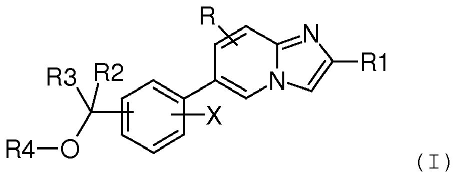 in which:
R₁ represents an isoxazolyl, pyridinyl, thiazolyl, quinolinyl, benzo[1,3]dioxolyl, indolyl, 1,2,3,4-tetrahydroquinolinyl, benzofuranyl, dihydrobenzofuranyl, dihydrobenzoxazolyl, furyl, thienyl, pyrrolo[2,3-*b*]pyridinyl, pyrimidinyl, benzothiazolyl, benzothiophenyl, benzimidazolyl, indazolyl, benzisoxazolyl, isoquinolinyl or pyrazolyl group;
it being possible for these groups to be optionally substituted with one or more atoms or groups chosen, independently of one another, from halogen, (C₁-C₁₀)alkyl, oxo, NRaRb, (C₁-C₁₀)alkoxy, aryl and CONRaRb;
Ra and Rb represent, independently of one another, a hydrogen atom or a (C₁-C₁₀)alkyl group;
X represents 1 or 2 hydrogen or halogen atoms;
R represents, at position 3, of the imidazo[1,2-a]pyridine, a hydrogen atom or a (C₁-C₁₀)alkyl group;
R₂ and R₃ represent, independently of one another, a hydrogen atom or a (C₁-C₁₀)alkyl group;
R₄ represents a hydrogen atom, or a (C₁-C₁₀) alkyl group optionally substituted with an Rf group;
Rf represents a (C₁-C₁₀)alkoxy group,
in the form of a base or of an addition salt with an acid.

2. Compounds of formula (I) according to any one of the preceding claims, **characterized in that**:
the group 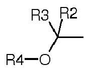 is at position 2, 3 or 4 of the phenyl which bears it.

3. Compounds of formula (I) according to Claim 1, **characterized in that**:
R₁ represents an isoxazolyl, pyridinyl, thiazolyl, quinolinyl, benzo[1,3]dioxolyl, indolyl, 1,2,3,4-tetrahydroquinolinyl, benzofuranyl, dihydrobenzofuranyl, dihydrobenzoxazolyl, furyl,
thienyl or pyrrolo[2,3-b]pyridinyl group, it being possible for these groups to be optionally substituted with one or more atoms or groups chosen, independently of one another, from halogen, (C₁-C₁₀) alkyl, oxo, NRaRb and aryl;
X represents a hydrogen;
R represents a hydrogen or a (C₁-C₁₀)alkyl group;
R₂ and R₃ represent, independently of one another, a hydrogen atom;
R₄ represents a hydrogen atom or a (C₁-C₁₀) alkyl group,
this group being optionally substituted with an Rf group;
Ra and Rb represent, independently of one another, a hydrogen atom or a (C₁-C₁₀)alkyl group;
Rf represents a (C₁-C₁₀)alkoxy group;
in the form of a base or of an addition salt with an acid.

4. Compounds:
• {3-[2-(5-methylisoxazol-4-yl)imidazo[1,2-*a*]pyridin-6-yl]phenyl}methanol;
• [3-[2-(pyridin-4-yl)imidazo[1,2-*a*]pyridin-6-yl]phenyl]methanol and the hydrochloride thereof;
• 6-(3-*tert*-butoxymethylphenyl)-2-(pyridin-3-yl)imidazo[1,2-*a*]pyridine hydrochloride (1:2);
• [3-[2-(thiazol-4-yl)imidazo[1,2-*a*]pyridin-6-yl]phenyl]methanol;
• [3-[2-(quinolin-3-yl)imidazo[1,2-*a*]pyridin-6-yl]phenyl]methanol and the hydrochloride thereof;
• {3-[2-(1,3-benzodioxol-5-yl)imidazo[1,2-*a*]pyridin-6-yl]phenyl}methanol and the hydrochloride thereof;
• [3-]2-(pyridin-3-yl)imidazo[1,2-*a*]pyridin-6-yl]phenyl]methanol and the hydrochloride thereof;
• {3-[2-[(1*H*-indol-5-yl)imidazo[1,2-*a*]pyridin-6-yl]phenyl}methanol and the hydrochloride thereof;
• 6-[6-(3-hydroxymethylphenyl)imidazo[1,2-*a*]pyridin-2-yl]-3,4-dihydro-1*H*-quinolin-2-one and the hydrochloride thereof;
• 2-(5-bromo-2,3-dihydrobenzofuran-7-yl)-6-(3-*tert-*butoxymethylphenyl)imidazo[1,2-*a*]pyridine;
• 6-[6-(3-hydroxymethylphenyl)imidazo[1,2-*a*]pyridin-2-yl]-3*H*-benzoxazol-2-one and the hydrochloride thereof;
• [2-(2-furan-3-ylimidazo[1,2-*a*]pyridin-6-yl)phenyl]methanol hydrochloride (1:1);
• {3-[2-(5-bromo-2,3-dihydrobenzofuran-7-yl)imidazo[1,2-*a*]pyridin-6-yllphenyllmethanol;
• {3-[2-(5-chlorothien-2-yl)imidazo[1,2-*a*]pyridin-6-yl]phenyl}methanol and the hydrochloride thereof;
• {3-[2-(6-dimethylaminopyridin-3-yl)imidazo[1,2-*a*]pyridin-6-yl]phenyl}methanol and the hydrochloride thereof;
• {3-[2-(1*H*-indol-4-yl)imidazo[1,2-*a*]pyridin-6-yl]phenyl}methanol and the hydrochloride thereof;
• {3-[2-(6-aminopyridin-3-yl)imidazo[1,2-*a*]pyridin-6-yl]phenyl} methanol and the hydrochloride thereof;
• {3-[2-(1*H*-indol-3-yl)imidazo[1,2-*a*]pyridin-6-yl]phenyl}methanol and the hydrochloride thereof;
• {3-[2-(2-aminopyridin-3-yl)imidazo[1,2-*a*]pyridin-6-yl]phenyl}methanol and the hydrochloride thereof;
• {3-[2-(1*H*-pyrrolo[2,3-*b*]pyridin-5-yl)imidazo[1,2-*a*]pyridin-6-yl]phenyl}methanol and the hydrochloride thereof;
• {3-[2-(3-phenylisoxazol-5-yl)imidazo[1,2-*a*]pyridin-6-yl]phenyl}methanol;
• [3-[2-(benzofuran-2-yl)imidazo[1,2-*a*]pyridin-6-yl]phenyl]methanol;
• [3-[2-(benzofuran-3-yl)imidazo[1,2-*a*]pyridin-6-yl)phenyl]methanol;
• [4-[2-(benzofuran-3-yl)imidazo[1,2-*a*]pyridin-6-yl]phenyl]methanol;
• [3-[2-(pyridin-2-yl)imidazo[1,2-*a*]pyridin-6-yl]phenyl]methanol;
• [4-[2-(pyridin-2-yl]imidazo[1,2-*a*]pyridin-6-yl]phenyl]methanol;
• [3-[2-(thien-2-yl)imidazo[1,2-*a*]pyridin-6-yl]phenyl]methanol;
• 2-(benzofuran-2-yl)-6-[3-(2-methoxyethoxymethyl)phenyl]imidazo[1,2-*a*]pyridine;
• 6-[3-(2-methoxyethoxymethyl)phenyl]-2-(thien-2-yl)imidazo[1,2-*a*]pyridine and the oxalate thereof;
• [3-(2-thien-3-ylimidazo[1,2-*a*]pyridin-6-yl)phenyl]methanol;
• [3-(3-methyl-2-thien-2-ylimidazo[1,2-*a*]pyridin-6-yl)phenyl]methanol and the hydrochloride thereof;
• {3-[2-(1*H*-indol-3-yl)-3-methylimidazo[1,2-*a*]pyridin-6-yl]phenyl}methanol and the hydrochloride thereof;
• {3-[2-(1*H*-indol-6-yl)imidazo[1,2-*a*]pyridin-6-yl]phenyl}methanol and the hydrochloride thereof;
• {3-[2-(2-ethoxypyrimidin-5-yl)imidazo[1,2-*a*]pyridin-6-yl]phenyl}methanol and the hydrochloride thereof;
• [2-(2-quinolin-3-ylimidazo[1,2-*a*]pyridin-6-yl)phenyl]methanol and the hydrochloride thereof;
• {3-[2-(2-chloropyridin-4-yl)imidazo[1,2-*a*]pyridin-6-yl]phenyl}methanol and the hydrochloride thereof;
• [3-(2-benzothiazol-2-ylimidazo[1,2-*a*]pyridin-6-yl)phenyl]methanol and the hydrochloride thereof;
• [3-(2-benzo[b]thiophen-2-ylimidazo[1,2-*a*]pyridin-6-yl)phenyl]methanol and the hydrochloride thereof;
• [3-(2-benzo[b]thiophen-5-ylimidazo[1,2-*a*]pyridin-6-yl)phenyl]methanol and the hydrochloride thereof;
• 3-(2-benzo[b]thiophen-3-ylimidazo[1,2-*a*]pyridin-6-yl)phenyl]methanol and the hydrochloride thereof;
• {2-[2-(1*H*-indol-6-yl)imidazo[1,2-*a*]pyridin-6-yl]phenyl}methanol and the hydrochloride thereof;
• {3-[2-(2,3-dihydrobenzofuran-5-yl)imidazo[1,2-*a*]pyridin-6-yl]phenyl}methanol and the hydrochloride thereof;
• {2-[2-(1H-indol-5-yl)imidazo[1,2-*a*]pyridin-6-yl]phenyl}methanol and the hydrochloride thereof;
• [3-(2-benzofuran-5-ylimidazo[1,2-*a*]pyridin-6-yl)phenyl]methanol;
• {3-[2-(3-chlorothien-2-yl)imidazo[1,2-*a*]pyridin-6-yl]phenyl}methanol;
• {2-fluoro-6-[2-(5-methylisoxazol-3-yl)imidazo[1,2-*a*]pyridin-6-yl]phenyl}methanol;
• 2-{3-[2-(1H-indol-6-yl)imidazo[1,2-*a*]pyridin-6-yl]phenyl}propan-2-ol;
• 2-[3-(3-methyl-2-thien-2-ylimidazo[1,2-*a*]pyridin-6-yl)phenyl]propan-2-ol;
• 2-[3-(2-thien-3-ylimidazo[1,2-*a*]pyridin-6-yl)phenyl]propan-2-ol;
• 2-[3-(2-pyridin-2-ylimidazo[1,2-*a*]pyridin-6-yl)phenyl]propan-2-ol;
• 2-{3-[2-(5-chlorothien-2-yl)imidazo[1,2-*a*]pyridin-6-yl]phenyl}propan-2-ol;
• 2-[3-(2-benzofuran-2-ylimidazo[1,2-*a*]pyridin-6-yl)phenyl]propan-2-ol;
• 2-[3-(2-thien-2-ylimidazo[1,2-*a*]pyridin-6-yl)phenyl]propan-2-ol;
• 2-[3-(2-benzofuran-3-ylimidazo[1,2-*a*]pyridin-6-yl)phenyl]propan-2-ol;
• 2-[3-(2-benzothiazol-2-ylimidazo[1,2-*a*]pyridin-6-yl)phenyl]propan-2-ol;
• 2-[3-(2-benzo[b]thienyl-2-ylimidazo[1,2-*a*]pyridin-6-yl)phenyl]propan-2-ol;
• 2-{3-[2-(1-methyl-1H-benzimidazol-2-yl)imidazo[1,2-*a*]pyridin-6-yl]phenyl}propan-2-ol;
• 2-{3-[2-(2,3-dihydrobenzofuran-5-yl)imidazo[1,2-*a*]pyridin-6-yl]phenyl}propan-2-ol;
• 2-[3-(2-furan-2-ylimidazo[1,2-*a*]pyridin-6-yl)phenyl]propan-2-ol;
• 2-[3-(2-benzofuran-5-ylimidazo[1,2-*a*]pyridin-6-yl)phenyl]propan-2-ol;
• 2-[3-(2-benzo[*b*]thienyl-5-ylimidazo[1,2-*a*]pyridin-6-yl)phenyl]propan-2-ol;
• 2-[3-(2-thiazol-2-ylimidazo[1,2-*a*]pyridin-6-yl)phenyl]propan-2-ol;
• {2-fluoro-6-[2-(1*H*-indazol-3-yl)imidazo[1,2-*a*]pyridin-6-yl]phenyl}methanol and the hydrochloride thereof;
• 2-{3-[2-(1*H*-indazol-3-yl)imidazo[1,2-*a*]pyridin-6-yl]phenyl}propan-2-ol and the hydrochloride thereof;
• {2,6-difluoro-3-[2-(1*H*-indazol-3-yl)imidazo[1,2-*a*]pyridin-6-yl]phenyl}methanol and the hydrochloride thereof;
• [2-fluoro-6-(2-thien-3-ylimidazo[1,2-*a*]pyridin-6-yl)phenyl]methanol;
• [2-fluoro-6-(2-pyridin-2-ylimidazo[1,2-*a*]pyridin-6-yl)phenyl]methanol;
• [2-fluoro-6-(2-thien-2-ylimidazo[1,2-*a*]pyridin-6-yl)phenyl]methanol;
• [2-(2-benzothiazol-2-ylimidazo[1,2-*a*]pyridin-6-yl)-6-fluorophenyl]methanol;
• [2-(2-benzo[b]thiophen-2-ylimidazo[1,2-*a*]pyridin-6-yl)-6-fluorophenyl]methanol;
• {2-[2-(2,3-dihydrobenzofuran-5-yl)imidazo[1,2-*a*]pyridin-6-yl]-6-fluorophenyl}methanol;
• [2-(2-benzofuran-5-ylimidazo[1,2-*a*]pyridin-6-yl)-6-fluorophenyl]methanol;
• [2-(2-benzo[b]thienyl-5-ylimidazo[1,2-*a*]pyridin-6-yl)-6-fluorophenyl]methanol;
• [2-fluoro-6-(2-thiazol-2-ylimidazo[1,2-*a*]pyridin-6-yl)phenyl]methanol;
• [2,6-difluoro-3-(3-methyl-2-thien-2-ylimidazo[1,2-*a*]pyridin-6-yl)phenyl]methanol;
• [2,6-difluoro-3-(2-thien-3-ylimidazo[1,2-*a*]pyridin-6-yl)phenyl]methanol;
• [2,6-difluoro-3-(2-pyridin-2-ylimidazo[1,2-*a*]pyridin-6-yl)phenyl]methanol;
• {3-[2-(5-chlorothien-2-yl)imidazo[1,2-*a*]pyridin-6-yl]-2,6-difluorophenyl}methanol;
• [3-(2-benzofuran-2-ylimidazo[1,2-*a*]pyridin-6-yl)-2,6-difluorophenyl]methanol;
• [2,6-difluoro-3-(2-thien-2-ylimidazo[1,2-*a*]pyridin-6-yl)phenyl]methanol;
• [3-(2-benzofuran-3-ylimidazo[1,2-*a*]pyridin-6-yl)-2,6-difluorophenyl]methanol;
• [3-(2-benzothiazol-2-ylimidazo[1,2-*a*]pyridin-6-yl)-2,6-difluorophenyl]methanol;
• [2,6-difluoro-3-(2-thiazol-2-ylimidazo[1,2-*a*]pyridin-6-yl)phenyl]methanol;
• 6-(3-methoxymethylphenyl)-2-(1-methyl-1*H*-indol-6-yl)imidazo[1,2-*a*]pyridine and the hydrochloride thereof;
• 2-(1*H*-indol-6-yl)-6-(3-methoxymethylphenyl)imidazo[1,2-*a*]pyridine and the hydrochloride thereof;
• 2-{3-[2-(5-methylisoxazol-3-yl)imidazo[1,2-*a*]pyridin-6-yl]phenyl}propan-2-ol;
• [2-fluoro-6-(3-methyl-2-thienyl-2-ylimidazo[1,2-*a*]pyridin-6-yl)phenyl]methanol;
• {2-[2-(5-chlorothiophen-2-yl)imidazo[1,2-*a*]pyridin-6-yl]-6-fluorophenyl}methanol;
• [2-(2-benzofuran-2-ylimidazo[1,2-*a*]pyridin-6-yl)-6-fluorophenyl]methanol;
• [2-(2-benzofuran-3-ylimidazo[1,2-*a*]pyridin-6-yl)-6-fluorophenyl]methanol;
• {2-fluoro-6-[2-(1-methyl-1*H*-benzimidazol-2-yl)imidazo[1,2-*a*]pyridin-6-yl]phenyl}methanol;
• [2-fluoro-6-(2-furan-2-ylimidazo[1,2-*a*]pyridin-6-yl)phenyl]methanol;
• [3-(2-benzo[b]thienyl-2-ylimidazo[1,2-*a*]pyridin-6-yl)-2,6-difluorophenyl]methanol;
• {2,6-difluoro-3-[2-(1-methyl-1*H*-benzimidazol-2-yl)imidazo[1,2-*a*]pyridin-6-yl]phenyl}methanol;
• {3-[2-(2,3-dihydrobenzofuran-5-yl)imidazo[1,2-*a*]pyridin-6-yl]-2,6-difluorophenyl}methanol;
• [2,6-difluoro-3-(2-furan-2-ylimidazo[1,2-*a*]pyridin-6-yl)phenyl]methanol;
• [3-(2-benzofuran-5-ylimidazo[1,2-*a*]pyridin-6-yl)-2,6-difluorophenyl]methanol;
• [3-(2-benzo[*b*]thienyl-5-ylimidazo[1,2-*a*]pyridin-6-yl)-2,6-difluorophenyl]methanol;
• 2-(1*H*-indol-6-yl)-6-[3-[2-(methoxyethyl)oxymethyl]phenyl]imidazo[1,2-*a*]pyridine;
• 2-[3-(2-benzo[*d*]isoxazol-3-ylimidazo[1,2-*a*]pyridin-6-yl)phenyl]propan-2-ol;
• [2-(2-benzo[*d*]isoxazol-3-ylimidazo[1,2-*a*]pyridin-6-yl)-6-fluorophenyl]methanol;
• 2-{3-[2-(1*H*-indol-5-yl)imidazo[1,2-*a*]pyridin-6-yl]phenyl}propan-2-ol;
• 2-{3-[2-(1*H*-indol-4-yl)imidazo[1,2-*a*]pyridin-6-yl]phenyl}propan-2-ol;
• 2-{3-[2-(2-methoxypyridin-3-yl)imidazo[1,2-*a*]pyridin-6-yl]phenyl}propan-2-ol;
• 2-{3-[2-(4-methylthien-3-yl)imidazo[1,2-*a*]pyridin-6-yl]phenyl}propan-2-ol;
• 2-{3-[2-(1-methyl-1*H*-indol-5-yl)imidazo[1,2-*a*]pyridin-6-yl]phenyl}propan-2-ol;
• 2-[3-(2-quinolin-5-ylimidazo[1,2-*a*]pyridin-6-yl)phenyl]propan-2-ol;
• 2-[3-(2-isoquinolin-5-ylimidazo[1,2-*a*]pyridin-6-yl)phenyl]propan-2-ol;
• 2-{3-[2-(2,6-difluoropyridin-4-yl)imidazo[1,2-*a*]pyridin-6-yl]phenyl}propan-2-ol;
• 2-(3-{2-[1-(3-methylbutyl)-1*H*-pyrazol-4-yl]imidazo[1,2-alpyridin-6-yllphenyl)propan-2-ol;
• 2-[3-(2-quinolin-3-ylimidazo[1,2-*a*]pyridin-6-yl)phenyl]propan-2-ol;
• {2-fluoro-6-[2-(1*H*-indol-5-yl)imidazo[1,2-*a*]pyridin-6-yl]phenyl}methanol;
• {2-fluoro-6-[2-(6-methoxypyridin-3-yl)imidazo[1,2-*a*]pyridin-6-yl]phenyl}methanol;
• {2-fluoro-6-[2-(3-fluoropyridin-4-yl)imidazo[1,2-*a*]pyridin-6-yl]phenyl}methanol;
• {2-fluoro-6-[2-(4-methylthien-2-yl)imidazo[1,2-*a*]pyridin-6-yl]phenyl}methanol;
• [2-fluoro-6-(2-pyrimidin-5-ylimidazo[1,2-*a*]pyridin-6-yl)phenyl]methanol;
• {2-fluoro-6-[2-(1*H*-indol-4-yl)imidazo[1,2-*a*]pyridin-6-yl]phenyl}methanol;
• {2-fluoro-6-[2-(1*H*-indol-6-yl)imidazo[1,2-*a*]pyridin-6-yl]phenyl}methanol;
• {2-fluoro-6-[2-(2-methoxypyridin-3-yl)imidazo[1,2-*a*]pyridin-6-yl]phenyl}methanol;
• {2-fluoro-6-[2-(4-methylthien-3-yl)imidazo[1,2-*a*]pyridin-6-yl]phenyl}methanol;
• {2-fluoro-6-[2-(1-methyl-1*H*-indol-5-yl)imidazo[1,2-*a*]pyridin-6-yl]phenyl}methanol;
• [2-fluoro-6-(2-quinolin-5-ylimidazo[1,2-*a*]pyridin-6-yl)phenyl]methanol;
• [2-fluoro-6-(2-isoquinolin-5-ylimidazo[1,2-*a*]pyridin-6-yl)phenyl]methanol;
• {2-[2-(2,6-difluoropyridin-4-yl)imidazo[1,2-*a*]pyridin-6-yl]-6-fluorophenyl}methanol;
• (2-fluoro-6-{2-[1-(3-methylbutyl)-1*H*-pyrazol-4-yl]imidazo[1,2-*a*]pyridin-6-yl}phenyl)methanol;
• {2,6-difluoro-3-[2-(1*H*-indol-5-yl)imidazo[1,2-*a*]pyridin-6-yl]phenyl}methanol;
• {2,6-difluoro-3-[2-(6-methoxypyridin-3-yl)imidazo[1,2-*a*]pyridin-6-yl]phenyl}methanol;
• {2,6-difluoro-3-[2-(4-methylthien-2-yl)imidazo[1,2-*a*]pyridin-6-yl]phenyl}methanol;
• {2,6-difluoro-3-[2-(1*H*-indol-6-yl)imidazo[1,2-*a*]pyridin-6-yl]phenyl}methanol;
• {2,6-difluoro-3-[2-(2-methoxypyridin-3-yl)imidazo[1,2-*a*]pyridin-6-yl]phenyl}methanol;
• {2,6-difluoro-3-[2-(4-methylthien-3-yl)imidazo[1,2-*a*]pyridin-6-yl]phenyl}methanol;
• {2,6-difluoro-3-[2-(1-methyl-1*H*-indol-5-yl)imidazo[1,2-*a*]pyridin-6-yl]phenyl}methanol;
• [2,6-difluoro-3-(2-quinolin-5-ylimidazo[1,2-*a*]pyridin-6-yl)phenyl]methanol;
• [2,6-difluoro-3-(2-isoquinolin-5-ylimidazo[1,2-*a*]pyridin-6-yl)phenyl]methanol;
• {3-[2-(2,6-difluoropyridin-4-yl)imidazo[1,2-*a*]pyridin-6-yl]-2,6-difluorophenyl}methanol;
• (2,6-difluoro-3-{2-[1-(3-methylbutyl)-1*H*-pyrazol-4-yl]imidazo[1,2-*a*]pyridin-6-yl}phenyl)methanol;
• *N*-*tert*-butyl-5-[6-(2,4-difluoro-3-hydroxymethylphenyl)imidazo[1,2-*a*]pyridin-2-yl]nicotinamide.

5. Medicament, **characterized in that** it comprises a compound of formula (I) according to any one of Claims 1 to 4, or an addition salt of this compound with a pharmaceutically acceptable acid.

6. Pharmaceutical composition, **characterized in that** it comprises a compound of formula (I) according to any one of Claims 1 to 4, or a pharmaceutically acceptable salt of this compound, and also at least one pharmaceutically acceptable excipient.

7. Use of a compound of formula (I) according to any one of Claims 1 to 4, for the preparation of a medicament for use in the treatment and prevention of neurodegenerative diseases.

8. Use of a compound of formula (I) according to any one of Claims 1 to 4, for the preparation of a medicament for use in the treatment and prevention of cerebral traumas and of epilepsy.

9. Use of a compound of formula (I) according to any one of Claims 1 to 4, for the preparation of a medicament for use in the treatment and prevention of psychiatric diseases.

10. Use of a compound of formula (I) according to any one of Claims 1 to 4, for the preparation of a medicament for use in the treatment and prevention of inflammatory diseases.

11. Use of a compound of formula (I) according to any one of Claims 1 to 4, for the preparation of a medicament for use in the treatment and prevention of osteoporosis and cancers.

12. Use of a compound of formula (I) according to any one of Claims 1 to 4, for the preparation of a medicament for use in the treatment and prevention of Parkinson's disease, Alzheimer's disease, tauopathies, and multiple sclerosis.

13. Use of a compound of formula (I) according to any one of Claims 1 to 4, for the preparation of a medicament for use in the treatment and prevention of schizophrenia, depression, substance dependence and attention deficit hyperactivity disorders.

14. Process for synthesizing the compounds of formula (I) according to Claim 1, by reacting compounds of formula (XI) 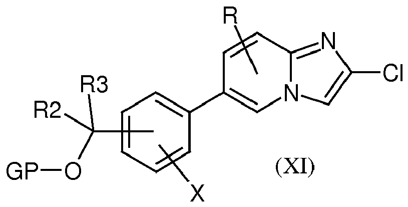 in which R, X, R3 and R2 are as defined in Claim 1 and GP is a hydroxyl-function-protecting group, with R1-W wherein R1 is as defined in Claim 1 and W is a boron or tin derivative, and then deprotecting the product obtained.

15. Process for synthesizing the compounds of formula (I) according to Claim 1, by reacting compounds of formula (IX) 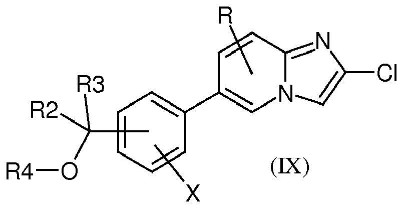 in which R, X, R3, R4 and R2 are as defined in Claim 1, with R1-W wherein R1 is as defined in Claim 1 and W is a boron or tin derivative.

16. Process for synthesizing the compounds of formula (I) according to Claim 1, by reacting compounds of formula (VIII) 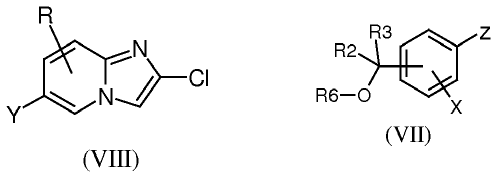 in which R is as defined in Claim 1 and Y represents a boron derivative, with a compound of formula (VII) in which R6 represents R4, and R2, R3, R4 and X are as defined in Claim 1 and Z is a halogen.

17. Process for synthesizing the compounds of formula (I) according to Claim 1, by reacting compounds of formula (XIII) 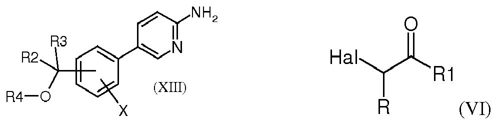 in which R2, R3, R4 and X are as defined in Claim 1, with a compound of formula (VI) in which R1 and R are as defined in Claim 1 and Hal is a halogen.

18. Process for synthesizing the compounds of formula (I) according to Claim 1, by reacting compounds of formula (XIV) 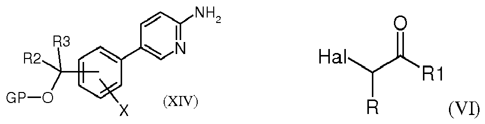 in which R2, R3, R4 and X are as defined in Claim 1 and GP is a hydroxyl-function-protecting group, with a compound of formula (VI) in which R1 and R are as defined in Claim 1 and Hal is a halogen, and then deprotecting the product obtained.

19. Intermediate compounds

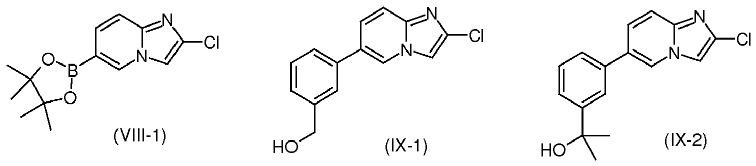

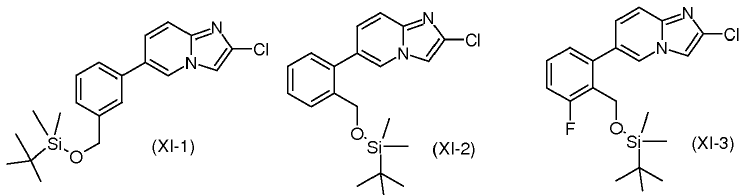

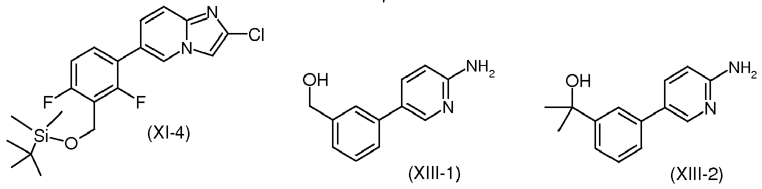

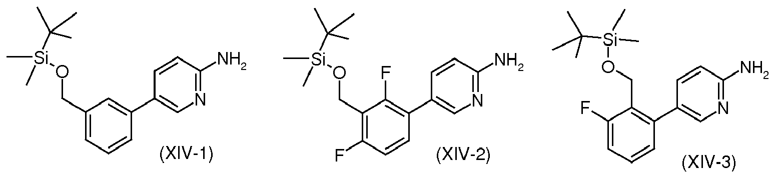

20. Synthesis process according to any one of Claims 14 to 18, wherein the compounds of general formulae (VIII), (IX), (XI), (XIII) and (XIV) are the compounds of general formulae (VIII-1), (IX-1), (IX-2), (XI-1), (XI-2), (XI-3), (XI-4), (XIII-1), (XIII-2), (XIV-1), (XIV-2) and (XIV-3).
